(19) Europäisches Patentamt
European Patent Office
Office européen des brevets

(11) EP 3 747 860 B1

(12) # EUROPÄISCHE PATENTSCHRIFT

(45) Veröffentlichungstag und Bekanntmachung des Hinweises auf die Patenterteilung:
**26.07.2023 Patentblatt 2023/30**

(21) Anmeldenummer: **20176235.8**

(22) Anmeldetag: **25.05.2020**

(51) Internationale Patentklassifikation (IPC):
***C07C 55/28*** *(2006.01)* ***C08K 5/12*** *(2006.01)*

(52) Gemeinsame Patentklassifikation (CPC):
(C-Sets verfügbar)
**C07C 55/28; C08K 5/12** (Forts.)

(54) **NEUE WEICHMACHER AUF CYCLOHEXANON-BASIS**
NOVEL CYCLOHEXANONE BASED PLASTICISERS
NOUVEAU PLASTIFIANT À BASE DE CYCLOHÉXANONE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.06.2019 EP 19178158**

(43) Veröffentlichungstag der Anmeldung:
**09.12.2020 Patentblatt 2020/50**

(73) Patentinhaber: **BASF SE**
**67056 Ludwigshafen am Rhein (DE)**

(72) Erfinder:
- **GRIMM, Axel**
  **67056 Ludwigshafen (DE)**
- **PFEIFFER, Matthias**
  **67056 Ludwigshafen (DE)**
- **MORGENSTERN, Herbert**
  **67056 Ludwigshafen (DE)**
- **KALLER, Martin**
  **67056 Ludwigshafen (DE)**
- **KASCHEL, Johannes**
  **67056 Ludwigshafen (DE)**
- **HAREMZA, Sylke**
  **69151 Neckargemünd (DE)**
- **MISSKE, Andrea**
  **67056 Ludwigshafen (DE)**

(74) Vertreter: **BASF IP Association**
**BASF SE**
**GBI-C006**
**67056 Ludwigshafen (DE)**

(56) Entgegenhaltungen:
**WO-A1-2018/024591**

- **DATABASE CAPLUS [Online] CHEMICAL ABSTRACTS SERVICE, COLUMBUS, OHIO, US; XP002795478, Database accession no. 1988:437711**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**C08K 5/12, C08L 27/06**

## Beschreibung

Die vorliegende Erfindung betrifft neue Weichmacher, ein Verfahren zur Herstellung der neuen Weichmacher, eine Weichmacher-Zusammensetzung, die ein oder mehrere der neuen Weichmacher enthält, eine Formmasse, die ein oder mehrere Polymere und ein oder mehrere der neuen Weichmacher enthält, ein Plastisol, das ein oder mehrere Polymere und ein oder mehrere der neuen Weichmacher enthält und die Verwendung einer Formmasse und eines Plastisol das einen oder mehrere der neuen Weichmacher enthält.

Weichmacher werden Polymeren oft zugegeben, um damit Eigenschaften der Polymere wie Flexibilität, Härte, Dehnbarkeit und/oder Verarbeitungstemperatur positiv zu beeinflussen. So ist beispielsweise Polyvinylchlorid (PVC) ein bis ca. 80°C hartes und sprödes Polymer. Durch die Zugabe geeigneter Weichmacher ist es möglich, die Härte und Flexibilität von PVC zu beeinflussen, wodurch weiches und flexibles PVC erhalten wird (Weich-PVC bzw. PVC-P). Neben PVC finden Weichmacher auch Anwendung in anderen Polymeren wie beispielsweise Polyvinylbutyral (PVB), Homo- und Copolymeren von Styrol, Polyacrylate, Polysulfide, in thermoplastischen Polyurethanen (PU), oder in Kautschuk. Eine Auflistung verschiedener Weichmacher ist beispielsweise in "Plasticizers", A. D. Godwin, in Applied Polymer Science 21st Century, edited by C. D. Craver und C. E. Carraher, Elsevier (2000); Seite 157 bis 175, oder "Plasticizers" D. F. Cadigan, C. J. Howick, in Ullmann's Encyclopedia of Industrial Chemistry, Wiley-VCH Verlag GmbH & Co. KGaA, Weinheim, Seite 599 bis 618, DOI: 10.1002/14356007.a20_439 beschrieben.

Die bis heute sicherlich am häufigsten verwendeten Weichmacher stellen Phthalsäureester dar. Aufgrund ihrer toxikologischen Eigenschaften geraten Phthalsäureester jedoch vor allem in Endnutzeranwendungen im Medizinischen- und/oder Lebensmittelbereich zunehmend unter regulatorischen Druck.

Zu Phthalsäureester alternative Weichmacher stellen Cyclohexanpolycarbonsäureester dar, wie sie beispielsweise in der WO 99/32427 beschrieben sind. Diese Verbindungen sind toxikologisch unbedenklich und können somit auch in sensiblen Anwendungsbereichen, wie Endnutzeranwendungen im Medizinischen- und/oder Lebensmittelbereich, eingesetzt werden.

Als weitere Alternative zu Phthalsäureester offenbaren beispielsweise die JP49-85136 und die US 3076775 tetra-substituierte Cyclohexanonderivate und deren Verwendung als Weichmacher in PVC.

CAS-Referat XP-002795478 zur Publikation von Huang, Huamin et al. in Gaodeng Xuexiao Huaxue Xuebao (1987), 8(6), 533-7 nennt Cyclohexan-2-on-1,3-dipropansäure-dipentylester im Zusammenhang mit der Herstellung von 8-Carboxyethyl-hexahydrocumarin. WO 2018/024591 A1 offenbart eine Weichmacher-Zusammensetzung, die mindestens einen Äpfelsäureester und mindestens einen 1,2-Cyclohexandicarbonsäureester enthält, sowie Formmassen und Plastisole, die eine solche Weichmacher-Zusammensetzung enthalten.

Aufgabe der vorliegenden Erfindung war es, neue Verbindungen zur Verfügung zu stellen, die als Weichmacher, oder als Bestandteil von Weichmacher-Zusammensetzung für Polymere, insbesondere thermoplastische Polymere, wie PVC, eingesetzt werden können. Die Verbindungen sollen als Weichmacher oder als Bestandteil von Weichmacher-Zusammensetzungen eine gute Effizienz und eine geringe Flüchtigkeit, wie Folienflüchtigkeit und/oder Prozessflüchtigkeit aufweisen. Eine gute Effizienz zeigt sich beispielsweise durch eine geringe Shore A Härte der damit weichgemachten Polymere. Werden die erfindungsgemäßen Verbindungen als Weichmacher oder als Bestandteil von Weichmacher-Zusammensetzungen in Plastisolen eingesetzt, sollen die damit weichgemachten Plastisole eine geringe Geliertemperatur und eine niedrige Viskosität im nicht gelierten Zustand aufweisen.

Die Aufgabe wird gelöst durch eine oder mehrere Verbindungen der allgemeinen Formel (I)

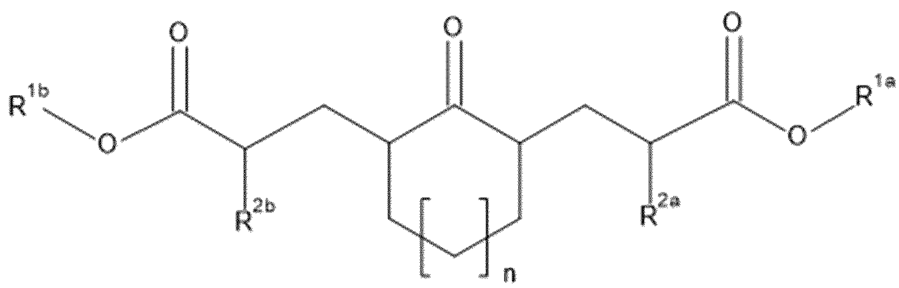

allgemeine Formel (I)

wobei

$R^{1a}$ und $R^{1b}$ unabhängig voneinander $C_8$- bis $C_{10}$-Alkyl sind,
$R^{2a}$ und $R^{2b}$ unabhängig voneinander H oder $CH_3$ sind,

n = 0, 1, 2 oder 7 ist.

Ein weiterer Gegenstand der vorliegenden Erfindung sind Verfahren zur Herstellung von einer oder mehrerer Verbindungen der allgemeinen Formel (I).

Ebenso ist Gegenstand der vorliegenden Erfindung eine Weichmacher-Zusammensetzung, die eine oder mehrere Verbindungen der allgemeinen Formel (I) und einen oder mehrere Weichmacher, die von den Verbindungen der allgemeinen Formel (I) unterschiedlich sind enthält.

Auch sind Formmassen und Plastisole die eine oder mehrere Verbindungen der allgemeinen Formel (I) enthalten Gegenstand der vorliegenden Erfindung.

Auch ist die Verwendung einer oder mehrerer Verbindungen der allgemeinen Formel (I) als Weichmacher oder als Bestandteile einer Weichmacher-Zusammensetzung für ein Polymer, eine Mischung verschiedener Polymere oder eines Plastisols Gegenstand der vorliegenden Erfindung.

Weiterhin sind auch Formkörper oder Folien die eine oder mehrere Verbindungen der allgemeinen Formel (I) enthalten Gegenstand der vorliegenden Erfindung.

## Definitionen

Polyvinylchlorid und Verfahren zur Herstellung von Polyvinylchlorid sind dem Fachmann bekannt (siehe beispielsweise "Poly(Vinyl Chloride)", A. W. Coaker, in Applied Polymer Science 21st Century, edited by C. D. Craver und C. E. Carraher, Elsevier (2000); Seite 107 bis 155, oder "Poly(Vinyl Chloride)" I. Fischer, W. F. Schmitt, H. C. Porth, M .W. Allsopp und G. Vianello, in Ullmann's Encyclopedia of Industrial Chemistry, Wiley-VCH Verlag GmbH & Co. KGaA, Weinheim, Seite 1 bis 29, DOI: 10.1002/14356007.a32_717.pub2). Polyvinylchlorid ist in vielen verschiedenen Arten verfügbar, wobei sich verschiedene Arten von Polyvinylchlorid in ihren Molekulargewichten, in ihrer Molekulargewichtsverteilung, der Partikelgröße der Polymerpartikel, der Partikelgrößenverteilung und/oder der Oberflächenstruktur der Polymerpartikel, die beispielsweise rau oder glatt sein kann, unterscheiden können. Verschiedene Arten von Polyvinylchlorid können sich auch im Grad ihrer Kettenverzweigung voneinander unterscheiden. Bei Polyvinylchlorid kann es sich um ein Homopolymer von Vinylchlorid oder um ein Copolymer von Vinylchlorid und Vinylacetat, Vinylidenchlorid, Ethylen und/oder Propylen handeln. Handelt es sich um ein Copolymer von Vinylchlorid und Vinylacetat, Vinylidenchlorid, Ethylen und/oder Propylen beträgt der Anteil an Vinylchlorid im Copolymer mindestens 50 Gewichtsprozent, bezogen auf das Gesamtgewicht des Copolymers.

Polyvinylchlorid wird beispielsweise durch Suspensionspolymerisation oder durch Emulsionspolymerisation hergestellt. Bei der Suspensionspolymerisation werden die Vinylmonomere in der Regel in Wasser unter Rühren und bei Temperatur- und Druckkontrolle suspendiert. Neben den Vinylmonomeren enthält die Suspension im Allgemeinen auch Suspendierhilfsmittel und Initiatoren. Nach Abschluss der Polymerisation wird die Suspension nach dem Fachmann bekannten Methoden aufgearbeitet. So kann die Suspension einem Stripper zugeführt werden, um nicht reagierte Monomere abzutrennen. Abschließend kann die Suspension gewaschen und getrocknet werden, um Polyvinylchlorid zu erhalten.

Durch Suspensionspolymerisation hergestelltes Polyvinylchlorid weist in der Regel agglomerierte Partikel mit einer Größe von 80 bis 200 Mikrometern auf. Durch Suspensionspolymerisation hergestelltes Polyvinylchlorid wird im Allgemeinen für Dry-Blend Anwendungen verwendet.

Die Herstellung von Polyvinylchlorid mittels Emulsionspolymerisation ähnelt der Herstellung von Polyvinylchlorid durch Suspensionspolymerisation. Ein Unterschied der Herstellverfahren liegt beispielsweise darin, dass bei der Emulsionspolymerisation die Vinylmonomere in Wasser, in der Regel unter Zuhilfenahme eines Emulgators, emulgiert werden, wodurch es zur Bildung von Latex-Partikeln kommt. Das Verhältnis von Wasser zu Vinylmonomere ist bei der Emulsionspolymerisation in der Regel größer als bei der Suspensionspolymerisation.

Durch Emulsionspolymerisation hergestelltes Polyvinylchlorid weist in der Regel agglomerierte Partikel mit einer Größe von 15 bis 20 Mikrometern auf. Im Allgemeinen werden durch Emulsionspolymerisation kleinere Polyvinylchloridpartikel als bei der Suspensionspolymerisation erhalten. Durch Emulsionspolymerisation hergestelltes Polyvinylchlorid wird oftmals bei der Herstellung von Plastisolen verwendet. So hergestellte Plastisole können beispielsweise als Beschichtungsmittel eingesetzt werden.

Polyvinylchlorid mit Partikelgrößen von 1 bis 40 Mikrometern kann beispielsweise durch Mikrosuspensionspolymerisation hergestellt werden. Durch Mikrosuspensionspolymerisation hergestelltes Polyvinylchlorid wird in Plastisolen verwendet.

Der K-Wert der die Molmasse des Polyvinylchlorids charakterisiert und nach DIN-EN 1628-2 (Nov 1999) bestimmt wird, liegt für das im Rahmen der vorliegenden Erfindung eingesetzte Polyvinylchlorid bevorzugt im Bereich von 57 bis 90, weiter bevorzugt im Bereich von 61 bis 85 und besonders bevorzugt im Bereich von 64 bis 80.

Bei einem Plastisol handelt es sich im Allgemeinen um eine Suspension von feinpulvrigem Polymer in flüssigem Weichmacher, wobei die Lösegeschwindigkeit des Polymers im flüssigen Weichmacher bei Raumtemperatur sehr gering

ist. Beim Erwärmen der Suspension von feinpulvrigem Polymer in flüssigem Weichmacher bildet sich eine weitgehend homogene Phase zwischen Polymer und Weichmacher. Dabei quellen und verbinden sich die einzelnen isolierten Kunststoffaggregate zu einem dreidimensionalen hochviskosen Gel. Dieser Vorgang wird in der Regel als Gelieren, im Englischen als "fusion", bezeichnet und findet ab einer gewissen Mindesttemperatur statt. Diese Mindesttemperatur wird generell als Gelier- oder Lösetemperatur bezeichnet, im Englischen "fusion point".

Der Ausdruck "(Meth)acryl-" steht für "Methacryl- oder Acryl-". So steht beispielsweise "(Meth)acrylsäureester" für "Methacrylsäureester oder Acrylsäureester".

Die Abkürzung phr (parts per hundred resin) steht für Gewichtsanteile pro hundert Gewichtsanteile Polymer. Polymer bezieht sich in diesem Zusammenhang auf die Gesamtmenge der in der erfindungsgemäßen Formmasse, bzw. im erfindungsgemäßen Plastisol, enthaltenen Polymere.

<u>Beschreibung der Erfindung</u>

<u>Eine oder mehrere Verbindungen der allgemeinen Formel (I)</u>

Die erfindungsgemäßen Verbindungen der allgemeinen Formel (I) können sowohl als reine cis-Isomere oder als reine trans-Isomere vorliegen wie nachfolgend gezeigt:

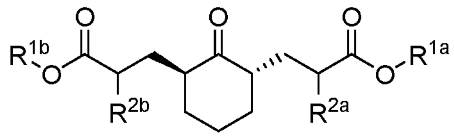
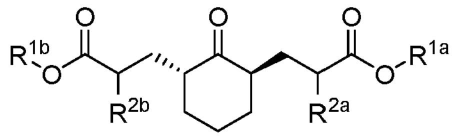

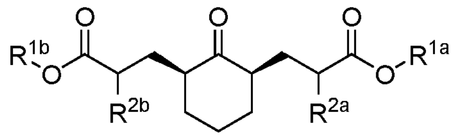
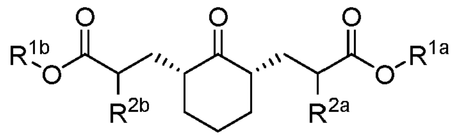

Die erfindungsgemäßen Verbindungen der allgemeinen Formel (I) können aber auch als cis/trans-Isomerengemische vorliegen. Für die Verwendung als Weichmacher eignen sich sowohl die reinen Isomere als auch die Isomerengemische.

$R^{1a}$ und $R^{1b}$ sind unabhängig voneinander $C_8$- bis $C_{10}$-Alkyl. $C_8$- bis $C_{10}$-Alkyl umfasst gerade oder verzweigte $C_8$- bis $C_{10}$-Alkylgruppen. Bei $C_8$- bis $C_{10}$-Alkyl handelt es sich beispielsweise um n-Octyl, iso-Octyl, 2-Ethylhexyl, n-Nonyl, iso-Nonyl, 2-Propylhexyl, n-Decyl, iso-Decyl oder 2-Propylheptyl. Es ist besonders bevorzugt, dass $R^{1a}$ und $R^{1b}$ unabhängig voneinander 2-Ethylhexyl, iso-Nonyl, oder 2-Propylheptyl sind.

Auch wenn $R^{1a}$ und $R^{1b}$ generell unabhängig voneinander sind, ist es bevorzugt, dass $R^{1a}$ und $R^{1b}$ gleich und $C_8$- bis $C_{10}$-Alkyl sind. $C_8$- bis $C_{10}$-Alkyl umfasst gerade oder verzweigte $C_8$- bis $C_{10}$-Alkylgruppen. Bei $C_8$- bis $C_{10}$-Alkyl handelt es sich beispielsweise um n-Octyl, iso-Octyl, 2-Ethylhexyl, n-Nonyl, iso-Nonyl, 2-Propylhexyl, n-Decyl, iso-Decyl oder 2-Propylheptyl. Es ist besonders bevorzugt, dass $R^{1a}$ und $R^{1b}$ gleich und 2-Ethylhexyl, iso-Nonyl, oder 2-Propylheptyl sind.

$R^{2a}$ und $R^{2b}$ sind unabhängig voneinander und unabhängig von $R^{1a}$ und $R^{1b}$ H oder $CH_3$. Es ist bevorzugt, dass $R^{2a}$ und $R^{2b}$ gleich und H oder $CH_3$ sind. Es ist besonders bevorzugt, dass $R^{2a}$ und $R^{2b}$ gleich und H sind.

In einer Verbindung der allgemeinen Formel (I) ist n = 0, 1, 2 oder 7. Es ist bevorzugt, dass n = 0 oder 1 ist. Es ist besonders bevorzugt, dass n = 1 ist.

In bevorzugten Verbindungen der allgemeinen Formel (I) sind:

- $R^{1a}$ und $R^{1b}$ gleich und $C_8$- bis $C_{10}$-Alkyl, $R^{2a}$ und $R^{2b}$ gleich und H oder $CH_3$, und n = 0, 1, 2 oder 7.

- $R^{1a}$ und $R^{1b}$ gleich und $C_8$- bis $C_{10}$-Alkyl, $R^{2a}$ und $R^{2b}$ gleich und H und n = 0, 1, 2 oder 7.

- $R^{1a}$ und $R^{1b}$ gleich und $C_8$- bis $C_{10}$-Alkyl, $R^{2a}$ und $R^{2b}$ gleich und $CH_3$, und n = 0, 1, 2 oder 7.

- $R^{1a}$ und $R^{1b}$ gleich und $C_8$- bis $C_{10}$-Alkyl, $R^{2a}$ und $R^{2b}$ gleich und H und n = 0 oder 1.

- $R^{1a}$ und $R^{1b}$ gleich und $C_8$- bis $C_{10}$-Alkyl, $R^{2a}$ und $R^{2b}$ gleich und $CH_3$ und n = 0 oder 1

- $R^{1a}$ und $R^{1b}$ gleich und 2-Ethylhexyl, iso-Nonyl oder 2-Propylheptyl, $R^{2a}$ und $R^{2b}$ gleich und H und n = 0 oder 1.
- $R^{1a}$ und $R^{1b}$ gleich und 2-Ethylhexyl, iso-Nonyl, oder 2-Propylheptyl, $R^{2a}$ und $R^{2b}$ gleich und H und n = 1.
- $R^{1a}$ und $R^{1b}$ gleich und 2-Ethylhexyl, iso-Nonyl, oder 2-Propylheptyl, $R^{2a}$ und $R^{2b}$ gleich und $CH_3$ und n = 0 oder 1.
- $R^{1a}$ und $R^{1b}$ gleich und 2-Ethylhexyl, iso-Nonyl, oder 2-Propylheptyl, $R^{2a}$ und $R^{2b}$ gleich und $CH_3$ und n = 1.

Verfahren zur Herstellung einer oder mehrerer Verbindungen der allgemeinen Formel (I)
Verbindungen der allgemeinen Formel (I) können über verschiedene Wege hergestellt werden.

Verfahren 1:

Verfahren zur Herstellung einer oder mehrerer Verbindungen der allgemeinen Formel (I)

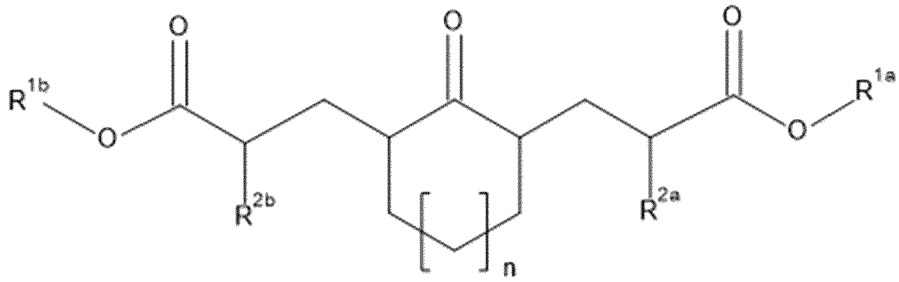

allgemeine Formel (I)

wobei

$R^{1a}$ und $R^{1b}$ unabhängig voneinander $C_8$- bis $C_{10}$-Alkyl sind,
$R^{2a}$ und $R^{2b}$ unabhängig voneinander H oder $CH_3$ sind,
n = 0, 1, 2 oder 7 ist,

das dadurch gekennzeichnet ist, dass

a) eine Verbindung der allgemeinen Formel (II)

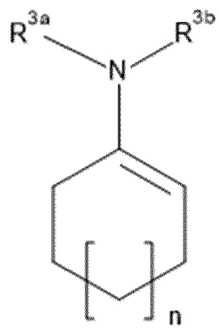

allgemeine Formel (II)

wobei

$R^{3a}$ und $R^{3b}$ unabhängig voneinander $C_1$- bis $C_{10}$-Alkyl sind oder zusammen einen Zyklus mit 4 bis 11 Kohlenstoffatomen bilden, oder
$R^{3a}$ H ist und $R^{3b}$ $C_1$- bis $C_{10}$-Alkyl ist, oder

$R^{3a}$ $C_1$-bis $C_{10}$-Alkyl ist und $R^{3b}$ H ist,
n = 0, 1, 2 oder 7 ist,
mit einer Verbindung der allgemeinen Formel (IIIa), (IIIb) oder einer Mischung aus Verbindungen der allgemeinen Formel (IIIa) und (IIIb)

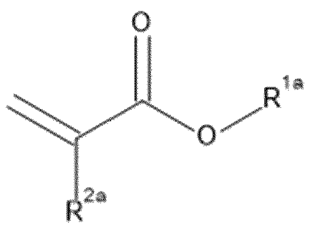

allgemeine Formel (IIIa)

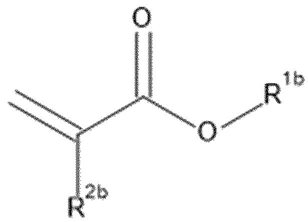

allgemeine Formel (IIIb)

wobei

$R^{1a}$ und $R^{1b}$ unabhängig voneinander $C_8$- bis $C_{10}$-Alkyl sind,
$R^{2a}$ und $R^{2b}$ unabhängig voneinander H oder $CH_3$ sind,
umgesetzt wird,

b) das Reaktionsprodukt aus Schritt a) zu einer Verbindung der allgemeinen Formel (I) hydrolysiert wird.

Bezüglich bevorzugter Ausführungsformen für $R^{1a}$, $R^{1b}$, $R^{2a}$, $R^{2b}$ und/oder n wird auf die vorherigen Angaben in vollem Umfang Bezug genommen.

Bei einer Verbindung der allgemeinen Formel (II) sind $R^{3a}$ und $R^{3b}$ unabhängig voneinander $C_1$- bis $C_{10}$-Alkyl oder bilden zusammen einen Zyklus mit 4 bis 11 Kohlenstoffatomen. Alternativ ist $R^{3a}$ H und $R^{3b}$ ist $C_1$- bis $C_{10}$-Alkyl, oder $R^{3b}$ ist H und $R^{3a}$ ist $C_1$- bis $C_{10}$-Alkyl.

$C_1$- bis $C_{10}$-Alkyl umfasst gerade, verzweigte oder cyclische $C_1$- bis $C_{10}$-Alkylgruppen. Bei $C_1$- bis $C_{10}$-Alkyl handelt es sich beispielsweise um Methyl, Ethyl, n-Propyl, iso-Propyl, n-Butyl, iso-Butyl, tert-Butyl, n-Pentyl, 2-Methylbutyl, 3-Methylbutyl, n-Hexyl, Cyclohexyl, 2-Methylpentyl, 3-Methylpentyl, 4-Methylpentyl, 3,3-Dimethylbutyl, 2-Ethylbutyl, n-Heptyl, 2-Methylhexyl, 2-Ethylpentyl, n-Octyl, iso-Octyl, 2-Ethylhexyl, n-Nonyl, iso-Nonyl, 2-Propylhexyl, n-Decyl, iso-Decyl oder 2-Propylheptyl.

Es ist bevorzugt, dass $R^{3a}$ und $R^{3b}$ unabhängig voneinander $C_1$- bis $C_5$-Alkyl sind. $C_1$- bis $C_5$-Alkyl umfasst gerade oder verzweigte $C_1$- bis $C_5$-Alkylgruppen. Bei $C_1$- bis $C_5$-Alkyl handelt es sich beispielsweise um Methyl, Ethyl, n-Propyl, iso-Propyl, n-Butyl, iso-Butyl, tert-Butyl, n-Pentyl, 2-Methylbutyl oder 3-Methylbutyl.

Bei einem Zyklus mit 4 bis 11 Kohlenstoffatomen sind die Kohlenstoffatome im Zyklus direkt oder über Heteroatome wie Sauerstoff, Stickstoff und/oder Schwefel, miteinander verbunden. Es ist bevorzugt, dass der Zyklus neben 4 bis 11 Kohlenstoffatomen und neben dem enaminbildenden Stickstoffatom kein, ein oder zwei Heteroatome enthält. Enthält der Zyklus neben dem enaminbildenden Stickstoffatom ein weiteres Heteroatom ist es bevorzugt, dass es sich bei dem Heteroatom um Sauerstoff, Stickstoff oder Schwefel handelt. So ist es beispielsweise bevorzugt, dass der Zyklus 4 Kohlenstoffatome und neben dem enaminbildenden Stickstoffatom kein weiteres Heteroatom, 4 Kohlenstoffatome und neben dem enaminbildenden Stickstoffatom ein weiteres Heteroatom, 5 Kohlenstoffatome und neben dem enaminbildenden Stickstoffatom kein weiteres Heteroatom oder 6 Kohlenstoffatome und neben dem enanminbildenden Stickstoffatom kein weiteres Heteroatom enthält. Auch wenn es generell möglich ist, dass zumindest ein Teil der Kohlenstoffatome im Zyklus als Alkenkohlenstoffatome vorliegt, ist es bevorzugt, dass alle Kohlenstoffatome im Zyklus als Alkanidylkohlenstoffatome, beispielsweise als $-CH_2-$, vorliegen. Die Kohlenstoffatome im Zyklus können einen oder mehrere Substituenten tragen, die von H unterschiedlich sind. Die einzelnen Substituenten können über ein Kohlenstoffatom oder ein Heteroatom, wie Sauerstoff oder Stickstoff mit dem jeweiligen Kohlenstoffatom im Zyklus verbunden sein. Bevorzugte Substituenten sind beispielsweise Methyl, Ethyl, Methoxy oder Ethoxy.

Es ist bevorzugt, dass in einer Verbindung der allgemeinen Formel (II) $R^{3a}$ und $R^{3b}$ unabhängig voneinander $C_1$- bis $C_5$-Alkyl sind oder zusammen ein Zyklus mit 4 bis 6 Kohlenstoffatomen bilden.

Es ist besonders bevorzugt, dass in einer Verbindung der allgemeinen Formel (II) $R^{3a}$ und $R^{3b}$ unabhängig voneinander $C_1$- bis $C_5$-Alkyl sind oder zusammen ein Zyklus mit 4 bis 6 Kohlenstoffatomen bilden, wobei in dem Zyklus neben dem enaminbildenden Stickstoffatom ein weiteres oder kein weiteres Heteroatom enthalten ist, alle Kohlenstoffatome als Alkanidylkohlenstoffatome vorliegen und keine von H unterschiedlichen Substituenten tragen.

Bei einer Verbindung der allgemeinen Formel (II) ist n = 0, 1, 2 oder 7. Es ist bevorzugt, dass n = 0 oder 1 ist.

So ist es insbesondere bevorzugt, dass in einer Verbindung der allgemeinen Formel (II) $R^{3a}$ und $R^{3b}$ unabhängig voneinander $C_1$- bis $C_5$-Alkyl sind oder zusammen ein Zyklus mit 4 bis 6 Kohlenstoffatomen bilden, wobei in dem Zyklus ein oder kein Heteroatom enthalten ist, alle Kohlenstoffatome als Alkanidylkohlenstoffatome vorliegen, keine von H unterschiedlichen Substituenten tragen und n = 0 oder 1 ist.

Verbindungen der allgemeinen Formel (II) können beispielsweise durch Umsetzung eines cyclischen Alkanons mit einer Aminverbindung hergestellt werden. So kann eine Verbindung der allgemeinen Formel (II) beispielsweise durch Umsetzung einer Verbindung der allgemeinen Formel (IV)

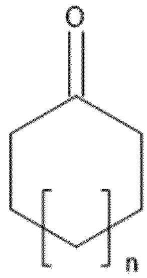

allgemeine Formel (IV)

wobei n = 0, 1, 2 oder 7 ist, mit einer Aminverbindung hergestellt werden.

Als Aminverbindung eignen sich prinzipiell alle Amine, die in der Lage sind ein Enamin mit einer Verbindung der allgemeinen Formel (IV) zu bilden. Die Amine können dabei als chemische Einzelverbindung oder als Mischung unterschiedlicher Einzelverbindungen eingesetzt werden. Sekundäre Amine sind bevorzugt. Bevorzugte sekundäre Amine weisen die allgemeine Formel $H\text{-}NR^{3a}R^{3b}$ auf, wobei $R^{3a}$ und $R^{3b}$ unabhängig voneinander $C_1$- bis $C_{10}$-Alkyl sind oder zusammen einen Zyklus mit 4 bis 11 Kohlenstoffatomen bilden. Besonders bevorzugte sekundäre Amine sind, Pyrrolidin, Piperidin, Hexamethylenimin, Morpholin oder eine beliebige Mischung aus zwei oder mehr der zuvor genannten sekundären Amine.

Es ist bevorzugt, dass das Molverhältnis zwischen Verbindungen der allgemeinen Formel (IV) und der Gesamtmenge der eingesetzten Amine 1 : 0,01 bis 1 : 5 beträgt. Es ist weiter bevorzugt, dass das Molverhältnis 1 : 0,5 bis 1: 2 beträgt. Es ist besonders bevorzugt, dass das Molverhältnis 1 : 0,8 bis 1 : 1,5 beträgt.

Die Herstellung einer Verbindung der allgemeinen Formel (II) kann in Gegenwart oder Abwesenheit eines Lösungsmittels erfolgen.

Als Lösungsmittel eignen sich prinzipiell alle Lösungsmittel die keine unerwünschten Nebenreaktionen mit den eingesetzten Edukten, Produkten und/oder Reagenzien eingehen und in denen die Edukte, Produkte und/oder Reagenzien eine ausreichende Löslichkeit aufweisen.

Geeignete Lösungsmittel sind beispielsweise chlorierte Kohlenwasserstoffe wie Dichlormethan oder Chlorbenzol, Alkohole wie $C_1$- bis $C_{13}$-Alkylalkohole, Ether wie Tetrahydrofuran, Dioxan oder MTBE, Cyclohexan, Methylcyclohexan, Nitrile wie Acetonitril, Benzol, Toluol, Xylol, Cumol oder eine beliebige Mischung aus zwei oder mehr der zuvor genannten Lösungsmittel. Es ist bevorzugt, dass das eingesetzte Lösungsmittel ein Azeotrop mit Wasser bildet.

Die Herstellung einer Verbindung der allgemeinen Formel (II) kann bei Umgebungsdruck oder bei vermindertem Druck oder bei erhöhtem Druck erfolgen.

Die Herstellung einer Verbindung der allgemeinen Formel (II) kann bei einer Temperatur von - 10 bis 100°C erfolgen, bevorzugt bei einer Temperatur von 0 bis 80°C.

Es ist von Vorteil die Temperatur und den Druck derart einzustellen, dass die Siedetemperatur des Lösungsmittel/Wasser-Azeotrops erreicht wird. Dadurch ist es möglich Reaktionswasser aus der Reaktion mittels Azeotropdestillation zu entfernen und das Gleichgewicht auf die Seite der Verbindung der allgemeinen Formel (II) zu verschieben. Als alternative oder zusätzliche Maßnahme können wasserentziehende Mittel der Reaktion zugesetzt werden. Als wasserentziehende Mittel eignen sich beispielsweise Molsiebe mit 3, 4 oder 5 Angström Porengröße, wobei Molsiebe mit 5 Angström Porengröße bevorzugt sind.

Detaillierte Ausführungsformen zur Herstellung von Verbindungen der allgemeinen Formel (II) finden sich beispielsweise in G. Stork et al. J. Am. Chem. Soc., 85, 207- 222 (1963), oder N. Matsumoto et al., Journal of Polymer Science: Polymer Chemistry Edition, 18, 1665-1678 (1980).

Verbindungen der allgemeinen Formel (III), bzw. (IIIa), (IIIb) können nach dem Fachmann bekannten Methoden hergestellt werden (siehe beispielsweise Ullmann's Encyclopedia of Industrial Chemistry, Acrylic Acid and Derivatives, DOI 10.1002/14356007.a01_161.pub3). So können Verbindungen der allgemeinen Formel (III) beispielsweise durch säurekatalysierte Veresterung von (Meth)acrylsäure mit $C_8$- bis $C_{10}$-Alkylalkoholen hergestellt werden. Veresterung-

sreaktionen von (Meth)acrylsäure können in der Gasphase oder in der Flüssigphase durchgeführt werden, wobei es bevorzugt ist, die Veresterungsreaktionen in der Flüssigphase durchzuführen. Je nach Herstellverfahren sind als Katalysatoren starke Säuren, wie p-Toluolsulfonsäure oder Schwefelsäure, oder saure Feststoffkatalysatoren, wie kationische Ionentauscherharze oder Lewis-saure Metallsalze bevorzugt. Verbindungen der allgemeinen Formel (III) mit längerkettigen Alkylresten, wie $C_8$- bis $C_{10}$-Alkyl, können beispielsweise auch durch Umesterung von (Meth)acrylsäu-remethyl- oder (Meth)acrylsäureethylester mit $C_8$- bis $C_{10}$-Alkylalkoholen hergestellt werden. Verfahren zur Umesterung von (Meth)acrylsäureester sind dem Fachmann ebenfalls bekannt oder erschließen sich ihm aus dem Stand der Technik (siehe beispielsweise Ullmann's Encyclopedia of Industrial Chemistry, Esters, Organic, DOI 10.1002/14356007.a09_565.pub2, insbesondere Kapitel 3.2).

Es ist bevorzugt, dass das Molverhältnis zwischen Verbindungen der allgemeinen Formel (II) und der Gesamtmenge der eingesetzten Verbindungen der allgemeinen Formel (III) 1 : 1 bis 1 : 8 beträgt. Es ist weiter bevorzugt, dass das Molverhältnis 1: 1,5 bis 1: 4 beträgt. Es ist besonders bevorzugt, dass das Molverhältnis 1 : 2 bis 1 : 3 beträgt.

Es ist bevorzugt, dass die Umsetzung einer Verbindung der allgemeinen Formel (II) mit einer Verbindung der allgemeinen Formel (IIIa), (IIIb) oder einer Mischung aus Verbindungen der allgemeinen Formel (IIIa) und (IIIb) in Gegenwart eines Polymerisationsstabilisators stattfindet. Der Einsatz eines Polymerisationsstabilisators hat den Vorteil, dass die Tendenz zur unerwünschten radikalischen Polymerisation von Verbindungen der allgemeinen Formel (IIIa) und/oder Formel (IIIb) vermindert wird.

Als Polymerisationsstabilisatoren eignen sich die im Stand der Technik bekannten Polymerisationsstabilisatoren von (Meth)acrylsäure und/oder (Meth)acrylsäureester. So eignen sich beispielsweise Kupfer(meth)acrylate, Kupferdithiocarbamate, Phenothiazine, phenolische Verbindungen, N-Oxyle, Phenylendiamine, Nitrosoverbindungen, Harnstoffe oder Thioharnstoffe. Diese Polymerisationsstabilisatoren können einzeln oder als beliebige Mischung eingesetzt werden. Bevorzugte Polymerisationsstabilisatoren sind Phenothiazine, phenolische Verbindungen, N-Oxyle oder beliebige Mischungen dieser.

Phenothiazine können beispielsweise Phenothiazin, Bis-($\alpha$-methylbenzyl)phenothiazin, 3,7-Dioctylphenothiazin, Bis-($\alpha$-dimethylbenzyl)phenothiazin oder eine beliebige Mischung dieser sein.

Phenolische Verbindungen können beispielsweise Hydrochinon, Hydrochinonmonomethylether, wie para-Methoxyphenol (MEHQ), Pyrogallol, Catechol, Resorcin, Phenol, Kresol, 2,4-Dimethyl-6-tert-butylphenol, 2,6-Di-tert-butyl-para-kresol oder eine beliebige Mischung dieser sein. Bevorzugte phenolische Verbindungen sind para-Methoxyphenol (MEHQ), 2,4-Dimethyl-6-tert-butylphenol, 2,6-Di-tert-butyl-para-kresol oder eine beliebige Mischung dieser.

N-Oxyle können beispielsweise Di-tert-butylnitroxid, 2,2,6,6-Tetramethyl-4-hydroxypiperidyl-1-oxyl, 2,2,6,6-Tetramethylpiperidyl-1-oxyl, 2,2,6,6-Tetramethylpiperidinoxyl, 4-Hydroxy-2,2,6,6-tetramethylpiperidinoxyl, 4,4',4"-Tris-1-(2,2,6,6-tetramethylpiperidinoxyl)phosphite oder eine beliebige Mischung dieser sein.

Der Polymerisationsstabilisator wird in den im Stand der Technik üblichen Mengen eingesetzt.

Die Umsetzung einer Verbindung der allgemeinen Formel (II) mit einer Verbindung der allgemeinen Formel (IIIa), (IIIb) oder einer Mischung aus Verbindungen der allgemeinen Formel (IIIa) und (IIIb) kann in Gegenwart oder Abwesenheit eines Lösungsmittels erfolgen.

Als Lösungsmittel eignen sich prinzipiell alle Lösungsmittel die keine unerwünschten Nebenreaktionen mit den eingesetzten Edukten, Produkten und/oder Reagenzien eingehen und in denen die Edukte, Produkte und/oder Reagenzien eine ausreichende Löslichkeit aufweisen. Polare Lösungsmittel sind bevorzugt, da diese zu höheren Ausbeuten führen.

Geeignete Lösungsmittel sind beispielsweise chlorierte Kohlenwasserstoffe wie Dichlormethan oder Chlorbenzol, Alkohole wie $C_1$- bis $C_{13}$-Alkylalkohole, beispielsweise Methanol, Ethanol, n-Propanol, iso-Propanol, n- Butanol, sec-Butanol, iso-Butanol, tert-Butanol, 3-Methyl-1-butanol, Ether wie Tetrahydrofuran, Dioxan, Dibutylether oder MTBE, Nitrile wie Acetonitril, Nitromethan, Dimethylsulfoxid, Sulfolan, Dimethylformamid, Dimethylacetamid, Benzol, Toluol, Xylol oder eine beliebige Mischung aus zwei oder mehr der zuvor genannten Lösungsmittel.

Es ist bevorzugt, dass es sich bei dem Lösungsmittel um iso-Propanol, sec-Butanol, iso-Butanol, tert- Butanol oder um die $C_5$- bis $C_{13}$-Alkylalkohole handelt, die durch Esterhydrolyse der eingesetzten Verbindungen der allgemeinen Formel (IIIa) und/oder (IIIb) erhalten werden. Iso-Propanol, sec-Butanol, iso-Butanol oder tert- Butanol sind besonders bevorzugt.

Die Umsetzung einer Verbindung der allgemeinen Formel (II) mit einer Verbindung der allgemeinen Formel (IIIa), (IIIb) oder einer Mischung aus Verbindungen der allgemeinen Formel (IIIa) und (IIIb) kann bei Umgebungsdruck oder vermindertem Druck oder erhöhtem Druck erfolgen.

Es ist bevorzugt, dass die Reaktionstemperatur in Schritt a) 0 bis 60 °C beträgt. Es ist weiter bevorzugt, dass die Reaktionstemperatur in Schritt a) 5 bis 50 °C beträgt. Es ist besonders bevorzugt, dass die Reaktionstemperatur in Schritt a) 10 bis 30 °C beträgt.

Das aus der Umsetzung einer Verbindung der allgemeinen Formel (II) mit einer Verbindung der allgemeinen Formel (IIIa), (IIIb) oder einer Mischung von Verbindungen der allgemeinen Formel (IIIa) und (IIIb) erhaltene Reaktionsprodukt wird durch Hydrolyse zu einer Verbindung der allgemeinen Formel (I) umgesetzt. Die Hydrolyse kann beispielsweise durch Zugabe von Wasser oder Zugabe einer wässrigen Säure oder durch Zugabe eines sauren Ionentauschers mit Wasser erfolgen.

Bei einem sauren Ionentauscher handelt es sich beispielsweise um Ionentauscher wie sie unter den Namen Amberlite, Amberlyt, Amberjet, Dowex, Diaion, Duolite, Purolite oder Nafion vertrieben werden.

Bei einer wässrigen Säure kann es sich um eine Lewis-Säure oder um eine Brønsted-Säure handeln. Die Zugabe einer wässrigen Brønsted-Säure ist bevorzugt.

Bei einer Lewis-Säure handelt es sich beispielsweise um $In(OTf)_3$, $YB(OTf)_3$, $InCl_3$, $ZnCl_2$, $AlCl_3$ oder $FeCl_3$.

Bei einer wässrigen Brønsted-Säure handelt es sich bevorzugt um eine anorganische Brønsted-Säure, eine Mischung aus zwei oder mehr anorganischen Brransted-Säuren, eine organische Brønsted-Säure oder um eine Mischung aus zwei oder mehr organischen Brransted-Säuren.

Eine anorganische Brønsted-Säure ist beispielsweise Salzsäure, Schwefelsäure, Phosphorsäure oder Chlorsäure.

Eine organische Brønsted-Säure ist beispielsweise Methansulfonsäure, p-Toluolsulfonsäure, Essigsäure, Zitronensäure, Ethansulfonsäure, Ameisensäure oder Benzolsulfonsäure.

Es ist bevorzugt, dass es sich bei einer Säure um Schwefelsäure, Salzsäure, Phosphorsäure, Ameisensäure, Essigsäure oder Methansulfonsäure handelt.

Schwefelsäure wird bevorzugt in einer Konzentration von 0,1 bis 5 M eingesetzt.

Salzsäure wird bevorzugt in einer Konzentration von 0,1 bis 12,5 M eingesetzt.

Phosphorsäure wird bevorzugt in einer Konzentration von 0,1 bis 16 M eingesetzt.

Ameisensäure wird bevorzugt in einer Konzentration von 0,1 bis 56 M eingesetzt.

Essigsäure wird bevorzugt in einer Konzentration von 0,1 bis 18 M eingesetzt.

Methansulfonsäure wird bevorzugt in einer Konzentration von 0,1 bis 15,2 M eingesetzt.

Erfolgt die Hydrolyse durch Zugabe einer wässrigen Säure, ist es von Vorteil, die Menge der zugegebenen wässrigen Säure derart zu bemessen, dass sich in der Reaktionsmischung ein pH-Wert von 0 bis 5 einstellt, bevorzugt 0 bis 3. Der angegebene pH-Bereich bringt den Vorteil, dass die Hydrolyse zügig und weitgehend nebenproduktfrei abläuft. Weiterhin bringt der angegebene pH-Bereich den Vorteil, dass das durch Hydrolyse freigesetzte Amin weitgehend vollständig durch Protonierung in die wässrige Phase überführt wird und damit weitgehend vollständig aus der organischen Phase und damit von den Verbindungen der allgemeinen Formel (I) abgetrennt werden kann. Eine weitgehend vollständige Abtrennung des Amins ist dahingehend wichtig, da dieses zu Verfärbungen des aufgearbeiteten Reaktionsprodukts führen kann.

Die Zugabe der wässrigen Säure kann bis zur Einstellung des gewünschten pH-Werts kontinuierlich oder diskontinuierlich erfolgen. Die Zugabe der wässrigen Säure erfolgt bevorzugt nachdem 70 bis 100 Prozent der ursprünglich eingesetzten Verbindungen der allgemeinen Formel (II) zu Verbindungen der allgemeinen Formel (I) umgesetzt sind.

Es ist bevorzugt, dass die Reaktionstemperatur in Schritt b) 0 bis 60 °C beträgt. Es ist weiter bevorzugt, dass die Reaktionstemperatur in Schritt b) 5 bis 50 °C beträgt. Es ist besonders bevorzugt, dass die Reaktionstemperatur in Schritt b) 10 bis 30 °C beträgt.

Generell ist es bevorzugt, dass die Reaktionstemperatur in Schritt a) und b) im Wesentlichen gleich ist. Im Wesentlichen gleich bedeutet in diesem Zusammenhang, dass sich die Reaktionstemperaturen in Schritt a) und b) um maximal 10 °C, bevorzugt um maximal 5 °C unterscheiden.

Die Aufarbeitung der rohen Reaktionsmischung, die nach Schritt b) erhalten wird kann nach dem Fachmann bekannten Methoden erfolgen. Durch das Ergreifen geeigneter verfahrenstechnischer Maßnahmen ist darauf zu achten, dass das durch Hydrolyse gebildete Amin im Zuge der Aufarbeitung der rohen Reaktionsmischung weitgehend vollständig von den Verbindungen der allgemeinen Formel (I) abgetrennt wird.

Es kann bevorzugt sein, dass Schritt a) des erfindungsgemäßen Verfahrens 1, die Umsetzung einer Verbindung der allgemeinen Formel (IV) mit einer Aminverbindung der allgemeinen Formel $H\text{-}NR^{3a}R^{3b}$ vorangeht. Bezüglich bevorzugter Ausführungsformen betreffend die Aminverbindungen bzw. $R^{3a}$ und $R^{3b}$ wird auf die vorherigen Angaben in vollem Umfang Bezug genommen.

Demnach kann es bevorzugt sein, dass ein Verfahren zur Herstellung einer oder mehrerer Verbindungen der allgemeinen Formel (I)

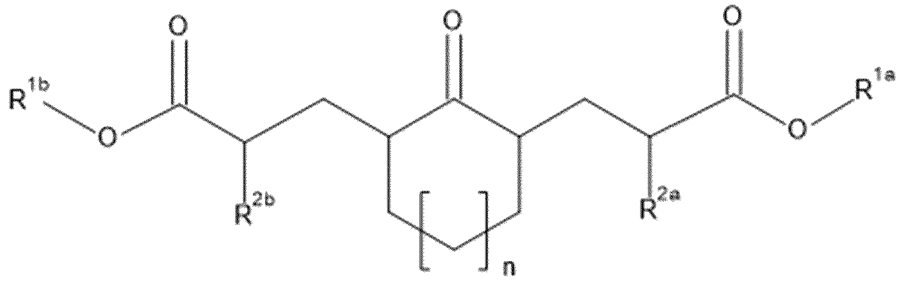

allgemeine Formel (I)

wobei

$R^{1a}$ und $R^{1b}$ unabhängig voneinander $C_8$- bis $C_{10}$-Alkyl sind,
$R^{2a}$ und $R^{2b}$ unabhängig voneinander H oder $CH_3$ sind,
n = 0, 1, 2 oder 7 ist,

dadurch gekennzeichnet ist, dass

a) eine Verbindung der allgemeinen Formel (II)

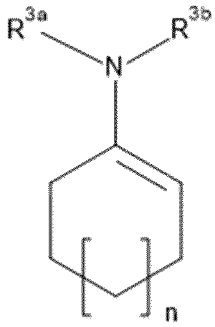

allgemeine Formel (II)

wobei

$R^{3a}$ und $R^{3b}$ unabhängig voneinander $C_1$- bis $C_{10}$-Alkyl sind oder zusammen einen Zyklus mit 4 bis 11 Kohlenstoffatomen bilden, oder
$R^{3a}$ H ist und $R^{3b}$ $C_1$- bis $C_{10}$-Alkyl ist, oder
$R^{3a}$ $C_1$-bis $C_{10}$-Alkyl ist und $R^{3b}$ H ist,
n = 0, 1, 2 oder 7 ist,
mit einer Verbindung der allgemeinen Formel (IIIa), (IIIb) oder einer Mischung aus Verbindungen der allgemeinen Formel (IIIa) und (IIIb)

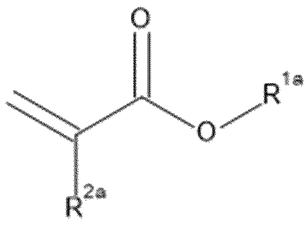

allgemeine Formel (IIIa)

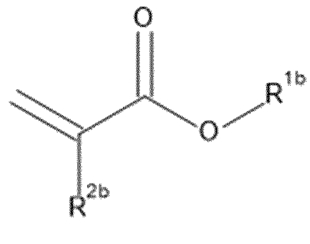

allgemeine Formel (IIIb)

wobei

$R^{1a}$ und $R^{1b}$ unabhängig voneinander $C_8$- bis $C_{10}$-Alkyl sind,
$R^{2a}$ und $R^{2b}$ unabhängig voneinander H oder $CH_3$ sind,
umgesetzt wird,

b) das Reaktionsprodukt aus Schritt a) zu einer Verbindung der allgemeinen Formel (I) hydrolysiert wird,

wobei dem Schritt a) die Umsetzung einer Verbindung der allgemeinen Formel (IV)

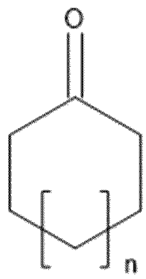

allgemeine Formel (IV)

wobei n = 0, 1, 2 oder 7 ist,

mit einer Aminverbindung der allgemeinen Formel H-$NR^{3a}R^{3b}$ unter Bildung einer Verbindung der allgemeinen Formel (II) vorangeht.

Sämtliche Schritte können separat in unterschiedlichen oder konsekutiv in einem Reaktionsgefäß durchgeführt werden. Die Reaktionsprodukte aus einem Schritt können aufgearbeitet oder nicht aufgearbeitet im nächsten Schritt eingesetzt werden.

Die gemäß Verfahren 1 erhaltene rohe Reaktionsmischung kann neben Verbindungen der allgemeinen Formel (I) auch eine oder mehrere der nachfolgenden Verbindungen als Nebenprodukte enthalten. Die nachfolgenden Verbindungen umfassen die entsprechenden Stereoisomere. Nach Aufarbeitung der rohen Reaktionsmischung können eine oder mehrere der nachfolgenden Verbindungen in Restmengen im Produkt enthalten sein. Die Gesamtmenge der Nebenprodukte in der rohen Reaktionsmischung und/oder im Produkt nach Aufarbeitung der rohen Reaktionsmischung beträgt in der Regel 0 bis 10 Gewichtsprozent bezogen auf die Gesamtmenge der Verbindungen der allgemeinen Formel (I). Sind Nebenprodukte im Produkt enthalten, können diese Einfluss auf die Weichmachereigenschaften haben, beispielsweise auf die Geliertemperatur und/oder Flüchtigkeit.

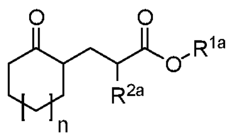
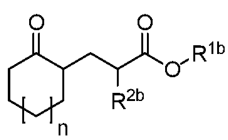
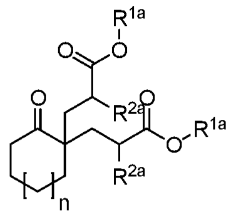
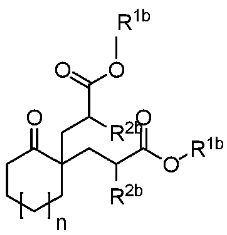
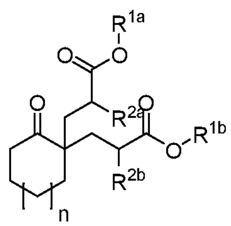

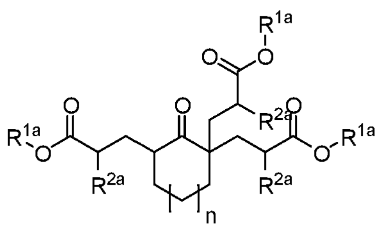
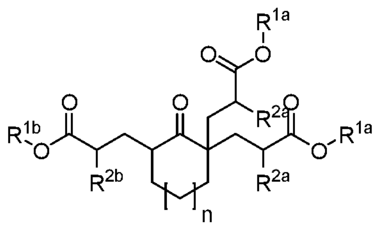
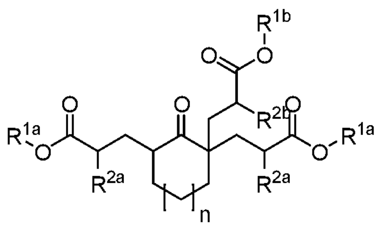

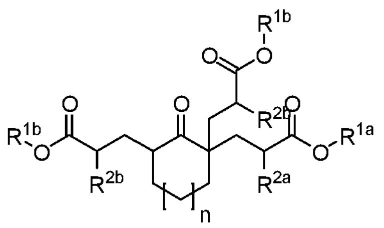
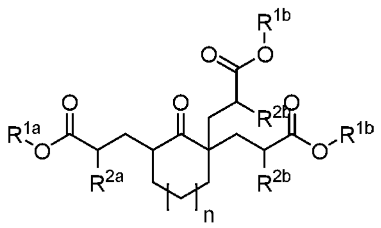
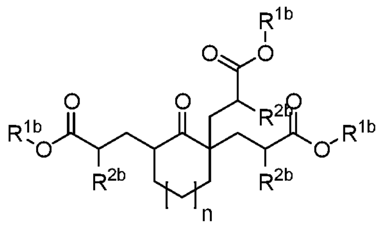

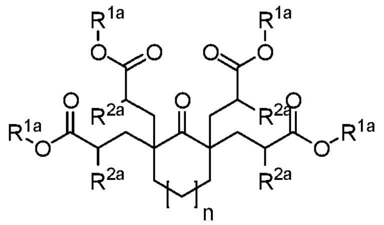
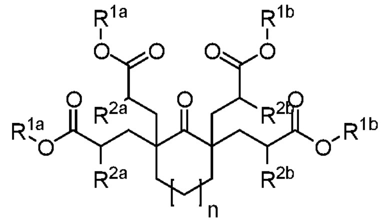
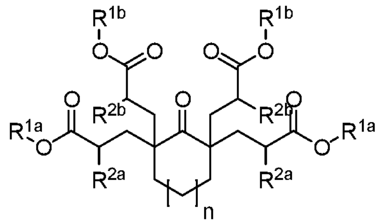

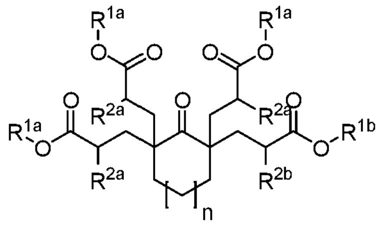
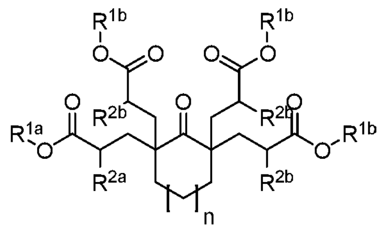
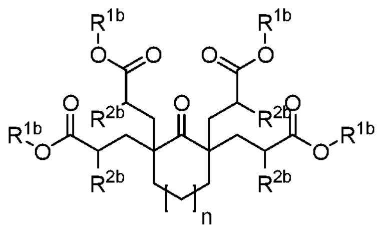

Verfahren 2:

Verfahren zur Herstellung einer oder mehrerer Verbindungen der allgemeinen Formel (I)

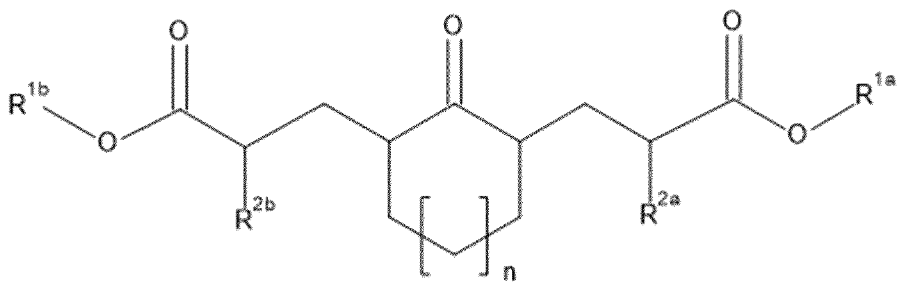

allgemeine Formel (I)

wobei

$R^{1a}$ und $R^{1b}$ unabhängig voneinander $C_5$- bis $C_{13}$-Alkyl sind,
$R^{2a}$ und $R^{2b}$ unabhängig voneinander H oder $CH_3$ sind,

n = 0, 1, 2 oder 7 ist,

das dadurch gekennzeichnet ist, dass

eine Verbindung der allgemeinen Formel (V) oder ein Säureanhydrid einer Verbindung der allgemeinen Formel (V) oder ein Säurehalogenid einer Verbindung der allgemeinen Formel (V)

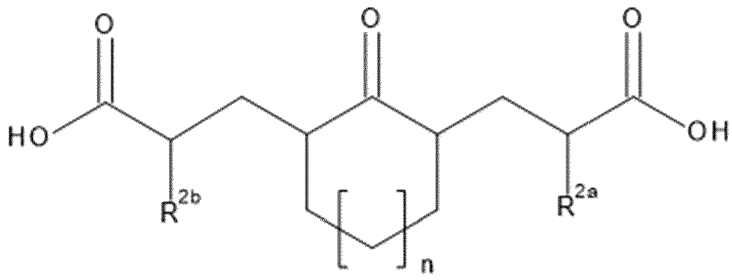

allgemeine Formel (V)

wobei

$R^{2a}$ und $R^{2b}$ unabhängig voneinander H oder $CH_3$ sind,
und

n = 0, 1, 2 oder 7 ist,

mit Alkoholen der allgemeinen Formel $R^{1a}$-OH und $R^{1b}$-OH, in Gegenwart eines Veresterungskatalysators umgesetzt wird, wobei $R^{1a}$ und $R^{1b}$ unabhängig voneinander $C_8$- bis $C_{10}$-Alkyl sind.

Bezüglich bevorzugter Ausführungsformen für $R^{1a}$, $R^{1b}$, $R^{2a}$, $R^{2b}$ und/oder n wird auf die vorherigen Angaben in vollem Umfang Bezug genommen.

Verbindungen der allgemeinen Formel (V) sind dem Fachmann bekannt und können nach dem Fachmann bekannten Methoden hergestellt werden (siehe beispielsweise JP49-85136).

Bei einem Säurehalogenid einer Verbindung der allgemeinen Formel (V) handelt es sich bevorzugt um ein Säurechlorid oder Säurebromid, wobei ein Säurechlorid besonders bevorzugt ist.

Als Veresterungskatalysator können die dafür üblichen Katalysatoren eingesetzt werden.

Handelt es sich bei einer Verbindung der allgemeinen Formel (V) um eine Dicarbonsäure werden als Veresterungskatalysatoren anorganische Brønstedsäuren, organische Brønsted-säuren, amphotere Katalysatoren oder ionische Flüssigkeiten eingesetzt, wobei amphotere Katalysatoren bevorzugt sind.

Handelt es sich bei einer Verbindung der allgemeinen Formel (V) um ein Säureanhydrid oder um ein Säurehalogenid werden als Veresterungskatalysatoren anorganische Base, organische Basen oder amphotere Katalysatoren eingesetzt, wobei amphotere Katalysatoren bevorzugt sind.

Bei anorganischen Brønsted-Säuren handelt es sich beispielweise um Salzsäure, Schwefelsäure oder Phosphorsäure.

Bei organischen Brønsted-Säuren handelt es sich beispielsweise um Methansulfonsäure, Ethansulfonsäure, Benzolsulfonsäure oder p-Toluolsulfonsäure.

Bei anorganischen Basen handelt es sich beispielsweise um Alkali- oder Erdalkalihydroxide, Alkali- oder Erdalkalicarbonate, Alkali- oder Erdalkalihydrogencarbonate, Alkali- oder Erdalkaliphosphate oder Alkali- oder Erdalkalihydroxycarbonate. Alkalihydroxide sind beispielsweise Natriumhydroxid oder Kaliumhydroxid.

Bei organischen Basen handelt es sich beispielsweise um Salze von schwachen organischen Säuren, wie Acetate oder Formiate.

Bei amphoteren Katalysatoren handelt es sich beispielsweise um Titan-, Zinn (IV)- oder Zirkoniumverbindungen. Bevorzugte amphotere Katalysatoren sind Titantetraalkoxytitanate, Titanacetylacetonat oder Zinnoxid. Besonders bevorzugte amphotere Katalysatoren sind Isopropyl-n-butyltitanat, Tetra-isopropyltitanat, Tetra-butyltitanat oder eine Mischung aus zwei oder mehr der zuvor genannten Katalysatoren. Bei ionischen Flüssigkeiten handelt es sich beispielweise um Methylimidazoliumbutansulfonsäure-triflat oder 1-Ethyl-3-methyl-imidazolium-hydrogensulfat.

Es ist bevorzugt, dass das Molverhältnis zwischen Verbindungen der allgemeinen Formel (V) und der Gesamtmenge des eingesetzten Veresterungskatalysators 1 : 0,001 bis 1 : 0,5 beträgt.

Die Veresterung kann in Gegenwart oder in Abwesenheit eines Lösungsmittels durchgeführt werden. Bei einem Lösungsmittel handelt es sich um eine chemische Einzelverbindung oder um eine Mischung chemischer Einzelverbindungen.

Wird die Veresterung in Gegenwart eines Lösungsmittels durchgeführt, eignen sich prinzipiell alle Lösungsmittel die keine unerwünschten Nebenreaktionen mit den eingesetzten Edukten, Produkten, Reagenzien und/oder dem Veresterungskatalysator eingehen und in denen die Edukte, Produkte, Reagenzien und/oder der Veresterungskatalysator

eine ausreichende Löslichkeit aufweisen. Es ist zudem bevorzugt, dass das Lösungsmittel ein Azeotrop mit Wasser bildet.

Geeignete Lösungsmittel sind beispielsweise chlorierte Kohlenwasserstoffe wie Dichlormethan oder Chlorbenzol, Cyclohexan, Methylcyclohexan, Ether wie Tetrahydrofuran, Dioxan oder MTBE, Nitrile wie Acetonitril, Benzol, Toluol, Xylol, Cumol oder eine beliebige Mischung aus zwei oder mehr der zuvor genannten Lösungsmittel. Es ist bevorzugt, dass das eingesetzte Lösungsmittel ein Azeotrop mit Wasser bildet.

Die Veresterung kann bei Umgebungsdruck oder vermindertem oder erhöhtem Druck erfolgen.

Die Veresterung erfolgt bevorzugt bei einer Temperatur von 10 bis 200 °C, insbesondere bei einer Temperatur von 90 bis 180°C.

Die Veresterung kann in Abwesenheit oder in Gegenwart eines Inertgases erfolgen. Unter einem Inertgas wird ein Gas verstanden, welches unter den gegebenen Reaktionsbedingungen keine Reaktion mit den Edukten, Produkten, Reagenzien, Veresterungskatalysator, und/oder wenn ein Lösungsmittel eingesetzt wird, mit dem Lösungsmittel eingeht. Bei einem Inertgas handelt es sich bevorzugt um Stickstoff.

Es ist bevorzugt, dass das Molverhältnis zwischen Verbindungen der allgemeinen Formel (V) und der Gesamtmenge der eingesetzten Alkohole der allgemeinen Formel $R^{1a}$-OH und $R^{1b}$-OH, 1 : 0,5 bis 1 : 400 beträgt. Es ist weiter bevorzugt, dass das Molverhältnis 1 : 1 bis 1: 4 beträgt. Es ist besonders bevorzugt, dass das Molverhältnis 1 : 2 bis 1 : 2,5 beträgt. Das Molverhältnis von $R^{1a}$-OH zu $R^{1b}$-OH kann über weite Bereiche variieren. So kann das Molverhältnis von $R^{1a}$-OH zu $R^{1b}$-OH beispielsweise 100:1 bis 1:100 betragen.

Es ist zweckdienlich, das bei der Veresterung entstehende Wasser mittels der üblichen Maßnahmen, beispielsweise durch Destillation, aus der Reaktion zu entfernen.

Bezüglich spezifischer Verfahrensausgestaltungen zu Verfahren 2 orientiert sich der Fachmann am Stand der Technik zu Veresterungsreaktionen. So beschreibt beispielsweise die WO 02/038531 ein Verfahren zur Herstellung von Estern, bei dem man a) in einer Reaktionszone ein im Wesentlichen aus der Säurekomponente oder einem Anhydrid davon und der Alkoholkomponente bestehendes Gemisch in Gegenwart eines Veresterungskatalysators zum Sieden erhitzt, b) die Alkohol und Wasser enthaltenden Dämpfe rektifikativ in eine alkoholreiche Fraktion und eine wasserreiche Fraktion auftrennt, c) die alkoholreiche Fraktion in die Reaktionszone zurückführt und die wasserreiche Fraktion aus dem Verfahren ausleitet. Als Veresterungskatalysatoren eignen sich beispielsweise die zuvor aufgeführten Veresterungskatalysatoren. Weitere detaillierte Darstellungen zur Durchführung von Veresterungsreaktionen finden sich beispielsweise in der US 6,310,235, US 5,324,853, DE-A 2612355 oder DE-A 1945359.

Die Aufarbeitung der rohen Reaktionsmischung kann nach dem Fachmann bekannten Methoden erfolgen.

Generell kann die Veresterung auch enzymatisch erfolgen. Als Enzyme eignen sich beispielsweise Lipasen wie Novozym 435. Bei einer enzymatischen Veresterung ist eine Veresterung mit linearen Alkoholen bevorzugt. Reaktionstemperatur und Lösungsmittel sind an das jeweils verwendete Enzym anzupassen.

Die gemäß Verfahren 2 erhaltene rohe Reaktionsmischung kann neben Verbindungen der allgemeinen Formel (I) auch eine oder mehrere der nachfolgenden Verbindungen als Nebenprodukte enthalten. Die nachfolgenden Verbindungen umfassen die entsprechenden Stereoisomere. Nach Aufarbeitung der rohen Reaktionsmischung können eine oder mehrere der nachfolgenden Verbindungen in Restmengen im Produkt enthalten sein. Die Gesamtmenge der Nebenprodukte in der rohen Reaktionsmischung und/oder im Produkt nach Aufarbeitung der rohen Reaktionsmischung beträgt in der Regel 0 bis 10 Gewichtsprozent bezogen auf die Gesamtmenge der Verbindungen der allgemeinen Formel (I). Sind Nebenprodukte im Produkt enthalten, können diese Einfluss auf die Weichmachereigenschaften haben, beispielsweise auf die Geliertemperatur und/oder Flüchtigkeit.

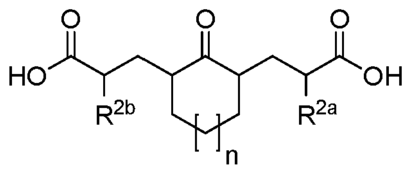

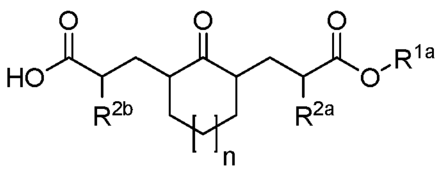

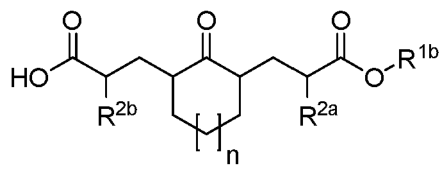

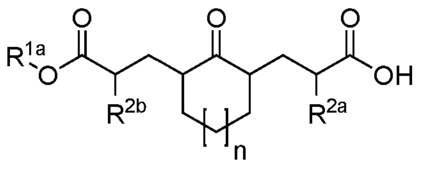

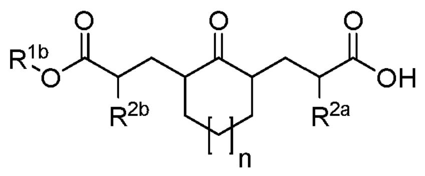

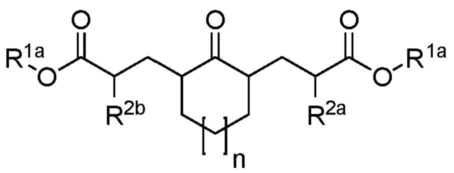

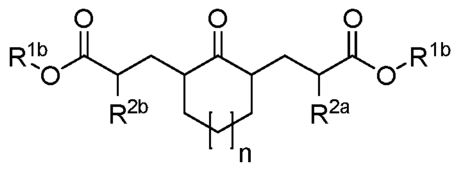

Sind $R^{1a}$ und $R^{1b}$ gleich, handelt es sich bei den dargestellten Diestern nicht um Nebenprodukte.

Verfahren 3:

Verfahren zur Herstellung einer oder mehrerer Verbindungen der allgemeinen Formel (I)

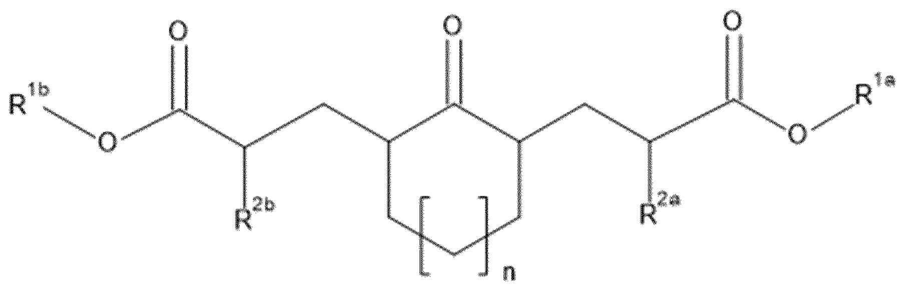

allgemeine Formel (I)

wobei

$R^{1a}$ und $R^{1b}$ unabhängig voneinander $C_5$- bis $C_{13}$-Alkyl sind,
$R^{2a}$ und $R^{2b}$ unabhängig voneinander H oder $CH_3$ sind,

n = 0, 1, 2 oder 7 ist,
das dadurch gekennzeichnet ist, dass
eine Verbindung der allgemeinen Formel (VI)

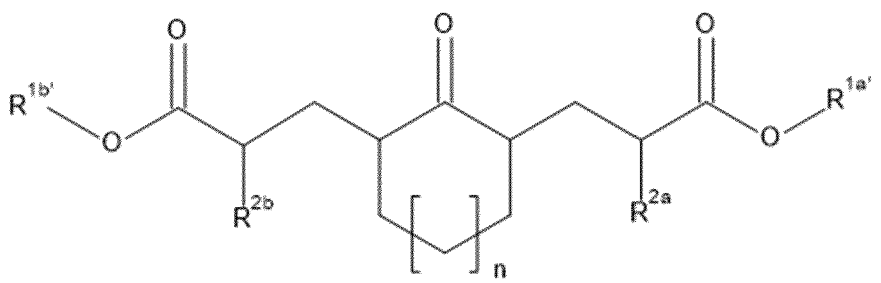

allgemeine Formel (VI)

wobei
$R^{1a'}$ und $R^{1b'}$ unabhängig voneinander Methyl, Ethyl, n-Propyl, iso-Propyl, n-Butyl, iso-Butyl, oder tert-Butyl sind,

$R^{2a}$ und $R^{2b}$ unabhängig voneinander H oder $CH_3$ sind,
n = 0, 1, 2 oder 7 ist,

mit Alkoholen der allgemeinen Formel $R^{1a}$-OH und $R^{1b}$-OH in Gegenwart eines Umesterungskatalysators umgesetzt wird, wobei $R^{1a}$ und $R^{1b}$ unabhängig voneinander $C_8$- bis $C_{10}$-Alkyl sind.

Bezüglich bevorzugter Ausführungsformen für $R^{1a}$, $R^{1b}$, $R^{2a}$, $R^{2b}$ und/oder n wird auf die vorherigen Angaben in vollem Umfang Bezug genommen.

Verbindungen der allgemeinen Formel (VI) sind dem Fachmann bekannt und können nach dem Fachmann bekannten Methoden hergestellt werden (siehe beispielsweise G. Stork et al. J. Am. Chem. Soc., 85, 207- 222 (1963), oder N. Matsumoto et al., Journal of Polymer Science: Polymer Chemistry Edition, 18, 1665-1678 (1980)).

In einer Verbindung der allgemeinen Formel (VI) sind $R^{1a'}$ und $R^{1b'}$ unabhängig voneinander Methyl, Ethyl, n-Propyl, iso-Propyl, n-Butyl, iso-Butyl oder tert-Butyl. Es ist bevorzugt, dass $R^{1a'}$ und $R^{1b'}$ gleich und Methyl oder Ethyl sind.

Als Umesterungskatalysator können die für Umesterungsreaktionen üblichen Katalysatoren eingesetzt werden, z.B. anorganische Säuren wie Schwefelsäure oder Phosphorsäure; organische Sulfonsäuren, wie Methansulfonsäure oder p-Toluolsulfonsäure; oder spezielle Metallkatalysatoren aus der Gruppe der Zinn(IV)-Katalysatoren, beispielsweise Dialkylzinndicarboxylate, wie Dibutylzinndiacetat; Trialkylzinnalkoxide; Monoalkylzinnverbindungen wie Monobutylzinnoxid; Zinnsalze wie Zinnacetat; oder Zinnoxide; aus der Gruppe der Titankatalysatoren, monomere oder polymere Titanate oder Titanchelate wie Tetraethylorthotitanat, Tetrapropylorthotitanat, Tetrabutylorthotitanat, Triethanolamintitanat; aus der Gruppe der Zirkonkatalysatoren, Zirkonate oder Zirkonchelate wie Tetrapropylzirkonat, Tetrabutylzirkonat, Triethanolaminzirkonat; sowie Lithiumkatalysatoren wie Lithiumsalze, Lithiumalkoxide; oder Kalium-, Magnesium- oder Cersalze wie Kaliumphosphat, oder Aluminium(III)-, Chrom(III)-, Eisen(III)-, Kobalt(II)-, Nickel(II)- und Zink(II)-acetylacetonat. Bevorzugte Umsterungskatalysatoren sind Isopropyl-n-butyltitanat, Tetra-isopropyltitanat, Tetra-butyltitanat oder eine Mischung aus zwei oder mehr dieser Katalysatoren.

Es ist bevorzugt, dass das Molverhältnis zwischen Verbindungen der allgemeinen Formel (VI) und der Gesamtmenge des eingesetzten Umesterungskatalysators 1 : 0,001 bis 1 : 0,5 beträgt.

Die Umesterung kann in Gegenwart oder in Abwesenheit eines Lösungsmittels durchgeführt werden. Bei einem Lösungsmittel handelt es sich um eine chemische Einzelverbindung oder um eine Mischung chemischer Einzelverbindungen.

Wird die Umesterung in Gegenwart eines Lösungsmittels durchgeführt, eignen sich prinzipiell alle Lösungsmittel die keine unerwünschten Nebenreaktionen mit den eingesetzten Edukten, Produkten, Reagenzien und/oder dem Umesterungskatalysator eingehen und in denen die Edukte, Produkte, Reagenzien und/oder der Umesterungskatalysator eine ausreichende Löslichkeit aufweisen.

Geeignete Lösungsmittel sind beispielsweise chlorierte Kohlenwasserstoffe wie Dichlormethan oder Chlorbenzol, Cyclohexan, Methylcyclohexan, Ether wie Tetrahydrofuran, Dioxan oder MTBE, Nitrile wie Acetonitril, Benzol, Toluol, Xylol, Cumol oder eine beliebige Mischung aus zwei oder mehr der zuvor genannten Lösungsmittel.

Die Umesterung kann bei Umgebungsdruck oder vermindertem oder erhöhtem Druck erfolgen. So wird die Umesterung bevorzugt bei einem Druck von 0,001 bis 200 bar und weiter bevorzugt bei einem Druck von 0,01 bis 5 bar durchgeführt.

Die Umesterung erfolgt bevorzugt bei einer Temperatur von 10 bis 200 °C und bevorzugt bei einer Temperatur von 90 bis 180°C.

Die Umesterung kann in Abwesenheit oder in Gegenwart eines Inertgases erfolgen. Unter einem Inertgas wird ein Gas verstanden, welches unter den gegebenen Reaktionsbedingungen keine Reaktion mit den Edukten, Produkten, Reagenzien, Umesterungskatalysator, und/oder wenn ein Lösungsmittel eingesetzt wird, mit dem Lösungsmittel eingeht. Bei einem Inertgas handelt es sich bevorzugt um Stickstoff.

Es ist bevorzugt, dass das Molverhältnis zwischen Verbindungen der allgemeinen Formel (VI) und der Gesamtmenge der eingesetzten Alkohole $R^{1a}$-OH und $R^{1b}$-OH, , 1 : 0,5 bis 1 : 400 beträgt. Es ist weiter bevorzugt, dass das Molverhältnis 1 : 1 bis 1: 4 beträgt. Es ist besonders bevorzugt, dass das Molverhältnis 1 : 2 bis 1 : 2,5 beträgt. Das Molverhältnis von $R^{1a}$-OH zu $R^{1b}$-OH kann über weite Bereiche variieren. So kann das Molverhältnis von $R^{1a}$-OH zu $R^{1b}$-OH beispielsweise 100:1 bis 1:100 betragen.

Der bei der Umesterung freigesetzte, niedriger siedende Alkohol kann zwecks Verschiebung des Gleichgewichts aus der Umesterungsreaktion kontinuierlich abdestilliert werden. Die hierzu benötigte Destillationskolonne steht in der Regel in direkter Verbindung mit dem Umesterungsreaktor. Beispielsweise kann die Destillationskolonne direkt am Umesterungsreaktor installiert sein. Im Falle der Verwendung mehrerer, in Serie geschalteter, Umesterungsreaktoren kann jeder dieser Reaktoren mit einer Destillationskolonne ausgerüstet sein.

Wird für die Umesterung eine Kaskade von Umesterungsreaktoren verwendet kann es bevorzugt sein, das abgedampfte Alkoholgemisch über eine oder mehrere Sammelleitungen einer Destillationskolonne zuzuführen.

Der bei der Destillation zurückgewonnene höhersiedende Alkohol wird vorzugsweise wieder in die Umesterung

zurückgeführt.

Die Aufarbeitung der rohen Reaktionsmischung kann nach dem Fachmann bekannten Methoden erfolgen.

Die gemäß Verfahren 3 erhaltene rohe Reaktionsmischung kann neben Verbindungen der allgemeinen Formel (I) auch eine oder mehrere der nachfolgenden Verbindungen als Nebenprodukte enthalten. Die nachfolgenden Verbindungen umfassen die entsprechenden Stereoisomere. Nach Aufarbeitung der rohen Reaktionsmischung können eine oder mehrere der nachfolgenden Verbindungen in Restmengen im Produkt enthalten sein. Die Gesamtmenge der Nebenprodukte in der rohen Reaktionsmischung und/oder im Produkt nach Aufarbeitung der rohen Reaktionsmischung beträgt in der Regel 0 bis 10 Gewichtsprozent bezogen auf die Gesamtmenge der Verbindungen der allgemeinen Formel (I). Sind Nebenprodukte im Produkt enthalten, können diese Einfluss auf die Weichmachereigenschaften haben, beispielsweise auf die Geliertemperatur und/oder Flüchtigkeit.

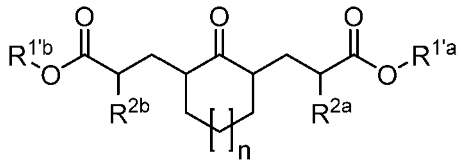
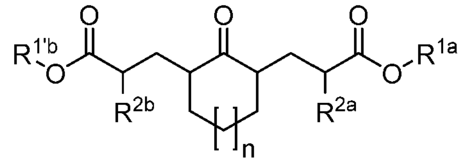

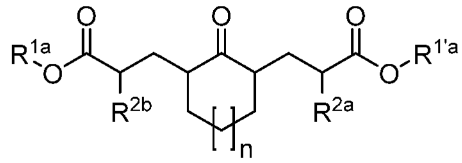
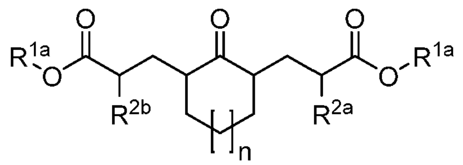

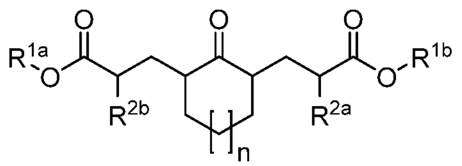
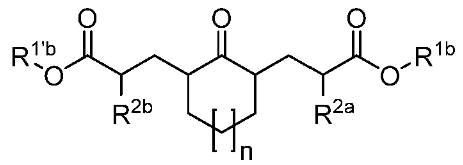

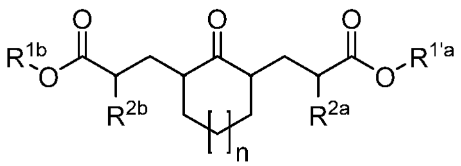
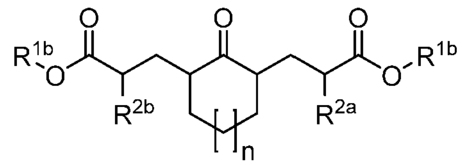

Sind $R^{1a}$ und $R^{1b}$ gleich handelt es sich bei den Diestern in denen beide Alkyl-Estergruppen $R^{1a}$ oder beide Alkyl-Estergruppen $R^{1b}$ sind, nicht um Nebenprodukte.

<u>Weichmacher-Zusammensetzung</u>

Gegenstand der vorliegenden Erfindung ist auch eine Weichmacher-Zusammensetzung, die eine oder mehrere Verbindungen der allgemeinen Formel (I) und einen oder mehrere Weichmacher, die von den Verbindungen der allgemeinen Formel (I) unterschiedlich sind, enthält. Ein oder mehrere von Formel (I) unterschiedliche Weichmacher können dazu genutzt werden die Weichmachereigenschaften der Weichmacher-Zusammensetzung bei deren Verwendung in unterschiedlichen Polymeren an verschiedene Bedingungen anzupassen. So kann die Weichmacher-Zusammensetzung beispielsweise einen oder mehrere von Formel (I) unterschiedliche Weichmacher enthalten, die die Geliertemperatur des weichgemachten Polymers weiter herabsetzen, die Kältebruchtemperatur verbessern, die Verträglichkeit mit dem weichzumachenden Polymer erhöhen und/oder das Migrationsverhalten in den weichgemachten Polymer verbessern. Weiterhin können beispielsweise folgende Eigenschaften beeinflusst werden, Flüchtigkeit, Shore A Härte, mechanische Eigenschaften, wie Zugfestigkeit, Witterungsbeständigkeit, Extraktionsverhalten, Thermostabilität, Flammbeständigkeit, mikrobiologisches Abbauverhalten und/oder die Viskosität.

Bezüglich bevorzugter Ausführungsformen für $R^{1a}$, $R^{1b}$, $R^{2a}$, $R^{2b}$ und/oder n wird auf die vorherigen Angaben in vollem Umfang Bezug genommen.

Weichmacher, die von Verbindungen der allgemeinen Formel (I) unterschiedlich sind, sind beispielsweise

Cyclohexan-1,2-dicarbonsäuredialkylester, Cyclohexan-1,3-dicarbonsäuredialkylester, Cyclohexan-1,4-dicarbonsäuredialkylester, Terephthalsäuredialkylester, Phthalsäuredialkylester, Äpfelsäuredialkylester, Acetyl-Äpfelsäuredialkylester, Trimellitsäuretrialkylester, Benzoesäurealkylester, Dibenzoesäureester, tetra-Ester von Pentaerythrit, Monocarbonsäurealkylester wie gesättigte oder ungesättigte Monocarbonsäurealkylester, Dicarbonsäuredialkylester wie gesättigte oder ungesättigte Dicarbonsäuredialkylester, aromatische Sulfonsäureester, Alkylsulfonsäureester, Glycerinester, Isosorbidester, Phosphorsäureester, Citronensäuretriester, Alkylpyrrolidonderivate, 2,5-Furandicarbonsäuredialkylester, 2,5-Tetrahydrofurandicarbonsäuredialkylester, Polyester aus aromatischen und/oder aliphatischen Polycarbonsäuren mit mindestens zweiwertigen Alkoholen, epoxidiertes Pflanzenöl oder epoxidierte Fettsäuremonoalkylester.

Cyclohexan-1,2-dicarbonsäuredialkylester weisen bevorzugt 4 bis 13 Kohlenstoffatome in den Alkylresten auf. Die Alkylreste können unabhängig voneinander unterschiedlich oder gleich sein, wobei es bevorzugt ist, dass die Alkylreste gleich sind. Das cis/trans Verhältnis der Substituenten in Cyclohexan-1,2-dicarbonsäuredialkylester kann bedingt durch das Herstellverfahren und/oder durch Aufreinigung variieren. Übliche cis/trans Verhältnisse liegen im Bereich von 90/10 bis 10/90. Bevorzugte Cyclohexan-1,2-dicarbonsäuredialkylester sind beispielsweise Cyclohexan-1,2-dicarbonsäuredi-n-butylester, Cyclohexan-1,2-dicarbonsäuredi-iso-butylester, Cyclohexan-1,2-dicarbonsäuredi-n-pentylester, Cyclohexan-1,2-dicarbonsäuredi-iso-pentylester, Cyclohexan-1,2-dicarbonsäuredi-n-hexylester, Cyclohexan-1,2-dicarbonsäuredi-cyclohexylester, Cyclohexan-1,2-dicarbonsäuredi-n-heptylester, Cyclohexan-1,2-dicarbonsäuredi-n-octylester, Cyclohexan-1,2-dicarbonsäuredi-iso-octylester, Cyclohexan-1,2-dicarbonsäuredi-(2-ethylhexyl)ester, Cyclohexan-1,2-dicarbonsäuredi-n-nonylester, Cyclohexan-1,2-dicarbonsäu-redi-iso-nonylester, Cyclohexan-1,2-dicarbonsäuredi-(2-propylhexyl)ester, Cyclohexan-1,2-di-carbonsäuredi-n-decylester, Cyclohexan-1,2-dicarbonsäuredi-iso-decylester, Cyclohexan-1,2-dicarbonsäuredi-(2-propylheptyl)ester, Cyclohexan-1,2-dicarbonsäuredi-n-undecylester, Cyclohexan-1,2-dicarbonsäuredi-iso-undecylester, Cyclohexan-1,2-dicarbonsäuredi-n-dodecylester, Cyclohexan-1,2-dicarbonsäuredi-iso-dodecylester, Cyclohexan-1,2-dicarbonsäuredi-n-tridecylester oder Cyclohexan-1,2-dicarbonsäuredi-iso-tridecylester.

Cyclohexan-1,2-dicarbonsäuredialkylester weisen weiter bevorzugt 8 bis 10 Kohlenstoffatome in den Alkylresten auf. Die Alkylreste können unabhängig voneinander unterschiedlich oder gleich sein, wobei es bevorzugt ist, dass die Alkylreste gleich sind. Weiter bevorzugte Cyclohexan-1,2-dicarbonsäuredialkylester sind beispielsweise Cyclohexan-1,2-dicarbonsäuredi-n-octylester, Cyclohexan-1,2-dicarbonsäuredi-iso-octylester, Cyclohexan-1,2-dicarbonsäuredi-(2-ethylhexyl)ester, Cyclohexan-1,2-dicarbonsäuredi-n-nonylester, Cyclohexan-1,2-dicarbon-säuredi-iso-nonylester, Cyclohexan-1,2-dicarbonsäuredi-(2-propylhexyl)ester, Cyclohexan-1,2-dicarbonsäuredi-n-decylester, Cyclohexan-1,2-dicarbonsäuredi-iso-decylester oder Cyclohexan-1,2-dicarbonsäuredi-(2-propylheptyl)ester.

Besonders bevorzugte Cyclohexan-1,2-dicarbonsäuredialkylester sind Cyclohexan-1,2-dicar-bonsäuredi-n-nonylester, Cyclohexan-1,2-dicarbonsäuredi-iso-nonylester oder Cyclohexan-1,2-dicarbonsäuredi-(2-propylheptyl)ester.

Cyclohexan-1,3-dicarbonsäuredialkylester weisen bevorzugt 4 bis 13 Kohlenstoffatome in den Alkylresten auf. Die Alkylreste können unabhängig voneinander unterschiedlich oder gleich sein, wobei es bevorzugt ist, dass die Alkylreste gleich sind.

Cyclohexan-1,4-dicarbonsäuredialkylester weisen bevorzugt 4 bis 13 Kohlenstoffatome in den Alkylresten auf. Das cis/trans Verhältnis der Substituenten in Cyclohexan-1,4-dicarbonsäuredialkylester kann bedingt durch das Herstellverfahren und/oder durch Aufreinigung variieren. Übliche cis/trans Verhältnisse liegen im Bereich von 90/10 bis 10/90. Die Alkylreste können unabhängig voneinander unterschiedlich oder gleich sein, wobei es bevorzugt ist, dass die Alkylreste gleich sind. Bevorzugte Cyclohexan-1,4-dicarbonsäuredialkylester sind beispielsweise Cyclohexan-1,4-dicarbonsäuredi-n-butylester, Cyclohexan-1,4-dicarbonsäuredi-iso-butylester, Cyclohexan-1,4-dicarbonsäuredi-n-pentylester, Cyclohexan-1,4-dicarbonsäuredi-iso-pentylester, Cyclohexan-1,4-dicarbonsäuredi-n-hexylester, Cyclohexan-1,4-dicarbonsäuredi-cyclohexylester, Cyclohexan-1,4-dicarbonsäuredi-n-heptylester, Cyclohexan-1,4-dicarbonsäuredi-n-octylester, Cyclohexan-1,4-dicarbonsäuredi-iso-octylester, Cyclohexan-1,4-dicarbonsäuredi-(2-ethylhexyl)ester, Cyclohexan-1,4-dicarbonsäuredi-n-nonylester, Cyclohexan-1,4-dicarbonsäuredi-iso-nonylester, Cyclohexan-1,4-dicarbonsäuredi-(2-propylhexyl)ester, Cyclohexan-1,4-dicarbonsäu-redi-n-decylester, Cyclohexan-1,4-dicarbonsäuredi-iso-decylester, Cyclohexan-1,4-dicarbon-säuredi-(2-propylheptyl)ester, Cyclohexan-1,4-dicarbonsäuredi-n-undecylester, Cyclohexan-1,4-dicarbonsäuredi-iso-undecylester, Cyclohexan-1,4-dicarbonsäuredi-n-dodecylester, Cyclohexan-1,4-dicarbonsäuredi-iso-dodecylester, Cyclohexan-1,4-dicarbonsäuredi-n-tridecylester oder Cyclohexan-1,4-dicarbonsäuredi-iso-tridecylester.

Cyclohexan-1,4-dicarbonsäuredialkylester weisen weiter bevorzugt 8 bis 10 Kohlenstoffatome in den Alkylresten auf. Die Alkylreste können unabhängig voneinander unterschiedlich oder gleich sein, wobei es bevorzugt ist, dass die Alkylreste gleich sind. Weiter bevorzugte Cyclohexan-1,4-dicarbonsäuredialkylester sind beispielsweise Cyclohexan-1,4-dicarbonsäuredi-n-octylester, Cyclohexan-1,4-dicarbonsäuredi-iso-octylester, Cyclohexan-1,4-dicarbonsäuredi-(2-ethylhexyl)ester, Cyclohexan-1,4-dicarbonsäuredi-n-nonylester, Cyclohexan-1,4-dicarbonsäu-redi-iso-nonylester, Cyclohexan-1,4-dicarbonsäuredi-(2-propylhexyl)ester, Cyclohexan-1,4-di-carbonsäuredi-n-decylester, Cyclohexan-1,4-dicarbonsäuredi-iso-decylester oder Cyclohexan-1,4-dicarbonsäuredi-(2-propylheptyl)ester.

Besonders bevorzugte Cyclohexan-1,4-dicarbonsäuredialkylester sind Cyclohexan-1,4-dica-bonsäuredi-(2-ethylhexyl)ester, Cyclohexan-1,4-dicarbonsäuredi-n-nonylester, Cyclohexan-1,4-dicarbonsäuredi-iso-nonylester oder Cyclohexan-1,4-dicarbonsäuredi-(2-propylheptyl)ester.

Terephthalsäuredialkylester weisen bevorzugt 4 bis 13 Kohlenstoffatome in den Alkylresten auf. Die Alkylreste können unabhängig voneinander unterschiedlich oder gleich sein, wobei es bevorzugt ist, dass die Alkylreste gleich sind. Bevorzugte Terephthalsäuredialkylester sind beispielsweise Terephthalsäuredi-n-butylester, Terephthalsäuredi-iso-butyl-ester, Terephthalsäure-n-butyl-iso-butylester, Terephthalsäuredi-n-pentylester, Terephthalsäure-di-iso-pentylester, Terephthalsäuredi-n-hexylester, Terephthalsäuredi-cyclohexylester, Terephthalsäuredi-n-heptylester, Terephthalsäuredi-n-octylester, Terephthalsäuredi-iso-octylester, Terephthalsäuredi-(2-ethylhexyl)ester, Terephthalsäuredi-n-nonylester, Terephthalsäuredi-iso-nonylester, Terephthalsäuredi-(2-propylhexyl)ester, Terephthalsäuredi-n-decylester, Terephthalsäuredi-iso-decylester, Terephthalsäuredi-(2-propylhexyl)ester, Terephthalsäuredi-n-undecylester, Terephthalsäuredi-iso-undecylester, Terephthalsäuredi-n-dodecylester, Terephthalsäuredi-iso-dodecylester, Terephthalsäure-n-tridecylester oder Terephthalsäuredi-iso-tridecylester.

Terephthalsäuredialkylester weisen weiter bevorzugt 4, 5 oder 8 bis 10 Kohlenstoffatome in den Alkylresten auf. Die Alkylreste können unabhängig voneinander unterschiedlich oder gleich sein, wobei es bevorzugt ist, dass die Alkylreste gleich sind. Weiter bevorzugte Terephthalsäuredialkylester sind Terephthalsäuredi-n-butylester, Terephthalsäuredi-iso-butyl-ester, Terephthalsäure-n-butyl-iso-butylester, Terephthalsäuredi-iso-pentylester, Terephthalsäuredi-n-octylester, Terephthalsäuredi-iso-octylester, Terephthalsäuredi-(2-ethylhexyl)ester, Terephthalsäuredi-n-nonylester, Terephthalsäuredi-iso-nonylester, Terephthalsäuredi-(2-propylhexyl)ester, Terephthalsäuredi-n-decylester oder Terephthalsäuredi-iso-decylester.

Besonders bevorzugte Terephthalsäuredialkylester sind Terephthalsäuredi-n-butylester, Terephthalsäuredi-iso-butyl-ester, Terephthalsäuredi-iso-pentylester oder Terephthalsäuredi-(2-ethylhexyl)ester.

Phthalsäuredialkylester weisen bevorzugt 9 bis 13 Kohlenstoffatome in den Alkylresten auf. Die Alkylreste können unabhängig voneinander unterschiedlich oder gleich sein, wobei es bevorzugt ist, dass die Alkylreste gleich sind. Bei einem bevorzugten Phthalsäuredialkylester handelt es sich beispielsweise um Di-isononylphthalat oder Di-2-propylheptylphthalat.

Äpfelsäuredialkylester oder Acetyl-Äpfelsäuredialkylester weisen bevorzugt 4 bis 13 Kohlenstoffatome in den Alkylresten auf. Die Alkylreste können unabhängig voneinander unterschiedlich oder gleich sein, wobei es bevorzugt ist, dass die Alkylreste gleich sind.

Trimellitsäuretrialkylester weisen bevorzugt 4 bis 13 Kohlenstoffatome in den Alkylresten auf. Die Alkylreste können unabhängig voneinander unterschiedlich oder gleich sein, wobei es bevorzugt ist, dass die Alkylreste gleich sind. Bevorzugte Trimellitsäuretrialkylester sind beispielsweise Trimellitsäuretri-n-butylester, Trimellitsäuretri-iso-butylester, Trimellitsäuretri-n-pentylester, Trimellitsäuretri-n-hexylester, Trimellitsäuretri-cyclohexylester, Trimellitsäuretri-n-heptylester, Trimellitsäuretri-n-octylester, Trimellitsäuretri-iso-octylester, Trimellitsäuretri-(2-ethylhexyl)ester, Trimellitsäuretri-n-nonylester, Trimellitsäuretri-iso-nonylester, Trimellitsäuretri-(2-propylhexyl)ester, Trimellitsäuretri-n-decylester, Trimellitsäuretri-iso-decylester, Trimellitsäuretri-(2-propylheptyl)ester, Trimellitsäuretri-n-undecylester, Trimellitsäuretri-iso-undecylester, Trimellitsäure-n-dodecylester, Trimellitsäure-iso-dodecylester, Trimellitsäure-n-tridecylester oder Trimellitsäuretri-iso-tridecylester. Ein bevorzugtes Trimellitat mit unterschiedlichen Alkylresten ist beispielsweise Tri(n-octyl-n-decyl)trimellitat.

Trimellitsäuretrialkylester weisen weiter bevorzugt 4 oder 8 bis 10 Kohlenstoffatome in den Alkylresten auf. Die Alkylreste können unabhängig voneinander unterschiedlich oder gleich sein, wobei es bevorzugt ist, dass die Alkylreste gleich sind. Weiter bevorzugte Trimellitsäuretrialkylester sind beispielsweise Trimellitsäuretri-n-butylester, Trimellitsäuretri-iso-butylester, Trimellitsäuretri-n-octylester, Trimellitsäuretri-iso-octylester, Trimellitsäuretri-(2-ethylhexyl)ester, Trimellitsäuretri-n-nonylester, Trimellitsäuretri-iso-nonylester, Trimellitsäuretri-(2-propylhexyl)ester, Trimellitsäuretri-n-decylester oder Trimellitsäuretri-iso-decylester. Ein bevorzugtes Trimellitat mit unterschiedlichen Alkylresten ist beispielsweise Tri(n-octyl-n-decyl)trimellitat, dieses ist beispielsweise als Palatinol® 810 TM erhältlich (Cas No. 68130-50-7).

Besonders bevorzugte Trimellitsäuretrialkylester sind Trimellitsäuretri-n-butylester, Trimellitsäuretri-iso-butylester, Trimellitsäuretri-(2-ethylhexyl)ester, Trimellitsäuretri-n-nonylester, Trimellitsäuretri-iso-nonylester oder Tri(n-octyl-n-decyl)trimellitat.

Benzoesäurealkylester weisen bevorzugt 7 bis 13 Kohlenstoffatome in ihrem Alkylrest auf. Bevorzugt Benzoesäurealkylester sind beispielsweise Isononylbenzoat, Isodecylbenzoat oder 2-Propylheptylbenzoat.

Bevorzugte Dibenzoesäureester sind beispielsweise Diethylenglycoldibenzoat, Dipropylenglycoldibenzoat, Tripropylenglycoldibenzoat oder Dibutylenglycoldibenzoat. Bei Dipropylenglycoldibenzoat, Tripropylenglycoldibenzoat oder Dibutylenglycoldibenzoat kann es sich jeweils um Mischungen von Isomeren handeln.

Ein bevorzugter tetra-Ester von Pentaerythrit ist beispielsweise Pentaerythritol tetra(valerat) (Pevalen™).

Bevorzugte gesättigte Monocarbonsäureester sind beispielsweise Ester der Essigsäure, Ester der Buttersäure, Ester der Valeriansäure oder Ester der Milchsäure. Bevorzugte gesättigte Monocarbonsäureester sind ebenfalls Ester einer gesättigten Monocarbonsäure mit einem mehrwertigen Alkohol. So ist beispielsweise Petraerythrit vollständig

verestert mit Valeriansäure ebenfalls bevorzugt.

Bevorzugte ungesättigte Monocarbonsäureester sind beispielsweise Ester der Acrylsäure.

Bevorzugte ungesättigte Dicarbonsäureester sind beispielsweise Ester der Maleinsäure.

Aromatische Sulfonsäureester weisen bevorzugt 8 bis 22 Kohlenstoffatome in ihren Resten auf. Bevorzugte aromatische Sulfonsäureester sind beispielsweise Phenyl- oder Cresylester.

Alkylsulfonsäureester weisen bevorzugt 8 bis 22 Kohlenstoffatome in ihren Alkylresten auf. Ein bevorzugter Alkylsulfonsäureester ist beispielsweise Pentadecylsulfonsäureester.

Isosorbidester sind Isosorbidmono- und/oder Isosorbiddiester. Isosorbidester weisen bevorzugt 8 bis 13 Kohlenstoffatome in den Alkylresten auf. Die Alkylreste können unabhängig voneinander unterschiedlich oder gleich sein, wobei es bevorzugt ist, dass die Alkylreste gleich sind.

Phosphorsäureester sind beispielsweise Tri-2-ethylhexylphosphat, Trioctylphosphat, Triphenylphosphat, Isodecyldiphenylphosphat, Bis-2(2-ethylhexyl)phenyl-phosphat, 2-Ethylhexyldiphenyl-phosphat oder eine Mischung aus zwei oder mehr der zuvor genannten Phophorsäureester.

Bei Citronensäuretriestern liegt die OH-Gruppe entweder in freier oder carboxylierter Form, beispielsweise acetylierter Form vor. Citronensäuretriester weisen bevorzugt 4 bis 8 Kohlenstoffatome in den Alkylresten auf. Die Alkylreste können unabhängig voneinander unterschiedlich oder gleich sein, wobei es bevorzugt ist, dass die Alkylreste gleich sind.

Alkylpyrrolidonderivate weisen bevorzugt 4 bis 18 Kohlenstoffatome in den Alkylketten auf.

2,5-Furandicarbonsäuredialkylester weisen bevorzugt 4 bis 13 Kohlenstoffatome in den Alkylresten auf. Die Alkylreste können unabhängig voneinander unterschiedlich oder gleich sein, wobei es bevorzugt ist, dass die Alkylreste gleich sind.

2,5-Tetrahydrofurandicarbonsäuredialkylester weisen bevorzugt 4 bis 13 Kohlenstoffatome in den Alkylresten auf. Die Alkylreste können unabhängig voneinander unterschiedlich oder gleich sein, wobei es bevorzugt ist, dass die Alkylreste gleich sind.

Polyester aus aromatischen und/oder aliphatischen Polycarbonsäuren mit zwei- oder mehrwertigen Alkoholen, sind beispielsweise Polyester auf Basis von Adipinsäure, Sebacinsäure, Glutarsäure oder Azelainsäure mit mehrwertigen Alkoholen, wie Dialkylenglykolpolyadipate mit 2 bis 6 Kohlenstoffatomen in der Alkyleinheit; oder Polyester auf Basis von Phthalsäure mit mehrwertigen Alkoholen wie Dialkylenglykolpolyphthalate mit 2 bis 6 Kohlenstoffatomen in der Alkyleinheit.

Epoxidiertes Pflanzenöl ist beispielsweise epoxidiertes Sojaöl.

Ein kommerziell erhältlicher epoxidierter Fettsäuremonoalkylester ist beispielsweise Reflex 100 (Firma PolyOne).

Es ist bevorzugt, dass in der erfindungsgemäßen Weichmacher-Zusammensetzung eine oder mehrere Verbindungen der allgemeinen Formel (I) in einer Gesamtmenge von 5 bis 95 Gewichtsprozent und ein oder mehrere Weichmacher, die von den Verbindungen der allgemeinen Formel (I) unterschiedlich sind, in einer Gesamtmenge von 5 bis 95 Gewichtsprozent enthalten sind, wobei sich die Gewichtsprozentangaben auf das Gesamtgewicht aller in der Weichmacher-Zusammensetzung enthaltenen Weichmacher beziehen und sich auf 100 Prozent addieren.

Es ist weiter bevorzugt, dass in der erfindungsgemäßen Weichmacher-Zusammensetzung eine oder mehrere Verbindungen der allgemeinen Formel (I) in einer Gesamtmenge von 20 bis 80 Gewichtsprozent und ein oder mehrere Weichmacher, die von den Verbindungen der allgemeinen Formel (I) unterschiedlich sind, in einer Gesamtmenge von 20 bis 80 Gewichtsprozent enthalten sind, wobei sich die Gewichtsprozentangaben auf das Gesamtgewicht aller in der Weichmacher-Zusammensetzung enthaltenen Weichmacher beziehen und sich auf 100 Prozent addieren.

Es ist bevorzugt, dass in der erfindungsgemäßen Weichmacher-Zusammensetzung eine oder mehrere Verbindungen der allgemeinen Formel (I) in einer Gesamtmenge von 30 bis 70 Gewichtsprozent und ein oder mehrere Weichmacher, die von den Verbindungen der allgemeinen Formel (I) unterschiedlich sind, in einer Gesamtmenge von 30 bis 70 Gewichtsprozent enthalten sind, wobei sich die Gewichtsprozentangaben auf das Gesamtgewicht aller in der Weichmacher-Zusammensetzung enthaltenen Weichmacher beziehen und sich auf 100 Prozent addieren.

## Formmasse

Gegenstand der vorliegenden Erfindung ist auch eine Formmasse, die eine oder mehrere Verbindungen der allgemeinen Formel (I) und ein oder mehrere Polymere enthält. Ebenfalls Gegenstand der vorliegenden Erfindung ist auch eine Formmasse, die die erfindungsgemäße Weichmacher-Zusammensetzung und ein oder mehrere Polymere enthält.

Bezüglich bevorzugter Ausführungsformen für $R^{1a}$, $R^{1b}$, $R^{2a}$, $R^{2b}$ und/oder n wird auf die vorherigen Angaben in vollem Umfang Bezug genommen.

Bei einem Polymer handelt es sich um einen Thermoplast oder um ein Elastomer. Die erfindungsgemäße Formmasse kann demnach ein oder mehrere Thermoplaste, ein oder mehrere Elastomere oder eine Mischung aus

einem oder mehreren Thermoplasten und einem oder mehreren Elastomeren enthalten.

Enthält die Formmasse ein oder mehrere Thermoplaste und ein oder mehrere Elastomere ist es bevorzugt, dass das Gewichtsverhältnis zwischen der Gesamtmenge an Thermoplaste und der Gesamtmenge an Elastomere 1 : 100 bis 100 : 1 beträgt.

Bei einem Thermoplast handelt es sich beispielsweise um ein Homo- oder Copolymer, das in einpolymerisierter Form ein oder mehrere Monomere enthält, die ausgewählt sind unter $C_2$- bis $C_{10}$-Monoolefinen, beispielsweise Ethylen oder Propylen; 1,3-Butadien, 2-Chloro-1,3-butadien, Vinylalkoholen oder deren $C_2$- bis $C_{10}$-Alkylestern; Vinylacetat, Vinylchlorid, Vinylidenchlorid, Vinylidenfluorid, Tetrafluorethylen, Glycidylacrylat, Glycidylmethacrylat, Acrylsäureester oder Methacrylsäureester mit geradkettigen oder verzweigten $C_1$- bis $C_{10}$-Alkoholen; Vinylaromaten wie Stryol; Acrylnitril, Methacrylnitril, $\alpha,\beta$-ethylenisch ungesättige Mono- oder Dicarbonsäuren und Maleinsäureanhydrid.

Bei einem Thermoplast handelt es sich beispielsweise auch um Polyamide, Polyvinylester, Polycarbonat, Polyether, Polyetherketon, thermoplastisches Polyurethan, Polysulfid, Polysulfon, Polyester, Polyalkylenterephthalat, Polyhydroxyalkanoat, Polybutylensuccinat, Polybutylensuccinatadipat, Polyacrylat oder Polymethacrylat mit gleichen oder unterschiedlichen $C_1$- bis $C_8$-Alkylresten wie Butyl, Hexyl, n-Octyl, iso-Octyl oder 2-Ethylhexyl; Methylmethacrylat-Butylacrylat-Copolymer, Acrylnitril-Butadien-Styrol-Copolymer, Ethylen-Propylen-Copolymer, Polystyrol, Styrol-Acrylnitril-Copolymer, Acrylnitril-Styrol-Acrylat, Styrol-Butadien-Methylmethacrylat-Copolymer, Styrol-Maleinsäureanhydrid-Copolymer, Styrol-Methacrylsäure-Copolymer, Polyoxymethylen, Polyvinylalkohol, Polyvinylacetat, Polyvinylbutyral, Polyvinylchlorid, Polycaprolacton, Polyhydroxybuttersäure, Polyhydroxyvaleriansäure, Polymilchsäure, Ethylcellulose, Celluloseacetat, Cellulosepropionat oder Cellulose-Acetat/Butyrat.

Bei einem Elastomer handelt es sich beispielsweise um natürlichen Kautschuk oder synthetischen Kautschuk wie Polyisopren-Kautschuk, Styrol-Butadien-Kautschuk, Butadien-Kautschuk, Nitril-Butadien-Kautschuk, oder Chloropren-Kautschuk.

Es ist bevorzugt, dass Polyvinylchlorid in der Formmasse enthalten ist.

Abhängig von dem in der Formmasse enthaltenen Polymer, bzw. den in der Formmasse enthaltenen Polymeren, kann es sein, dass zur Erzielung der gewünschten thermoplastischen Eigenschaften unterschiedliche Mengen an einer oder mehrerer Verbindungen der allgemeinen Formel (I) in der Formmasse enthalten sein müssen. Die Einstellung der gewünschten thermoplastischen Eigenschaften der Formmasse unterliegt der Routinetätigkeit des Fachmanns.

Ist kein Polyvinylchlorid und kein Elastomer bzw. keine Elastomere in der Formmasse enthalten, ist es bevorzugt, dass die Gesamtmenge an einer oder mehrerer Verbindungen der allgemeinen Formel (I), die in der Formmasse enthalten ist 0,5 bis 300 phr beträgt. Es ist weiter bevorzugt, dass die Gesamtmenge an einer oder mehrerer Verbindungen der allgemeinen Formel (I), die in der Formmasse enthalten ist 1 bis 130 phr und besonders bevorzugt 2 bis 100 beträgt.

Ist Polyvinylchlorid und ein oder mehrere Thermoplaste in der Formmasse enthalten, jedoch kein Elastomer, bzw. keine Elastomere, ist es bevorzugt, dass die Gesamtmenge an einer oder mehrerer Verbindungen der allgemeinen Formel (I), die in der Formmasse enthalten ist 5 bis 300 phr beträgt. Es ist weiter bevorzugt, dass die Gesamtmenge an einer oder mehrerer Verbindungen der allgemeinen Formel (I), die in der Formmasse enthalten ist 15 bis 200 phr und besonders bevorzugt 30 bis 150 beträgt.

Enthält die Formmasse ein oder mehrere Elastomere und ein oder mehrere Thermoplaste, jedoch kein Polyvinylchlorid, ist es bevorzugt, dass die Gesamtmenge an einer oder mehrerer Verbindungen der allgemeinen Formel (I), die in der Formmasse enthalten ist 1 bis 60 phr beträgt. Es ist weiter bevorzugt, dass die Gesamtmenge an einer oder mehrerer Verbindungen der allgemeinen Formel (I), die in der Formmasse enthalten ist 2 bis 40 phr und besonders bevorzugt 3 bis 30 beträgt.

Ist Polyvinylchlorid, ein oder mehrere Elastomere in der Formmasse enthalten und ggf. ein oder mehrere von Polyvinylchlorid unterschiedliche Thermoplaste, ist es bevorzugt, dass die Gesamtmenge an einer oder mehrerer Verbindungen der allgemeinen Formel (I), die in der Formmasse enthalten sind 0,5 bis 300 phr beträgt. Es ist weiter bevorzugt, dass die Gesamtmenge an einer oder mehrerer Verbindungen der allgemeinen Formel (I), die in der Formmasse enthalten ist 1 bis 150 phr und besonders bevorzugt 2 bis 120 phr beträgt.

Plastisol

Gegenstand der vorliegenden Erfindung ist auch ein Plastisol, das eine oder mehrere Verbindungen der allgemeinen Formel (I) und ein oder mehrere Thermoplaste enthält. Ebenfalls Gegenstand der vorliegenden Erfindung ist ein Plastisol, das die erfindungsgemäße Weichmacher-Zusammensetzung und ein oder mehrere Thermoplaste enthält.

Bezüglich bevorzugter Ausführungsformen für $R^{1a}$, $R^{1b}$, $R^{2a}$, $R^{2b}$ und/oder n wird auf die vorherigen Angaben in vollem Umfang Bezug genommen.

Neben einem oder mehreren Thermoplasten kann ein Plastisol auch ein oder mehrere Elastomere enthalten. Enthält das Plastisol neben einem oder mehreren Thermoplasten ein oder mehrere Elastomere ist es bevorzugt, dass das Gewichtsverhältnis zwischen der Gesamtmenge an Thermoplaste und der Gesamtmenge an Elastomere 1 : 100 bis 100 : 1 beträgt.

Bei einem Thermoplast handelt es sich beispielsweise um ein Homo- oder Copolymer, das in einpolymerisierter Form ein oder mehrere Monomere enthält, die ausgewählt sind unter $C_2$- bis $C_{10}$-Monoolefinen, beispielsweise Ethylen oder Propylen; 1,3-Butadien, 2-Chloro-1,3-butadien, Vinylalkoholen oder deren $C_2$- bis $C_{10}$-Alkylestern; Vinylacetat, Vinylchlorid, Vinylidenchlorid, Vinylidenfluorid, Tetrafluorethylen, Glycidylacrylat, Glycidylmethacrylat, Acrylsäureester oder Methacrylsäureester mit geradkettigen oder verzweigten $C_1$- bis $C_{10}$-Alkoholen; Vinylaromaten wie Styrol; Acrylnitril, Methacrylnitril, $\alpha,\beta$-ethyienisch ungesättigte Mono- oder Dicarbonsäuren und Maleinsäureanhydrid.

Bei einem Thermoplast handelt es sich beispielsweise auch um Polyamid, Polyvinylester, Polycarbonat, Polyether, Polyetherketon, thermoplastisches Polyurethan, Polysulfid, Polysulfon, Polyester, Polyalkylenterephthalat, Polyhydroxyalkanoat, Polybutylensuccinat, Polybutylensuccinatadipat, Polyacrylat oder Polymethacrylat mit gleichen oder unterschiedlichen $C_1$- bis $C_8$-Alkylresten wie Butyl, Hexyl, n-Octyl, iso-Octyl oder 2-Ethylhexyl; Methylmethacrylat-Butylacrylat-Copolymer, Acrylnitril-Butadien-Styrol-Copolymer, Ethylen-Propylen-Copolymer, Polystyrol, Styrol-Acrylnitril-Copolymer, Acrylnitril-Styrol-Acrylat, Styrol-Butadien-Methylmethacrylat-Copolymer, Styrol-Maleinsäureanhydrid-Copolymer, Styrol-Methacrylsäure-Copolymer, Polyoxymethylen, Polyvinylalkohol, Polyvinylacetat, Polyvinylbutyral, Polyvinylchlorid, Polycaprolacton, Polyhydroxybuttersäure, Polyhydroxyvaleriansäure, Polymilchsäure, Ethylcellulose, Celluloseacetat, Cellulosepropionat oder Cellulose-Acetat/Butyrat.

Bei einem Elastomer handelt es sich beispielsweise um natürlichen Kautschuk oder synthetischen Kautschuk wie Polyisopren-Kautschuk, Styrol-Butadien-Kautschuk, Butadien-Kautschuk, Nitril-Butadien-Kautschuk, oder Chloropren-Kautschuk.

Es ist bevorzugt, dass Polyvinylchlorid im Plastisol enthalten ist.

Abhängig von dem im Plastisol enthaltenen Polymer, bzw. den im Plastisol enthaltenen Polymeren, kann es sein, dass zur Erzielung der gewünschten thermoplastischen Eigenschaften unterschiedliche Mengen an einer oder mehrerer Verbindungen der allgemeinen Formel (I) im Plastisol enthalten sein müssen. Die Einstellung der gewünschten thermoplastischen Eigenschaften des Plastisols unterliegt der Routinetätigkeit des Fachmanns.

Es ist bevorzugt, dass das Plastisol eine oder mehrere Verbindungen der allgemeinen Formel (I) in einer Gesamtmenge von 30 bis 400 phr enthält. Es ist weiter bevorzugt, dass das Plastisol eine oder mehrere Verbindungen der allgemeinen Formel (I) in einer Gesamtmenge von 50 bis 200 phr enthält.

So ist es beispielsweise bevorzugt, dass das Plastisol Polyvinylchlorid und eine oder mehrere Verbindungen der allgemeinen Formel (I) in einer Gesamtmenge von 30 bis 400 phr und besonders bevorzugt in einer Gesamtmenge von 50 bis 200 phr enthält.

Zusatzstoffe in Formmasse oder Plastisol

Die erfindungsgemäße Formmasse, bzw. das erfindungsgemäße Plastisol können zusätzlich ein oder mehrere Zusatzstoffe enthalten. Bei Zusatzstoffen handelt es sich beispielsweise um Stabilisatoren, Füllstoffe, Farbmittel, Flamminhibitoren, Lichtstabilisatoren, Treibmittel, polymere Verarbeitungsmittel, Schlagzähverbesserer, Viskositätsreduzierer, optische Aufheller, Antistatika oder Biostabilisatoren.

Die im Folgenden beschriebenen Zusatzstoffe stellen keine Einschränkung der erfindungsgemäßen Formmasse, bzw. des erfindungsgemäßen Plastisols dar, sondern dienen lediglich der Erläuterung. Vielmehr orientiert sich der Fachmann bei der Auswahl geeigneter Zusatzstoffe an seinem Fachwissen und dem intendierten Verwendungszweck der Formmasse, bzw. des Plastisol.

Stabilisatoren können die üblichen Polyvinylchlorid-Stabilisatoren in fester oder flüssiger Form, wie Ca/Zn-, Ba/Zn-, Pb-, Sn-Stabilisatoren, Carbonate wie Hydrotalcit, säurebindende Schichtsilikate oder eine beliebige Mischung aus zwei oder mehr der zuvor genannten Stabilisatoren sein.

Der Anteil an Stabilisatoren in der erfindungsgemäßen Formmasse, bzw. im erfindungsgemä-βen Plastisol beträgt bevorzugt 0,05 bis 14 phr, weiter bevorzugt 0,2 bis 10 phr und besonders bevorzugt 0,5 bis 6 phr.

Füllstoffe werden im Allgemeinen dazu eingesetzt, um die Druck-, Zug-, und/oder Biegefestigkeit, die Härte und/oder die Wärmeformbeständigkeit der erfindungsgemäßen Formmasse, bzw. des erfindungsgemäßen Plastisols positiv zu beeinflussen.

Als Füllstoffe können beispielsweise Ruß und/oder anorganische Füllstoffe in der erfindungsgemäßen Formmasse enthalten sein. Anorganische Füllstoffe sind beispielsweise natürliche Calciumcarbonate wie Kreide, Kalkstein oder Marmore; synthetische Calciumcarbonate, Dolomit, Silikate, Kieselsäure, Sand, Diatomenerde, Aluminiumsilikate wie Kaolin, Glimmer oder Feldspat; oder eine beliebige Mischung aus zwei oder mehr der zuvor genannten Füllstoffe.

Der Anteil an Füllstoffe in der erfindungsgemäßen Formmasse, bzw. im erfindungsgemäßen Plastisol beträgt bevorzugt 0,01 bis 300 phr, weiter bevorzugt 1 bis 200 phr und besonders bevorzugt 1 bis 100 phr.

Farbmittel können dazu dienen, die erfindungsgemäße Formmasse, bzw. das erfindungsgemäße Plastisol an unterschiedliche Einsatzmöglichkeiten anzupassen. Bei Farbmittel handelt es sich beispielsweise um Pigmente oder Farbstoffe.

Als Pigmente können beispielsweise anorganische und/oder organische Pigmente in der erfindungsgemäßen

Formmasse enthalten sein. Anorganische Pigmente sind beispielsweise Kobalt-Pigmente wie $CoO/Al_2O_3$ und/oder Chrom-Pigmente wie $Cr_2O_3$. Weitere anorganische Pigmente sind beispielsweise Titandioxid, Eisenoxid (insbesondere $Fe_2O_3$) und/oder Nickeltitanate. Organische Pigmente sind beispielsweise Monoazopigmente, kondensierte Azopigmente, Azomethinpigmente, Anthrachinonpigmente, Chinacridone, Phthalocyaninpigmente und/oder Dioxazinpigmente.

Der Anteil an Farbmitteln in der erfindungsgemäßen Formmasse, bzw. im erfindungsgemäßen Plastisol beträgt bevorzugt 0,01 bis 20 phr, weiter bevorzugt 0,1 bis 10 phr und besonders bevorzugt 0,2 bis 6 phr.

Flamminhibitoren können dazu dienen, die Entflammbarkeit der erfindungsgemäßen Formmasse, bzw. des erfindungsgemäßen Plastisols zu vermindern und die Rauchbildung beim Verbrennen zu reduzieren.

Als Flamminhibitoren können beispielsweise Antimontrioxid, Chlorparaffin, Phosphatester, und/oder Aluminiumhydroxid eingesetzt werden.

Der Anteil an Flamminhibitoren in der erfindungsgemäßen Formmasse, bzw. im erfindungsgemäßen Plastisol beträgt bevorzugt 0,01 bis 20 phr, weiter bevorzugt 0,1 bis 10 phr und besonders bevorzugt 0,2 bis 6 phr.

Lichtstabilisatoren, wie UV-Absorber oder HALS-Stabilisatoren, können dazu dienen, Schädigungen der erfindungsgemäßen Formmasse, bzw. des erfindungsgemäßen Plastisols durch den Einfluss von Licht zu reduzieren.

Lichtstabilisatoren sind beispielsweise Hydroxybenzophenone, Hydroxyphenyl-benzotriazole, Cyanoacrylate, "hindered amine light stabilizers" wie Derivate von 2,2,6,6,-Tetramethylpiperidin, Hydroxyphenyltriazine oder eine beliebige Mischung aus zwei oder mehr der zuvor genannten Lichtstabilisatoren.

Der Anteil an Lichtstabilisatoren in der erfindungsgemäßen Formmasse, bzw. im erfindungsgemäßen Plastisol beträgt bevorzugt 0,01 bis 14 phr, weiter bevorzugt 0,04 bis 8 phr und besonders bevorzugt 1 bis 3 phr.

Viskositätsreduzierer können dazu dienen, die Viskosität des erfindungsgemäßen Plastisols zu reduzieren. Generell eigenen sich alle Verbindungen als Viskositätsreduzierer, die eine ausreichende Mischbarkeit mit dem Plastisol und eine geringe Eigenviskosität aufweisen.

Viskositätsreduzierer sind beispielsweise aromatische oder aliphatische Kohlenwasserstoffe oder Carbonsäureester. Kommerziell erhältliche Viskositätsreduzierer sind beispeislweise Shell-Sol der Firma Shell, Exxsol™ oder Isopar™ beide von der Firma Exxon Mobil oder VISCOBYK von der Firma BYK.

Der Anteil an Viskositätsreduzierer in dem erfindungsgemäßen Plastisol beträgt bevorzugt 0,01 bis 10 phr, weiter bevorzugt 0,1 bis 8 phr.

Verwendung von Verbindungen der allgemeinen Formel (I)

Gegenstand der vorliegenden Erfindung ist auch die Verwendung einer oder mehrerer Verbindungen der allgemeinen Formel (I) als Weichmacher oder als Bestandteil einer Weichmacher-Zusammensetzung für ein Polymer, für eine Mischung aus zwei oder mehreren verschiedenen Polymeren oder für ein Plastisol.

Gegenstand der vorliegenden Erfindung ist demnach auch die Verwendung einer oder mehrerer Verbindungen der allgemeinen Formel (I) als Weichmacher für Polyvinylchlorid oder für ein Plastisol, das Polyvinylchlorid enthält.

Ebenfalls ist Gegenstand der vorliegenden Erfindung die Verwendung der erfindungsgemäßen Weichmacher-Zusammensetzung als Weichmacher für Polyvinylchlorid oder ein Plastisol, das Polyvinylchlorid enthält.

Bezüglich bevorzugter Ausführungsformen für $R^{1a}$, $R^{1b}$, $R^{2a}$, $R^{2b}$ und/oder n wird auf die vorherigen Angaben in vollem Umfang Bezug genommen.

Verwendung Formmasse

Die erfindungsgemäße Formmasse eignet sich zur Herstellung von Formkörpern oder Folien.

Bei Formkörpern handelt es sich beispielsweise um Behältnisse, Apparate oder geschäumte Vorrichtungen.

Behältnisse sind beispielswiese Gehäuse von Elektrogeräten wie Küchengeräten oder Computergehäuse; Rohre, Schläuche wie Wasser- oder Bewässerungsschläuche, Industrie-Gummischläuche, Chemieschläuche; Ummantelungen für Draht oder Kabel; Ummantelungen für Werkzeuge, Fahrrad-, Roller oder Schubkarrengriffe; Metallbeschichtungen oder Verpackungsbehälter.

Apparate sind beispielsweise Werkzeuge, Möbel wie Stühle, Regale oder Tische; Schallplatten, Profile wie Bodenprofile für den Außenbereich oder Profile für Förderbänder; Komponenten für den Fahrzeugbau wie Karosseriebestandteile, Unterbodenschutz oder Vibrationsdämpfer; oder Radiergummis.

Geschäumte Vorrichtungen sind beispielsweise Polster, Matratzen, Schaumstoffe oder Dämmmaterialien.

Bei Folien handelt es sich beispielsweise um Planen wie LKW-Planen, Dachplanen, Geomembranen, Stadiondächer oder Zeltplanen; Dichtungen, Verbundfolien wie Folien für Verbundsicherheitsglas, Selbstklebefolien, Kaschierfolien, Schrumpffolien, Bodenbeläge für den Außenbereich, Klebebandfolien, Beschichtungen, Schwimmteichfolien, Zierteichfolien oder Kunstleder.

Die erfindungsgemäße Formmasse eignet sich auch zur Herstellung von Formköpern und Folien, die in direkten Kontakt mit Menschen und/oder Nahrungsmittel kommen.

Formkörper oder Folien, die in direkten Kontakt mit Menschen und/oder Nahrungsmittel kommen, sind beispielsweise Medizinprodukte, Hygieneprodukte, Lebensmittelverpackungen, Produkte für den Innenraum, Baby- und Kinderprodukte, Kinderpflegeartikel, Sport- und Freizeitprodukte, Bekleidungen, Fasern oder Gewebe.

Medizinprodukte sind beispielsweise Schläuche für enterale Ernährung oder Hämodialyse, Beatmungsschläuche, Drainageschläuche, Infusionsschläuche, Infusionsbeutel, Blutbeutel, Katheter, Trachealtuben, Einmalspritzen, Handschuhe oder Atemmasken.

Lebensmittelverpackungen sind beispielsweise Frischhaltefolien, Lebensmittelschläuche, Trinkwasserschläuche, Behälter zur Aufbewahrung oder zum Einfrieren von Lebensmitteln, Deckeldichtungen, Verschlusskappen, Kronkorken oder künstliche Weinkorken.

Produkte für den Innenraum sind beispielsweise Bodenbeläge, welche homogen oder aus mehreren Schichten, umfassend mindestens eine geschäumte Schicht, aufgebaut sein können, Fußbodenbeläge, Schmutzfängermatten, Sportböden, Luxury Vinyl Tiles (LVT), Kunstleder, Wandbeläge, geschäumte oder nicht geschäumte Tapeten; Verkleidungen oder Konsolenabdeckungen in Fahrzeugen.

Baby- und Kinderprodukte sind beispielsweise Spielzeug wie Puppen, Spielfiguren oder Kneten; aufblasbares Spielzeug wie Bälle oder Ringe; Stoppersocken, Schwimmhilfen, Kinderwagenabdeckungen, Wickelauflagen, Wärmeflaschen, Beißringe oder Fläschchen.

Sport- oder Freizeitprodukte sind beispielsweise Gymnastikbälle, Übungsmatten, Sitzkissen, Massagebälle- oder rollen, Schuhe, Schuhsohlen, Bälle, Luftmatratzen oder Trinkflaschen.

Bei Bekleidung handelt es sich beispielsweise um Latexkleidung, Schutzbekleidung, Regenjacken oder Gummistiefel.

Verwendung Plastisole

Plastisole werden üblicherweise bei Umgebungstemperatur durch verschiedene Verfahren wie Streichverfahren, Gießverfahren wie Schalengieß- oder Rotationsgießverfahren; Tauchverfahren, Druckverfahren wie Siebdruckverfahren; Spritzverfahren und dergleichen in Form des fertigen Produkts gebracht. Anschließend erfolgt durch Erwärmung die Gelierung, wobei nach Abkühlung ein homogenes, mehr oder weniger flexibles Produkt erhalten wird.

Das erfindungsgemäße Plastisole eignet sich zur Herstellung von Folien, Tapeten, nahtlosen Hohlkörpern, Handschuhen, heterogenen Fußböden oder für Anwendung im Textilbereich wie Textilbeschichtungen.

Bei Folien handelt es sich beispielsweise um LKW-Planen, Dachplanen, Abdeckungen im Allgemeinen wie Bootsabdeckungen, Kinderwagenabdeckungen oder Stadiondächer; Zeltplanen, Geomembranen, Tischdecken, Beschichtungen, Schwimmteichfolien, Kunstleder oder Zierteichfolien.

Bei Handschuhen handelt es sich beispielsweise um Gartenhandschuhe, medizinische Handschuhe, Chemikalienhandschuhe, Schutzhandschuhe oder Einweghandschuhe.

Das erfindungsgemäße Plastisol eignet sich beispielsweise auch zur Herstellung von Dichtungen wie Deckeldichtungen; Verkleidungen oder Konsolenabdeckungen in Fahrzeugen, Spielzeug wie Puppen, Spielfiguren oder Kneten; aufblasbares Spielzeug wie Bälle oder Ringe; Stoppersocken, Schwimmhilfen, Wickelauflagen, Gymnastikbälle, Übungsmatten, Sitzkissen, Vibratoren, Massagebälle- oder rollen, Latexkleidung, Schutzbekleidung, Regenjacken oder Gummistiefel.

Weitere Verwendungen

Eine oder mehrere Verbindungen der allgemeinen Formel (I) oder die erfindungsgemäße Weichmacher-Zusammensetzung eignen sich auch zur Verwendung als Kalandrier-Hilfsmittel, Rheologie-Hilfsmittel, Bestandteil von oberflächenaktiven Zusammensetzungen wie Fließ- oder Filmbindehilfen, Entschäumern, Schaumverhütern, Benetzungsmitteln, Koaleszenzmitteln oder Emulgatoren; Bestandteil von Schmierstoffen wie Schmieröle, Schmierfette oder Schmierpasten. Auch eignen sich eine oder mehrere Verbindungen der allgemeinen Formel (I) oder die erfindungsgemäße Weichmacher-Zusammensetzung als Bestandteil von Quenschmittel für chemische Reaktionen, als Bestandteil von Phlegmatisierungsmittel in pharmazeutischen Produkten, als Bestandteil von Klebstoffen, Dichtstoffen, Druckertinten, Schlagzähmodifizierern oder Stellmitteln.

Formkörper oder Folien, die eine oder mehrere Verbindungen der allgemeinen Formel (I) oder die erfindungsgemäße Weichmacher-Zusammensetzung enthalten sind ebenfalls Gegenstand der Erfindung. Es wird auf die bei der Verwendung von Formmassen und/oder Plastisolen gemachten Angaben zu Formkörpern oder Folien Bezug genommen. Die dabei angeführten Beispiele für Formkörper oder Folien sind zur Auslegung der Begrifflichkeiten in diesem Abschnitt heranzuziehen, wobei diese nicht einschränkend zu verstehen sind.

Heptanol

Die zur Herstellung der Verbindungen der allgemeinen Formel (I) eingesetzten Heptanole können geradkettig oder verzweigt sein oder aus Gemischen aus geradkettigen und verzweigten Heptanolen bestehen. Es kann bevorzugt sein, dass Gemische aus verzweigten Heptanolen, auch als Isoheptanol bezeichnet, verwendet werden, die durch die Rhodium- oder vorzugsweise Kobalt-katalysierte Hydroformylierung von Dimerpropen, erhältlich z. B. nach dem Dimersol®-Verfahren, und anschließende Hydrierung der erhaltenen Isoheptanale zu einem Isoheptanol-Gemisch hergestellt werden. Entsprechend seiner Herstellung besteht das so gewonnene Isoheptanol-Gemisch aus mehreren Isomeren. Im Wesentlichen geradkettige Heptanole können durch die Rhodium- oder vorzugsweise Kobalt-katalysierte Hydroformylierung von 1-Hexen und anschließende Hydrierung des erhaltenen n-Heptanals zu n-Heptanol erhalten werden. Die Hydroformylierung von 1-Hexen bzw. Dimerpropen kann nach an sich bekannten Verfahren erfolgen: Bei der Hydroformylierung mit homogen im Reaktionsmedium gelösten Rhodiumkatalysatoren können sowohl unkomplexierte Rhodiumcarbonyle, die in situ unter den Bedingungen der Hydroformylierungsreaktion im Hydroformylierungsreaktionsgemisch unter Einwirkung von Synthesegas z. B. aus Rhodiumsalzen gebildet werden, als auch komplexe Rhodiumcarbonylverbindungen, insbesondere Komplexe mit organischen Phosphinen, wie Triphenylphosphin, oder Organophosphiten, vorzugsweise chelatisierenden Biphosphiten, wie z. B. in US-A 5288918 beschrieben, als Katalysator verwendet werden. Bei der Kobalt-katalysierten Hydroformylierung dieser Olefine werden im Allgemeinen homogen im Reaktionsgemisch lösliche Kobaltcarbonyl-Verbindungen eingesetzt, die sich unter den Bedingungen der Hydroformylierungsreaktion unter Einwirkung von Synthesegas in situ aus Kobaltsalzen bilden. Wird die Kobalt-katalysierte Hydroformylierung in Gegenwart von Trialkyl- oder Triarylphosphinen ausgeführt, bilden sich als Hydroformylierungsprodukt direkt die gewünschten Heptanole, so dass keine weitere Hydrierung der Aldehydfunktion mehr benötigt wird.

Zur Kobalt-katalysierten Hydroformylierung des 1-Hexens bzw. der Hexenisomerengemische eignen sich beispielsweise die in Falbe, New Syntheses with Carbon Monoxide, Springer, Berlin, 1980 auf den Seiten 162 - 168, erläuterten industriell etablierten Verfahren, wie das Ruhrchemie-Verfahren, das BASF-Verfahren, das Kuhlmann-Verfahren oder das Shell-Verfahren. Während das Ruhrchemie-, BASF- und das Kuhlmann-Verfahren mit nicht ligandmodifizierten Kobaltcarbonyl-Verbindungen als Katalysatoren arbeiten und dabei Hexanal-Gemische erhalten, verwendet das Shell-Verfahren (DE-A 1593368) Phosphin- oder Phosphit-Ligand-modifizierte Kobaltcarbonyl-Verbindungen als Katalysator, die aufgrund ihrer zusätzlichen hohen Hydrieraktivität direkt zu den Hexanolgemischen führen. Vorteilhafte Ausgestaltungen zur Durchführung der Hydroformylierung mit nicht-ligandmodifizierten Kobaltcarbonylkomplexen werden beispielsweise in DE-A 2139630, DE-A 2244373, DE-A 2404855 und WO 01014297 detailliert beschrieben.

Zur Rhodium-katalysierten Hydroformylierung des 1-Hexens bzw. der Hexenisomerengemische kann das industriell etablierte Rhodium-Niederdruck-Hydroformylierungs-verfahren mit Triphenylphosphinligand-modifizierten Rhodiumcarbonylverbindungen angewandt werden, wie es zum Beispiel Gegenstand von US-A 4148830 ist. Es kann Vorteilhaft sein, dass zur Rhodium-katalysierten Hydroformylierung langkettiger Olefine, wie der nach den vorstehend genannten Verfahren erhaltenen Hexenisomerengemische, nicht-ligandmodifizierte Rhodiumcarbonylverbindungen als Katalysator dienen, wobei im Unterschied zum Niederdruckverfahren ein höherer Druck von 80 bis 400 bar einzustellen ist. Die Durchführung solcher Rhodium-Hochdruck-Hydroformylierungsverfahren wird in z. B. EP-A 695734, EP-B 880494 und EP-B 1047655 beschrieben.

Die nach Hydroformylierung der Hexen-Isomerengemische erhaltenen Isoheptanalgemische können zum Beispiel auf an sich herkömmliche Weise zu Isoheptanolgemischen katalytisch hydriert werden. Es kann bevorzugt sein, dass hierzu heterogene Katalysatoren verwendet werden, die als katalytisch aktive Komponente Metalle und/oder Metalloxide der VI. bis VIII. sowie der I. Nebengruppe des Periodensystems der Elemente, insbesondere Chrom, Molybdän, Mangan, Rhenium, Eisen, Kobalt, Nickel und/oder Kupfer, gegebenenfalls abgeschieden auf einem Trägermaterial wie $Al_2O_3$, SiOz und/oder $TiO_2$, enthalten. Solche Katalysatoren werden z. B. in DE-A 3228881, DE-A 2628987 und DE-A 2445303 beschrieben. Weiter kann es bevorzugt sein, dass die Hydrierung der Isoheptanale mit einem Überschuss an Wasserstoff von 1,5 bis 20 % über der stöchiometrisch zur Hydrierung der Isoheptanale benötigten Wasserstoffmenge, bei Temperaturen von 50 bis 200 °C und bei einem Wasserstoffdruck von 25 bis 350 bar durchgeführt wird und zur Vermeidung von Nebenreaktionen dem Hydrierzulauf gemäß DE-A 2628987 eine geringe Menge Wasser, beispielsweise in Form einer wässrigen Lösung eines Alkalimetallhydroxids oder -carbonats entsprechend der Lehre von WO 01087809, zugefügt wird.

Octanol

2-Ethylhexanol, das für lange Jahre der in den größten Mengen produzierte Weichmacheralkohol war, kann zum Beispiel über die Aldolkondensation von n-Butyraldehyd zu 2-Ethylhexenal und dessen anschließende Hydrierung zu 2-Ethylhexanol gewonnen werden (s. Ullmann's Encyclopedia of Industrial Chemistry; 5. Auflage, Bd. A 10, S. 137 - 140, VCH Verlagsgesellschaft GmbH, Weinheim 1987).

Im Wesentlichen geradkettige Octanole können zum Beispiel durch die Rhodium- oder vorzugsweise Kobalt-katalysierte Hydroformylierung von 1-Hepten und anschließende Hydrierung des erhaltenen n-Octanals zu n-Octanol erhalten werden. Das dazu benötigte 1-Hepten kann beispielsweise aus der Fischer-Tropsch-Synthese von Kohlenwasserstoffen gewonnen werden.

Bei dem Alkohol Isooctanol handelt es sich im Unterschied zu 2-Ethylhexanol oder n-Octanol, bedingt durch seine Herstellungsweise, in der Regel nicht um eine einheitliche chemische Verbindung, sondern um ein Isomerengemisch aus unterschiedlich verzweigten $C_8$-Alkoholen, beispielsweise aus 2,3-Dimethyl-1-hexanol, 3,5-Dimethyl-1-hexanol, 4,5-Dimethyl-1-hexanol, 3-Methyl-1-heptanol und 5-Methyl-1-heptanol, die je nach den angewandten Herstellungsbedingungen und -verfahren in unterschiedlichen Mengenverhältnissen im Isooctanol vorliegen können. Isooctanol wird üblicherweise durch die Codimerisierung von Propen mit Butenen, wie n-Butenen, und anschließende Hydroformylierung des dabei erhaltenen Gemisches aus Heptenisomeren hergestellt. Das bei der Hydroformylierung erhaltene Octanal-Isomerengemisch kann anschließend auf an sich herkömmliche Weise zum Isooctanol hydriert werden.

Die Codimerisierung von Propen mit Butenen zu isomeren Heptenen kann beispielsweise mit Hilfe des homogenkatalysierten Dimersol®-Verfahrens (zum Beispiel Chauvin et al; Chem. Ind.; Mai 1974, S. 375 - 378) erfolgen, bei dem als Katalysator ein löslicher Nickel-Phosphin-Komplex in Gegenwart einer Ethylaluminiumchlor-Verbindung, beispielsweise Ethylaluminiumdichlorid, dient. Als Phosphin-Liganden für den Nickelkomplex-Katalysator können z. B. Tributylphosphin, Tri-isopropylphosphin, Tricyclohexylphosphin und/oder Tribenzylphosphin eingesetzt werden. Die Umsetzung findet im Allgemeinen bei Temperaturen von 0 bis 80°C statt, wobei es vorteilhaft sein kann, einen Druck einzustellen, bei dem die Olefine im flüssigen Reaktionsgemisch gelöst vorliegen (zum Beispiel Cornils; Hermann: Applied Homogeneous Catalysis with Organometallic Compounds; 2. Auflage; Bd. 1; S. 254 - 259, Wiley-VCH, Weinheim 2002).

Alternativ zum mit homogen im Reaktionsmedium gelösten Nickelkatalysatoren betriebenen Di-mersol®-Verfahren kann die Codimerisierung von Propen mit Butenen auch mit auf einem Träger abgeschiedenen, heterogenen NiO-Katalysatoren durchgeführt werden, wobei ähnliche Hepten-Isomerenverteilungen erhalten werden wie beim homogen katalysierten Verfahren. Solche Katalysatoren werden beispielsweise im so genannten OctolO-Verfahren (Hydrocarbon Processing, Februar 1986, S. 31 - 33) verwendet, ein gut geeigneter spezifischer Nickel-Heterogenkatalysator zur Olefindimerisierung bzw. Codimerisierung ist z. B. in WO 9514647 offenbart.

Anstelle von Katalysatoren auf Basis von Nickel können auch Brransted-acide heterogene Katalysatoren zur Codimerisierung von Propen mit Butenen verwendet werden, wobei in der Regel höher verzweigte Heptene als in den Nickel-katalysierten Verfahren erhalten werden. Beispiele von hierfür geeigneten Katalysatoren sind feste Phosphorsäure-Katalysatoren z. B. mit Phosphorsäure imprägnierte Kieselgur oder Diatomeenerde, wie sie beispielweise vom PolyGas®-Verfahren zur Olefindi- bzw. Oligomerisierung benutzt werden (zum Beispiel Chitnis et al; Hydrocarbon Engineering 10, Nr. 6 - Juni 2005). Zur Codimerisierung von Propen und Butenen zu Heptenen sehr gut geeignete Brønsted-acide Katalysatoren sind meist Zeolithe, deren sich beispielsweise das auf Basis des PolyGas®-Verfahrens weiterentwickelte EMOGAS®-Verfahren bedient.

Das 1-Hepten und die Hepten-Isomerengemische werden nach den vorstehend in Zusammenhang mit der Herstellung von n-Heptanal und Heptanal-Isomerengemische erläuterten bekannten Verfahren mittels Rhodium- oder Kobalt-katalysierter Hydroformylierung, vorzugsweise Kobalt-katalysierter Hydroformylierung, in n-Octanal bzw. Octanal-Isomerengemische überführt. Diese werden anschließend z. B. mittels eines der vorstehend in Zusammenhang mit der n-Heptanol- und Isoheptanol-Herstellung genannten Katalysatoren zu den entsprechenden Octanolen hydriert.

Nonanol

Im Wesentlichen geradkettiges Nonanol kann beispielsweise durch die rhodium- oder vorzugsweise kobaltkatalysierte Hydroformylierung von 1-Octen und nachfolgende Hydrierung des dabei erhaltenen n-Nonanals erhalten werden. Das Ausgangsolefin 1-Octen kann beispielsweise über eine Ethylenoligomerisierung mittels einem homogen im Reaktionsmedium - 1,4-Butandiol - löslichen Nickelkomplexkatalysator mit z. B. Diphenylphosphinoessigsäure oder 2-Diphenylphosphinobenzoesäure als Liganden erhalten werden. Dieses Verfahren ist auch unter der Bezeichnung Shell Higher Olefins Process oder SHOP-Verfahren bekannt (zum Beispiel Weisermel, Arpe: Industrielle Organische Chemie; 5. Auflage; S. 96; Wiley-VCH, Weinheim 1998).

Bei Isononanol, welches zur Synthese der Verbindungen der allgemeinen Formel (I) eingesetzt wird, handelt es sich nicht um eine einheitliche chemische Verbindung, sondern um ein Gemisch aus unterschiedlich verzweigten isomeren $C_9$-Alkoholen, die je nach der Art ihrer Herstellung, insbesondere auch der verwendeten Ausgangsstoffe, unterschiedliche Verzweigungsgrade haben können. Im Allgemeinen werden die Isononanole durch Dimerisierung von Butenen zu Isooctengemischen, anschließende Hydroformylierung der Isooctengemische und Hydrierung der dabei erhaltenen Isononanalgemische zu Isononanolgemischen hergestellt, wie zum Beispiel in Ull-mann's Encyclopedia of Industrial Chemistry, 5. Auflage, Bd. A1, S. 291 - 292, VCH Verlagsgesellschaft GmbH, Weinheim 1995, erläutert.

Als Ausgangsmaterial zur Herstellung der Isononanole können sowohl Isobuten, cis- und trans-2-Buten als auch 1-Buten oder Gemische dieser Butenisomere verwendet werden. Bei der vorwiegend mittels flüssiger, z. B. Schwefel- oder Phosphorsäure, oder fester, z. B. auf Kieselgur, SiOz oder $Al_2O_3$ als Trägermaterial aufgebrachter Phosphorsäure oder Zeolithe, oder Brønsted-Säuren katalysierten Dimerisierung von reinem Isobuten, wird überwiegend das stark verzweigte 2,4,4-Trimethylpenten, auch als Diisobutylen bezeichnet, erhalten, das nach Hydroformylierung und Hydrierung des Aldehyds hochverzweigte Isononanole liefert.

Isononanole mit einem geringeren Verzweigungsgrad können bevorzugt sein. Solche gering verzweigten Isononanol-Gemische werden aus den linearen Butenen 1-Buten, cis- und/oder trans-2-Buten, die gegebenenfalls noch geringere Mengen an Isobuten enthalten können beispielsweise über den vorstehend beschriebenen Weg der Butendimerisierung, Hydroformylierung des Isooctens und Hydrierung der erhaltenen Isononanal-Gemische hergestellt. Es kann bevorzugt sein, dass als Rohstoff Raffinat II eingesetzt wird. Raffinat II kann im Allgeminen aus dem $C_4$-Schnitt eines Crackers, beispielsweise eines Steamcrackers, das nach Eliminierung von Allenen, Acetylenen und Dienen, insbesondere 1,3-Butadien, durch dessen Partialhydrierung zu linearen Butenen oder dessen Abtrennung durch Extraktivdestillation, beispielsweise mittels N-Methylpyrrolidon, und nachfolgender Brønsted-Säure katalysierter Entfernung des darin enthaltenen Isobutens durch dessen Umsetzung mit Methanol oder Isobutanol nach großtechnisch etablierten Verfahren unter Bildung des Kraftstoffadditivs Methyl-tert.-Butylether (MTBE) oder des zur Gewinnung von Rein-Isobuten dienenden Isobutyl-tert.-Butylether, gewonnen werden.

Raffinat II kann neben 1-Buten und cis- und trans-2-Buten noch n- und iso-Butan und Restmengen von bis zu 5 Gew.-% an Isobuten enthalten.

Die Dimerisierung der linearen Butene oder des im Raffinat II enthaltenen Butengemischs kann zum Beispiel mittels der gängigen, großtechnisch praktizierten Verfahren, wie sie vorstehend in Zusammenhang mit der Erzeugung von Isoheptengemischen erläutert wurden, beispielsweise mittels heterogener, Brønsted-acider Katalysatoren, wie sie beispielsweise im PolyGas®- oder EMOGAS®-Verfahren eingesetzt werden, mittels des Dimersol®-Verfahrens unter Verwendung homogen im Reaktionsmedium gelöster Nickel-Komplex-Katalysatoren oder mittels heterogener, Nickel(II)oxid-haltiger Katalysatoren nach dem Octol®-Verfahren oder zum Beispiel dem Verfahren gemäß WO 9514647 durchgeführt werden. Die dabei erhaltenen Isoocten-Gemische werden nach den vorstehend in Zusammenhang mit der Herstellung von Heptanal-Isomerengemische erläuterten bekannten Verfahren mittels Rhodium- oder Kobalt-katalysierter Hydroformylierung, vorzugsweise Kobalt-katalysierter Hydroformylierung, in Isononanal-Gemische überführt. Diese werden anschließend z. B. mittels einem der vorstehend in Zusammenhang mit der Isoheptanol-Herstellung genannten Katalysatoren zu den geeigneten Isononanolgemischen hydriert.

Die so hergestellten Isononanol-Isomerengemische können über ihren Isoindex charakterisiert werden, der aus dem Verzweigungsgrad der einzelnen isomeren Isononanolkomponenten im Isononanolgemisch multipliziert mit deren prozentualen Anteil im Isononanolgemisch errechnet werden kann. So tragen z. B. n-Nonanol mit dem Wert 0, Methyloctanole (eine Verzweigung) mit dem Wert 1 und Dimethylheptanole (zwei Verzweigungen) mit dem Wert 2 zum Isoindex eines Isononanolgemisches bei. Je höher die Linearität, desto niedriger ist der Isoindex des betreffenden Isononanolgemisches. Dementsprechend kann der Isoindex eines Isononanolgemisches durch gaschromatographische Auftrennung des Isononanolgemisches in seine einzelnen Isomere und damit einhergehender Quantifizierung von deren prozentualen Mengenanteil im Isononanolgemisch, bestimmt nach Standardmethoden der gaschromatographischen Analyse, ermittelt werden. Zwecks Erhöhung der Flüchtigkeit und Verbesserung der gaschromatographischen Auftrennung der isomeren Nonanole werden diese zweckmäßigerweise vor der gaschromatographischen Analyse mittels Standardmethoden, beispielsweise durch Umsetzung mit N-Methyl-N-trimethylsilyltrifluoracetamid, trimethylsilyliert. Um eine möglichst gute Trennung der einzelnen Komponenten bei der gaschromatographischen Analyse zu erzielen, werden üblicherweise Kapillarsäulen mit Polydimethylsiloxan als stationärer Phase verwendet. Solche Kapillarsäulen sind im Handel erhältlich, und es bedarf lediglich einiger weniger Routineversuche des Fachmanns, um aus dem vielfältigen Angebot des Handels ein optimal für diese Trennaufgabe geeignetes Fabrikat zu wählen.

Verbindungen der allgemeinen Formel (I) mit Isononoanolrest(en) sind im Allgemeinen mit Isononanolen mit einem Isoindex von 0,8 bis 2, vorzugsweise von 1,0 bis 1,8 und besonders bevorzugt von 1,1 bis 1,5 verestert, die nach den vorstehend genannten Verfahren hergestellt werden können.

Lediglich beispielhaft werden im Folgenden mögliche Zusammensetzungen von Isononanolgemischen angegeben, wie sie zur Herstellung der Verbindungen der allgemeinen Formel (I) verwendet werden können, wobei anzumerken ist, dass die Anteile der im Einzelnen aufgeführten Isomeren im Isononanolgemisch abhängig von der Zusammensetzung des Ausgangsmaterials, beispielsweise Raffinat II, dessen Zusammensetzung an Butenen produktionsbedingt variieren kann und von Schwankungen in den angewandten Produktionsbedingungen, beispielsweise dem Alter der benutzten Katalysatoren und daran anzupassenden Temperatur- und Druckbedingungen, variieren können.

Beispielsweise kann ein Isononanolgemisch, das durch Kobalt-katalysierte Hydroformylierung und anschließende Hydrierung aus einem, unter Verwendung von Raffinat II als Rohstoff, mittels des Katalysators und Verfahrens gemäß WO 9514647 erzeugten Isooctengemisches hergestellt wurde, folgende Zusammensetzung haben:

- 1,73 bis 3,73 Gew.-%, bevorzugt 1,93 bis 3,53 Gew.-%, besonders bevorzugt 2,23 bis 3,23 Gew.-% 3-Ethyl-6-methyl-hexanol;

- 0,38 bis 1,38 Gew.-%, bevorzugt 0,48 bis 1,28 Gew.-%, besonders bevorzugt 0,58 bis 1,18 Gew.-% 2,6-Dimethylheptanol;
- 2,78 bis 4,78 Gew.-%, bevorzugt 2,98 bis 4,58 Gew.-%, besonders bevorzugt 3,28 bis 4,28 Gew.-% 3,5-Dimethylheptanol;
- 6,30 bis 16,30 Gew.-%, bevorzugt 7,30 bis 15,30 Gew.-%, besonders bevorzugt 8,30 bis 14,30 Gew.-% 3,6-Dimethylheptanol;
- 5,74 bis 11,74 Gew.-%, bevorzugt 6,24 bis 11,24 Gew.-%, besonders bevorzugt 6,74 bis 10,74 Gew.-% 4,6-Dimethylheptanol;
- 1,64 bis 3,64 Gew.-%, bevorzugt 1,84 bis 3,44 Gew.-%, besonders bevorzugt 2,14 bis 3,14 Gew.-% 3,4,5-Trimethylhexanol;
- 1,47 bis 5,47 Gew.-%, bevorzugt 1,97 bis 4,97 Gew.-%, besonders bevorzugt 2,47 bis 4,47 Gew.-% 3,4,5-Trimethylhexanol, 3-Methyl-4-ethylhexanol und 3-Ethyl-4-methylhexanol;
- 4,00 bis 10,00 Gew.-%, bevorzugt 4,50 bis 9,50 Gew.-%, besonders bevorzugt 5,00 bis 9,00 Gew.-% 3,4-Dimethylheptanol;
- 0,99 bis 2,99 Gew.-%, bevorzugt 1,19 bis 2,79 Gew.-%, besonders bevorzugt 1,49 bis 2,49 Gew.-% 4-Ethyl-5-methylhexanol und 3-Ethylheptanol;
- 2,45 bis 8,45 Gew.-%, bevorzugt 2,95 bis 7,95 Gew.-%, besonders bevorzugt 3,45 bis 7,45 Gew.-% 4,5-Dimethylheptanol und 3-Methyloctanol;
- 1,21 bis 5,21 Gew.-%, bevorzugt 1,71 bis 4,71 Gew.-%, besonders bevorzugt 2,21 bis 4,21 Gew.-% 4,5-Dimethylheptanol;
- 1,55 bis 5,55 Gew.-%, bevorzugt 2,05 bis 5,05 Gew.-%, besonders bevorzugt 2,55 bis 4,55 Gew.-% 5,6-Dimethylheptanol;
- 1,63 bis 3,63 Gew.-%, bevorzugt 1,83 bis 3,43 Gew.-%, besonders bevorzugt 2,13 bis 3,13 Gew.-% 4-Methyloctanol;
- 0,98 bis 2,98 Gew.-%, bevorzugt 1,18 bis 2,78 Gew.-%, besonders bevorzugt 1,48 bis 2,48 Gew.-% 5-Methyloctanol;
- 0,70 bis 2,70 Gew.-%, bevorzugt 0,90 bis 2,50 Gew.-%, besonders bevorzugt 1,20 bis 2,20 Gew.-% 3,6,6-Trimethylhexanol;
- 1,96 bis 3,96 Gew.-%, bevorzugt 2,16 bis 3,76 Gew.-%, besonders bevorzugt 2,46 bis 3,46 Gew.-% 7-Methyloctanol;
- 1,24 bis 3,24 Gew.-%, bevorzugt 1,44 bis 3,04 Gew.-%, besonders bevorzugt 1,74 bis 2,74 Gew.-% 6-Methyloctanol;
- 0,1 bis 3 Gew.-%, bevorzugt 0,2 bis 2 Gew.-%, besonders bevorzugt 0,3 bis 1 Gew.-% n-Nonanol;
- 25 bis 35 Gew.-%, bevorzugt 28 bis 33 Gew.-%, besonders bevorzugt 29 bis 32 Gew.-% sonstige Alkohole mit 9 und 10 Kohlenstoffatomen; mit der Maßgabe, dass die Gesamtsumme der genannten Komponenten 100 Gew.-% ergibt.

Entsprechend den vorstehenden Ausführungen kann ein Isononanolgemisch, das durch Kobalt-katalysierte Hydroformylierung und anschließende Hydrierung unter Verwendung eines Ethylenhaltigen Butengemisches als Rohstoff mittels des PolyGas®- oder EMOGAS®-Verfahrens erzeugten Isooctengemisches hergestellt wurde, im Bereich der folgenden Zusammensetzungen, abhängig von der Rohstoffzusammensetzung und Schwankungen der angewandten Reaktionsbedingungen variieren:

- 6,0 bis 16,0 Gew.-%, bevorzugt 7,0 bis 15,0 Gew.-%, besonders bevorzugt 8,0 bis 14,0 Gew.-% n-Nonanol;
- 12,8 bis 28,8 Gew.-%, bevorzugt 14,8 bis 26,8 Gew.-%, besonders bevorzugt 15,8 bis 25,8 Gew.-% 6-Methyloctanol;
- 12,5 bis 28,8 Gew.-%, bevorzugt 14,5 bis 26,5 Gew.-%, besonders bevorzugt 15,5 bis 25,5 Gew.-% 4-Methyloctanol;
- 3,3 bis 7,3 Gew.-%, bevorzugt 3,8 bis 6,8 Gew.-%, besonders bevorzugt 4,3 bis 6,3 Gew.% 2-Methyloctanol;
- 5,7 bis 11,7 Gew.-%, bevorzugt 6,3 bis 11,3 Gew.-%, besonders bevorzugt 6,7 bis 10,7 Gew.-% 3-Ethylheptanol;
- 1,9 bis 3,9 Gew.-%, bevorzugt 2,1 bis 3,7 Gew.-%, besonders bevorzugt 2,4 bis 3,4 Gew.% 2-Ethylheptanol;
- 1,7 bis 3,7 Gew.-%, bevorzugt 1,9 bis 3,5 Gew.-%, besonders bevorzugt 2,2 bis 3,2 Gew.% 2-Propylhexanol;
- 3,2 bis 9,2 Gew.-%, bevorzugt 3,7 bis 8,7 Gew.-%, besonders bevorzugt 4,2 bis 8,2 Gew.% 3,5-Dimethylheptanol;
- 6,0 bis 16,0 Gew.-%, bevorzugt 7,0 bis 15,0 Gew.-%, besonders bevorzugt 8,0 bis 14,0 Gew.-% 2,5-Dimethylheptanol;
- 1,8 bis 3,8 Gew.-%, bevorzugt 2,0 bis 3,6 Gew.-%, besonders bevorzugt 2,3 bis 3,3 Gew.% 2,3-Dimethylheptanol;
- 0,6 bis 2,6 Gew.-%, bevorzugt 0,8 bis 2,4 Gew.-%, besonders bevorzugt 1,1 bis 2,1 Gew.% 3-Ethyl-4-methylhexanol;
- 2,0 bis 4,0 Gew.-%, bevorzugt 2,2 bis 3,8 Gew.-%, besonders bevorzugt 2,5 bis 3,5 Gew.% 2-Ethyl-4-methylhexanol;
- 0,5 bis 6,5 Gew.-%, bevorzugt 1,5 bis 6 Gew.-%, besonders bevorzugt 1,5 bis 5,5 Gew.-% sonstige Alkohole mit 9 Kohlenstoffatomen;

mit der Maßgabe, dass sich die Gesamtsumme der genannten Komponenten 100 Gew.-% ergibt.

Decanol

Bei Isodecanol, welches zur Synthese der Verbindungen der allgemeinen Formel (I) eingesetzt wird, handelt es sich in der Regel nicht um eine einheitliche chemische Verbindung, sondern um ein komplexes Gemisch unterschiedlich verzweigter isomerer Decanole.

Diese werden im Allgemeinen durch die Nickel- oder Bremsted-Säure-katalysierte Trimerisierung von Propylen, beispielsweise nach dem vorstehend erläuterten PolyGas®- oder dem EMOGAS®-Verfahren, nachfolgende Hydroformylierung des dabei erhaltenen Isononen-Isomerengemisches mittels homogener Rhodium- oder Kobaltcarbonyl-Katalysatoren, vorzugsweise mittels Kobaltcarbonyl-Katalysatoren und Hydrierung des entstandenen Isodecanal-Isomerengemisches, z. B. mittels der vorstehend in Zusammenhang mit der Herstellung von $C_7$-$C_9$-Alkoholen genannten Katalysatoren und Verfahren (Ullmann's Encyclopedia of Industrial Chemistry; 5. Auflage, Bd. A1, S. 293, VCH Verlagsgesellschaft GmbH, Weinheim 1985), hergestellt. Das so produzierte Isodecanol ist im Allgemeinen stark verzweigt.

Bei 2-Propylheptanol, welches zur Synthese der Verbindungen der allgemeinen Formel (I) eingesetzt wird, kann es sich um reines 2-Propylheptanol handeln oder um Propylheptanol-Isomerengemische, wie sie im Allgemeinen bei der industriellen Herstellung von 2-Propylheptanol gebildet werden und gemeinhin ebenfalls als 2-Propylheptanol bezeichnet werden.

Reines 2-Propylheptanol kann beispielsweise durch Aldolkondensation von n-Valeraldehyd und nachfolgende Hydrierung des dabei gebildeten 2-Propylheptenals, beispielsweise gemäß US-A 2921089, erhalten werden. Im Allgemeinen enthält kommerziell erhältliches 2-Propylheptanol neben der Hauptkomponente 2-Propylheptanol herstellungsbedingt eines oder mehrere der 2-Propylheptanol-Isomeren 2-Propyl-4-methylhexanol, 2-Propyl-5-methylhexanol, 2-Isopropyl-heptanol, 2-Isopropyl-4-methylhexanol, 2-Isopropyl-5-methylhexanol und/oder 2-Propyl-4,4-dimethyl-pentanol. Die Anwesenheit anderer Isomere des 2-Propylheptanols, beispielsweise 2-Ethyl-2,4-dimethylhexanol, 2-Ethyl-2-methyl-heptanol und/oder 2-Ethyl-2,5-dimethylhexanol im 2-Propylheptanol, ist möglich, aufgrund der geringen Bildungsraten der aldehydischen Vorläufer dieser Isomere im Zuge der Aldolkondensation sind diese, wenn überhaupt, nur in Spurenmengen im 2-Propylheptanol enthalten und spielen für die Weichmachereigenschaften des aus solchen 2-Propyheptanol-Isomerengemischen hergestellten Verbindungen praktisch keine Rolle.

Als Ausgangsmaterial zur Herstellung von 2-Propylheptanol können verschiedenerlei Kohlenwasserstoffquellen benutzt werden, beispielsweise 1-Buten, 2-Buten, Raffinat I - ein aus dem $C_4$-Schnitt eines Crackers nach Abtrennung von Allenen, Acetylenen und Dienen erhaltenes Alkan/Alken-Gemisch, das neben 1- und 2-Buten noch erhebliche Mengen an Isobuten enthält - oder Raffinat II, das aus Raffinat I durch Abtrennung von Isobuten erhalten wird und als Olefinkomponenten außer 1- und 2-Buten nur noch geringe Anteile an Isobuten enthält. Selbstverständlich können auch Gemische aus Raffinat I und Raffinat II als Rohstoff zur 2-Propylheptanol-Herstellung verwendet werden. Diese Olefine oder Olefingemische können nach an sich herkömmlichen Methoden mit Kobalt- oder Rhodium-Katalysatoren hydroformyliert werden, wobei aus 1-Buten ein Gemisch aus n- und iso-Valeraldehyd - die Bezeichnung iso-Valeraldehyd bezeichnet die Verbindung 2-Methylbutanal - gebildet wird, dessen n/iso-Verhältnis je nach verwendetem Katalysator und Hydroformylierungsbedingungen in relativ weiten Grenzen variieren kann. Beispielsweise wird bei Verwendung eines mit Triphenylphosphin modifizierten homogenen Rhodium-Katalysators (Rh/TPP) aus 1-Buten n- und iso-Valeraldehyd in einem n/iso-Verhältnis von im Allgemeinen 10 : 1 bis 20 : 1 gebildet, wohingegen bei Verwendung von mit Phosphit-Liganden, beispielsweise gemäß US-A 5288918 oder WO 05028407, oder von mit Phosphoamidit-Liganden, beispielsweise gemäß WO 0283695, modifizierten Rhodium-Hydroformylierungskatalysatoren fast ausschließlich n-Valeraldehyd gebildet wird. Während das Rh/TPP-Katalysatorsystem 2-Buten bei der Hydroformylierung nur sehr langsam umsetzt, so dass der größte Teil des 2-Butens aus dem Hydroformylierungsgemisch wieder zurückgewonnen werden kann, gelingt die Hydroformylierung des 2-Butens mit den erwähnten Phosphit-Ligand- oder Phosphoramidit-Ligand-modifizierten Rhodium-Katalysatoren, wobei vorwiegend n-Valeraldehyd gebildet wird. Hingegen wird im olefinischen Rohstoff enthaltenes Isobuten, wenn auch mit unterschiedlicher Geschwindigkeit, von praktisch allen Katalysatorsystemen zu 3-Methylbutanal und je nach Katalysator in geringerem Umfang zu Pivalaldehyd hydroformyliert.

Die je nach verwendeten Ausgangsmaterialien und Katalysatoren erhaltenen Cs-Aldehyde, d. h. n-Valeraldehyd gegebenenfalls im Gemisch mit iso-Valeraldehyd, 3-Methylbutanal und/oder Pivalaldehyd, können vor der Aldolkondensation gewünschtenfalls vollständig oder teilweise destillativ in die Einzelkomponenten aufgetrennt werden, so dass auch hier eine Möglichkeit besteht, die Isomerenzusammensetzung der $C_{10}$-Alkoholkomponente der offenbarungsgemäßen Estergemische zu beeinflussen und zu steuern. Desgleichen ist es möglich, das Cs-Aldehydgemisch, wie es bei der Hydroformylierung gebildet wird, ohne die vorherige Abtrennung einzelner Isomere der Aldolkondensation zuzuführen. Bei der Aldolkondensation, die mittels eines basischen Katalysators, wie einer wässrigen Lösung von Natrium- oder Kaliumhydroxid, beispielsweise nach den in EP-A 366089, US-A 4426524 oder US-A 5434313 beschriebenen Verfahren durchgeführt werden kann, entsteht bei Einsatz von n-Valeraldehyd als einziges Kondensationsprodukt 2-Propylheptenal, wohingegen bei Einsatz eines Gemisches isomerer Cs-Aldehyde ein Isomerengemisch aus den Produkten der Homoaldolkondensation gleicher Aldehydmoleküle und der gekreuzten Aldolkondensation unterschiedlicher Valeraldehyd-Isomere geformt wird. Selbstverständlich kann die Aldolkondensation durch die gezielte Umsetzung einzelner Isomere so gesteuert werden, dass überwiegend oder vollständig ein einzelnes Aldolkondensationsisomer gebildet wird. Die betreffenden Aldolkondensationsprodukte können anschließend, üblicherweise nach vorausgegangener, meist destillativer Abtrennung aus der Reaktionsmischung und gewünschtenfalls destillativer Reinigung, mit herkömmlichen Hydrierkatalysatoren, beispielsweise den vorstehend zur Hydrierung von Aldehyden genannten, zu den entsprechenden Alkoholen oder Alkoholgemischen hydriert werden.

Wie bereits erwähnt, können die in der offenbarten Weichmacher-Zusammensetzung enthaltenen Verbindungen der allgemeinen Formel (II) mit reinem 2-Propylheptanol verestert sein. Im Allgemeinen werden zur Herstellung dieser Ester jedoch Gemische des 2-Propylheptanols mit den genannten Propylheptanol-Isomeren eingesetzt, in denen der Gehalt an 2-Propylheptanol mindestens 50 Gew.-% beträgt. Es kann bevorzugt sein, dass der Gehalt an 2-Propylheptanol 60 bis 98 Gew.-% und weiter bevorzugt 80 bis 95 Gew.-% und besonders bevorzugt 85 bis 95 Gew.-% beträgt.

Geeignete Mischungen von 2-Propylheptanol mit den Propylheptanol-Isomeren umfassen beispielsweise solche aus 60 bis 98 Gew.-% 2-Propylheptanol, 1 bis 15 Gew.-% 2-Propyl-4-methyl-hexanol und 0,01 bis 20 Gew.-% 2-Propyl-5-methyl-hexanol und 0,01 bis 24 Gew.-% 2-Isopropy-Iheptanol, wobei die Summe der Anteile der einzelnen Bestandteile 100 Gew.-% nicht überschreitet. Es kann bevorzugt sein, dass sich die Anteile der einzelnen Bestandteile zu 100 Gew.-% addieren.

Weitere geeignete Mischungen aus 2-Propylheptanol mit den Propylheptanol-Isomeren umfassen beispielsweise solche aus 75 bis 95 Gew.-% 2-Propylheptanol, 2 bis 15 Gew.-% 2-Propyl-4-methyl-hexanol, 1 bis 20 Gew.-% 2-Propyl-5-methyl-hexanol, 0,1 bis 4 Gew.-% 2-Isopropylheptanol, 0,1 bis 2 Gew.-% 2-Isopropyl-4-methylhexanol und 0,1 bis 2 Gew.-% 2-Isopropyl-5-methyl-hexanol, wobei die Summe der Anteile der einzelnen Bestandteile 100 Gew.-% nicht überschreitet. Es kann bevorzugt sein, dass sich die Anteile der einzelnen Bestandteile zu 100 Gew.-% addieren.

Es kann bevorzugt sein, dass Mischungen von 2-Propylheptanol mit den Propylheptanol-Isomeren solche mit 85 bis 95 Gew.-% 2-Propylheptanol, 5 bis 12 Gew.-% 2-Propyl-4-methyl-hexanol und 0,1 bis 2 Gew.-% 2-Propyl-5-methylhexanol und 0,01 bis 1 Gew.-% 2-Isopropylheptanol umfassen, wobei die Summe der Anteile der einzelnen Bestandteile 100 Gew.-% nicht überschreitet. Es kann bevorzugt sein, dass sich die Anteile der einzelnen Bestandteile zu 100 Gew.-% addieren.

Bei Verwendung der genannten 2-Propylheptanol-Isomerengemische anstelle von reinem 2-Propylheptanol zur Herstellung der Verbindungen der allgemeinen Formeln (II) entspricht die Isomerenzusammensetzung der Alkylestergruppen bzw. Alkylethergruppen praktisch der Zusammensetzung der zur Veresterung verwendeten Propylheptanol-Isomerengemische.

Undecanol

Die Undecanole, die zur Herstellung der Verbindungen der allgemeinen Formel (I) eingesetzt werden, können geradkettig oder verzweigt sein oder aus Gemischen geradkettiger und verzweigter Undecanole zusammengesetzt sein. Es kann bevorzugt sein, dass Gemische aus verzweigten Undecanolen, auch als Isoundecanol bezeichnet, als Alkoholkomponente verwendet werden.

Im Wesentlichen geradkettiges Undecanol kann beispielsweise durch die Rhodium- oder vorzugsweise Kobaltkatalysierte Hydroformylierung von 1-Decen und nachfolgende Hydrierung des dabei erhaltenen n-Undecanals erhalten werden. Das Ausgangsolefin 1-Decen wird beispielsweise über das zuvor bei der Herstellung von 1-Octen erwähnte SHOP-Verfahren hergestellt.

Zur Herstellung verzweigten Isoundecanols kann das im SHOP-Verfahren erhaltene 1-Decen einer Skelettisomerisierung, z. B. mittels acider zeolithischer Molekularsiebe, wie in WO 9823566 beschrieben, unterzogen werden, wobei sich Gemische aus isomeren Decenen bilden, deren Rhodium- oder vorzugsweise Kobalt-katalysierte Hydroformylierung und nachfolgende Hydrierung der erhaltenen Isoundecanal-Gemische zudem zur Herstellung der Verbindungen der allgemeinen Formel (I) verwendeten Isoundecanols führt. Die Hydroformylierung von 1-Decen oder Isodecen-Gemischen mittels Rhodium- oder Kobalt-Katalyse kann wie zuvor in Zusammenhang mit der Synthese von $C_7$- bis $C_{10}$-Alkoholen beschrieben erfolgen. Entsprechendes gilt für die Hydrierung von n-Undecanal oder Isoundecanal-Gemischen zu n-Undecanol bzw. Isoundecanol.

Nach destillativer Reinigung des Austrags der Hydrierung können die so erhaltenen $C_7$- bis $C_{11}$-Alkylalkohole bzw. deren Gemische, wie vorstehend beschrieben, zur Herstellung der Verbindungen der allgemeinen Formel (I) verwendet werden.

Dodecanol

Im Wesentlichen geradkettiges Dodecanol kann beispielsweise über das Alfol®- oder Epal®-Verfahren gewonnen werden. Diese Verfahren beinhalten die Oxidation und Hydrolyse geradkettiger Trialkylaluminium-Verbindungen, welche ausgehend von Triethylaluminium schrittweise über mehrere Ethylierungsreaktionen unter Verwendung von Ziegler-Natta-Katalysatoren aufgebaut werden. Aus den daraus resultierenden Gemischen weitgehend geradkettiger

Alkylalkohole unterschiedlicher Kettenlänge kann nach dem destillativen Austrag der $C_{12}$-Alkylalkohol-Fraktion das gewünschte n-Dodecanol erhalten werden.

Alternativ kann n-Dodecanol auch durch Hydrogenierung natürlicher Fettsäuremethylester, beispielsweise aus Kokosnussöl, hergestellt werden.

Verzweigtes Isododecanol kann analog zu den bekannten Verfahren zur Codimerisierung und/oder Oligomerisierung von Olefinen, wie beispielsweise in der WO 0063151 beschrieben, mit nachfolgender Hydroformylierung und Hydrierung der Isoundecen-Gemische, wie beispielsweise in der DE-A 4339713 beschrieben, erhalten werden. Nach destillativer Reinigung des Austrags der Hydrierung können die so erhaltenen Isododecanole bzw. deren Gemische, wie vorstehend beschrieben, zur Herstellung der Verbindungen der allgemeinen Formel (I) verwendet werden.

## Beispiele

Die Beispiele dienen der Veranschaulichung der Erfindung ohne diese einzuschränken.

## GC-MS

### $C_8$-Diester und $C_{10}$-Diester (verzweigt) mit Cyclohexanonkern

Für die Gaschromatographie wurde ein Gaschromatograph von Fa. Agilent 6890series und als Säule Optima 5 Amin (Länge = 30m, Innendurchmesser = 0,25mm, Außendurchmesser = 0,40 mm, Filmdicke = 0,5µm) der Fa. Macherey&Nagel (Best.Nr. 726354.30) verwendet. Als Injektor diente ein Split/Splitless mit Topaz Split Precision Liner Whool der Fa. Restek (# 23305).

Die Injektionsbedingungen waren: Injektortemperatur = 280°C, Injektionsvolumen = 1µL, Split 1:50, Splitflow 150mL/min, Septum Purge 3,0 mL/min (gemessen bei Ofentemp. 80°C). Als Trägergas diente Stickstoff 28PSI = 3,0mL/min (gemessen bei Ofentemp. 80 °C).

Das Temperaturprogramm war: Start: 60°C, Verweildauer 1: 5min, Temperaturrampe 1: 8°C/min, Endtemperatur 1: 240°C, Verweildauer 2: amin, Temperaturrampe 2: 30°C/min, Endtemperatur 2: 300°C, Verweildauer 3: 30min, Gesamtlaufzeit: 59,5min.

Die Detektion erfolgte mittels FID mit 300mL/min Luft, 30mL/min Wasserstoff und 30ml/min Make-Up-Gas (Stickstoff) bei 320°C.

### $C_{10}$-Diester (linear) mit Cyclohexanonkern

Für die Gaschromatographie wurde ein Gaschromatograph von Fa. Agilent 6890series und als Säule Optima 1 (Länge = 10m, Innendurchmesser = 0,25mm, Außendurchmesser = 0,40 mm, Filmdicke = 0,25µm) der Fa. Macherey&Nagel (Best.Nr. 726050.10) verwendet. Als Injektor diente ein Split/Splitless mit Topaz Split Precision Liner Whool der Fa. Restek (# 23305).

Die Injektionsbedingungen waren: Injektortemperatur = 280°C, Injektionsvolumen = 1µL, Split 1:50, Splitflow 53,6 mL/min, Septum Purge 2,6 mL/min (gemessen bei Ofentemp. 80°C). Als Trägergas diente Stickstoff 5,1PSI = 1,0mL/min (gemessen bei Ofentemp. 80 °C). Das Temperaturprogramm war: Start: 60°C, Verweildauer 1: amin, Temperaturrampe 1: 15°C/min, Endtemperatur 1: 320°C, Verweildauer 2: 10min, Gesamtlaufzeit: 27,3min.

Die Detektion erfolgte mittels FID mit 300mL/min Luft, 30mL/min Wasserstoff und 30ml/min Make-Up-Gas (Stickstoff) bei 320°C.

### $C_8$-Diester mit Cyclopentanonkern und $C_8$-Tetraester mit Cyclohexanonkern

Für die Gaschromatographie wurde ein Gaschromatograph von Fa. Agilent 6890series und als Säule Optima 1 (Länge = 10m, Innendurchmesser = 0,25mm, Außendurchmesser = 0,40 mm, Filmdicke = 0,25µm) der Fa. Macherey&Nagel (Best.Nr. 726050.10) verwendet. Als Injektor diente ein Split/Splitless mit Topaz Split Precision Liner Whool der Fa. Restek (# 23305).

Die Injektionsbedingungen waren: Injektortemperatur = 280°C, Injektionsvolumen = 1µL, Split 1:50, Splitflow 50mL/min, Septum Purge 2,6 mL/min (gemessen bei Ofentemp. 80°C). Als Trägergas diente Stickstoff 5,1PSI = 1,0mL/min (gemessen bei Ofentemp. 80 °C).

Das Temperaturprogramm war: Start: 80°C, Verweildauer 1: 5min, Temperaturrampe 1: 20°C/min, Endtemperatur 1: 320°C, Verweildauer 2: 20min, Gesamtlaufzeit: 37,0min.

Die Detektion erfolgte mittels FID mit 300mL/min Luft, 30mL/min Wasserstoff und 30ml/min Make-Up-Gas (Stickstoff) bei 320°C.

$C_1$-Diester mit Cyclohexanonkern

Für die Gaschromatographie wurde ein Gaschromatograph von Fa. Agilent 6890series und als Säule Optima 5 Amin (Länge = 30m, Innendurchmesser = 0,25mm, Außendurchmesser = 0,40 mm, Filmdicke = 0,5μm) der Fa. Macherey&Nagel (Best.Nr. 726354.30) verwendet. Als Injektor diente ein Split/Splitless mit Topaz Split Precision Liner Whool der Fa. Restek (# 23305).

Die Injektionsbedingungen waren: Injektortemperatur = 280°C, Injektionsvolumen = 1μL, Split 1:50, Splitflow 150mL/min, Septum Purge 3,0 mL/min (gemessen bei Ofentemp. 80°C). Als Trägergas diente Stickstoff 28PSI = 3,0mL/min (gemessen bei Ofentemp. 80 °C).

Das Temperaturprogramm war: Start: 60°C, Verweildauer 1: 5min, Temperaturrampe 1: 8°C/min, Endtemperatur 1: 240°C, Verweildauer 2: amin, Temperaturrampe 2: 30°C/min, Endtemperatur 2: 300°C, Verweildauer 3: 10min, Gesamtlaufzeit: 39,5min.

Die Detektion erfolgte mittels FID mit 300mL/min Luft, 30mL/min Wasserstoff und 30ml/min Make-Up-Gas (Stickstoff) bei 320°C.

$C_9$-Diester mit Cyclohexanonkern

Für die Gaschromatographie wurde ein Gaschromatograph von Fa. Agilent 6890series und als Säule Optima 1 (Länge = 25m, Innendurchmesser = 0,25mm, Außendurchmesser = 0,40 mm, Filmdicke = 0,25μm) der Fa. Macherey&Nagel (Best.Nr. 726050.25) verwendet. Als Injektor diente ein Split/Splitless mit Topaz Split Precision Liner Whool der Fa. Restek (# 23305).

Die Injektionsbedingungen waren: Injektortemperatur = 280°C, Injektionsvolumen = 1μL, Split 1:36, Splitflow 25,2mL/min, Septum Purge 2,0 mL/min (gemessen bei Ofentemp. 80°C). Als Trägergas diente Stickstoff 8,3PSI = 0,7mL/min (gemessen bei Ofentemp. 80 °C).

Das Temperaturprogramm war: Start: 80°C, Verweildauer 1: 5min, Temperaturrampe 1: 8°C/min, Endtemperatur 1: 300°C, Verweildauer 2: 15min, Gesamtlaufzeit: 47,5min.

Die Detektion erfolgte mittels FID mit 300mL/min Luft, 30mL/min Wasserstoff und 30ml/min Make-Up-Gas (Stickstoff) bei 320°C.

$C_9$- Monoester und $C_{10}$-Monoester mit Cyclohexanonkern

Für die Gaschromatographie wurde ein Gaschromatograph von Fa. Agilent 6890series und als Säule Optima 5 Amin (Länge = 30m, Innendurchmesser = 0,25mm, Außendurchmesser = 0,40 mm, Filmdicke = 0,5μm) der Fa. Macherey&Nagel (Best.Nr. 726354.30) verwendet. Als Injektor diente ein Split/Splitless mit Topaz Split Precision Liner Whool der Fa. Restek (# 23305).

Die Injektionsbedingungen waren: Injektortemperatur = 280°C, Injektionsvolumen = 1μL, Split 1:50, Splitflow 150mL/min, Septum Purge 3,0 mL/min (gemessen bei Ofentemp. 80°C). Als Trägergas diente Stickstoff 28PSI = 3,0mL/min (gemessen bei Ofentemp. 80 °C).

Das Temperaturprogramm war: Start: 60°C, Verweildauer 1: 5min, Temperaturrampe 1: 8°C/min, Endtemperatur 1: 240°C, Verweildauer 2: amin, Temperaturrampe 2: 30°C/min, Endtemperatur 2: 300°C, Verweildauer 3: 10min, Gesamtlaufzeit: 59,5min.

Die Detektion erfolgte mittels FID mit 300mL/min Luft, 30mL/min Wasserstoff und 30ml/min Make-Up-Gas (Stickstoff) bei 320°C.

Synthese

Einsatzstoffe:

| Verbindung | Hersteller | Reinheit | Kommentar |
|---|---|---|---|
| Enamin aus Cyclohexanon u. Pyrrolidin (1-(1-Cyclohexen-1-yl)pyrrolidin) | Sigma-Aldrich (SAFC) | 97%ig | |
| Methylacrylat | BASF | 100% | MEHQ (15 ppm) |

(fortgesetzt)

| Verbindung | Hersteller | Reinheit | Kommentar |
|---|---|---|---|
| Kaliumphosphat | Fa. Budenheim | | |
| Tyzor TPT-20B | | | |
| 2-Ethylhexylacrylat | BASF | 99,9% | MEHQ (15 ppm) |
| Cyclohexanon | Sigma Aldrich | Min. 99,5% | |
| 2-Propylheptylacrylat | BASF | Etwa 97%ig | MEHQ (320 ppm) |
| Pyrrolidin | Sigma-Aldrich | Min. 99% | |
| Cyclopentanon | BASF | 100% (GC) | |
| Isononanol | BASF | 100% (GC) | |
| Isononylacrylat | BASF | >99% | MEHQ (120 ppm) |
| Acrylnitril | BASF | 94,8 % | MEHQ (30-45 ppm) |
| Lorol C10 (n-Decanol) | BASF | 97,7% | Charge 0017729669 |
| Acrylsäure | BASF | glacial | MEHQ (15 ppm) |
| Methacrylsäure | BASF | glacial | MEHQ (15 ppm) |
| 2-Propylheptanol | BASF | 99,5% | CAS 10042-59-8 |
| Nonanol N (iso-Nonanol) | BASF | | CAS 27458-94-2, Isoindex 1,2 |

Synthese von (Meth)acrylaten:

Die Synthese der (Meth)acrylate fand unter Einleitung von Luft statt. Der Fortschritt der Reaktion wurde durch Probennahme and Analyse der Proben mittels GC oder GC-MS bestimmt.

Isononylmethacrylat:

0,29 g Methylhydrochinon (MEHQ) und 1400 g Methylmethacrylat (stabilisiert mit 15 ppm MEHQ) wurden bei Raumtemperatur vorgelegt. 1010 g Nonanol N (CAS: 27458-94-2, Isomerengemisch, Isoindex 1,2) und 29,7 g Kaliumphosphat wurden zugegeben und das Reaktionsgemisch bei einer Badtemperatur von 90 °C aufgeheizt. Es wurde ein Druck von 350 mbar (abs.) eingestellt und kontinuierlich ein Azeotrop aus Methanol und Methylmethacrylat abdestilliert. Nach Beendigung der Reaktion wurde überschüssiges Methylmethacrylat bei 60 °C Badtemperatur im Vakuum abdestilliert. Das Produkt wurde nach Abkühlen auf Raumtemperatur drei Mal mit je 500 mL Wasser gewaschen und die wässrigen Phasen jeweils abgetrennt und verworfen. Der so erhaltenen organischen Phase wurden 120 mg MEHQ zugesetzt, der Wasserrest im Vakuum abdestilliert und das dünnflüssige Produkt über einen Papierfilter filtriert.
Das Produkt Isononylmethacrylat wurde in einer Ausbeute 1474 g (99 %) mit einer Reinheit von > 99 GC Flächen-% erhalten. Der MEHQ-Gehalt betrug 120 ppm.

2-Propylheptylacrylat:

Ethylacrylat (2403 g), MeHQ (2,29 g) und 2-Propylheptanol (1900 g) wurden vorgelegt. 200g Ethylacrylat wurden zwecks Entwässerung abdestilliert und anschließend wieder ergänzt. Nach Abkühlen auf Raumtemperatur wurde Titantetraisopropoxylat (35,16 g) zudosiert und unter Magerlufteileitung und Rühren bei einem Vakuum von zunächst 600 mbar auf eine Sumpftemperatur von 92°C erhitzt. Das Vakuum wurde auf 300 mbar reduziert und die Sumpftemperatur auf 104 °C erhöht. Nach Beendigung der Reaktion wurde die Reaktionsmischung mit 300 ml Wasser versetzt, das Wasser und überschüssiges Ethylacrylat nach Hydrolyse des Katalysators abdestilliert und die Mischung über einen Tiefenfilter filtriert.
Das Produkt wird in 2320 g (91,1% Ausbeute) und einer Reinheit von 97,7 GC Flächgen-% erhalten. Der MEHQ-

Gehalt betrug 320 ppm.

N-Decylacrylat:

Ethylacrylat (2673 g), MeHQ (1,75 g) und Lorol C10 (1448 g) vorgelegt. 200g Ethylacrylat werden zwecks Entwässerung abdestilliert. Bei einer Sumpftemperatur von 88°C wird Titantetraisopropoxylat (13,4 g) zudosiert und unter Magerlufteileitung und Rühren bei einem Vakuum von zunächst 850 mbar auf eine Sumpftemperatur von 94°C hochgeheizt. Das Vakuum wurde auf 700 mbar reduziert und die Sumpftemperatur auf 104 °C erhöht. Nach Beendigung der Reaktion wurde die Reaktionsmischung mit 150 ml Wasser versetzt, das Wasser und überschüssiges Ethylacrylat nach Hydrolyse des Katalysators abdestilliert und die Mischung über einen Tiefenfilter filtriert.
Das Produkt wird in 1874 g (96,5% Ausbeute) und einer Reinheit von 99,7 GC Flächen-% erhalten. Der MEHQ-Gehalt beträgt ca. 900 pmm (HPLC).

## Versuch 1: Herstellung $C_8$-Diester

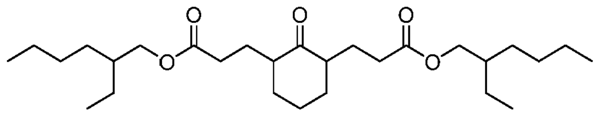

1-(1-Cyclohexen-1-yl)pyrrolidin (227 g, 1.50 mol, 1.0 Äq.) wurde in Isopropanol (2.5 L) bei Raumtemperatur gelöst. 2-Ethylhexylacrylat (500 g, 2.71 mol, 1.8 Äq.) wurde langsam zudosiert. Die Reaktionsmischung wurde auf 52°C (Heizbadtemperatur) erhitzt und 163 g 2-Ethylhexylacrylat langsam über 1 h zudosiert. Die Heizbadtemperatur wurde auf 58° erhöht und die Reaktionsmischung für 5.5 h gerührt. Im Anschluss wurde die Reaktionsmischung bei Raumtemperatur über Nacht gerührt. Das Isopropanol wurde destillativ entfernt und der Rückstand in Toluol aufgenommen. Salzsäure (32%, 194 g, 1.7 mol) wurde innerhalb von 30 min zugetropft und die Mischung für weitere 30 min gerührt. Die wässrige Phase wurde abgetrennt und die organische Phase mit Wasser gewaschen und über Natriumsulfat getrocknet. Die organische Phase wurde eingeengt und mit MeHQ (0.037 g) versetzt. Das darin enthaltene überschüssige 2-Ethylhexylacrylat und Toluol wurden mittels Wasserdampfdestillation entfernt. Restliche Feuchtigkeit wurde durch Ausblasen mit Stickstoff (bei 195 °C für 20 min) entfernt. Der Rückstand wurde mit Aluminiumoxid (neutral, 60 g) versetzt und über einen mit Aluminiumoxid (neutral, 250 g) überdeckten Aktivkohlefilter filtriert.

Ausbeute: 382 g (0.82 mol, 55%), Reinheit: 99% (GC)

## Versuch 2: Herstellung von $C_1$-Diester

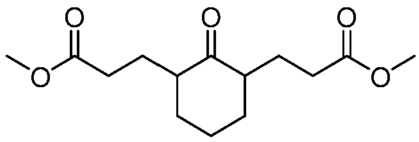

Die Reaktion wurde unter eine Stickstoffatmosphäre durchgeführt. 1-(1-Cyclohexen-1-yl)pyrrolidin (529.4 g, 3,44 mol, 1.0 Äq.) wurde in 900 mL Methanol bei Raumtemperatur gelöst. Über einen Zeitraum von 2.5 h wurde Methylacrylat (662.9 g, 7.70 mol, 2.24 Äq.) zugetropft. Die Reaktionsmischung wurde über Nacht bei Raumtemperatur gerührt. 0.65 mol (237 g) 10%ige Salzsäure wurde über 1 h unter Kühlung (Eiswasser) zudosiert. Die Reaktionsmischung wurde anschließend für 100 min bei Raumtemperatur gerührt. 552 mL Wasser wurden zugegeben und die Reaktionsmischung wurde anschließend über Nacht bei Raumtemperatur gerührt. Das Produkt wurde abgesaugt, mit Wasser, mit HCl (5%) und nochmals mit Wasser gewaschen. Das Produkt wurde im Vakuum bei 50°C über Nacht getrocknet.
Ausbeute: 85%; Reinheit:100% (GC)

Versuch 3: Umesterung von $C_1$-Diester zu $C_8$-Diester

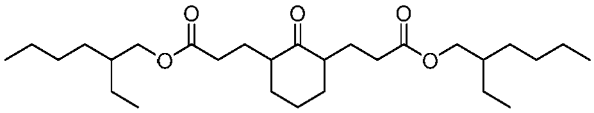

Die Reaktion wurde unter eine Stickstoffatmosphäre durchgeführt. 2-Ethyl-1-hexanol (781.4 g, 6.0 mol, 3.0 Äq.) wurde mit einem Dimethylester gemäß Versuch 2 (540.6 g, 2.0 mol, 1.0 Äq.) und Kaliumphosphat (38.2 g, 0.18 mol, 0.09 Äq.) versetzt. Die Reaktionsmischung wurde unter Rühren für etwa 4 h erhitzt (Bad-Temperatur: 150-180 °C), sodass das entstehende Methanol kontinuierlich abdestilliert wurde. Das überschüssige 2-Ethylhexanol wurde destillativ entfernt. n-Heptan wurde zugegeben und das Reaktionsgemisch für 30 min gerührt. Hyflo Super Cel wurde zugegeben und die Mischung für 1 h bei 50 °C gerührt. Die Mischung wurde über einen unbeheizten Seitz-Filterhalter (Filterplatte K 100, bis 2.8 bar). langsam aber kontinuierlich filtriert. Das n-Heptan wurde abdestilliert.

Das Produkt wurde bei 0.2 mbar und 214°C mit 700 U/min in einer Sambay-Apparatur destilliert. Dabei wurden die Leichtsieder über Kopf entfernt. Das Sumpfprodukt wurde in einer zweiten Sambay-Destillation bei 0.1 mbar und 234 °C mit 700 U/min über Kopf destilliert.

Ausbeute: 60-70%, Reinheit: 99.2% (GC)

Versuch 4: Herstellung $C_9$-Diester

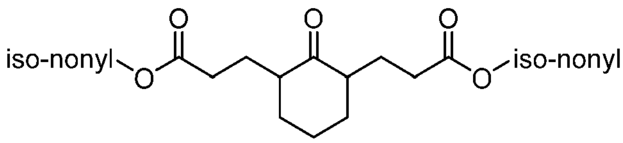

Unter einer Stickstoffatmosphäre wurde Dimethylester gemäß Versuch 2 (216 g, 0.80 mol, 1.0 Äq) mit Isononanol (346 g, 2,4 mol, 3.0 Äq) und Magnesiumcarbonat (2.0 g) versetzt und unter Rühren erhitzt. Die Temperatur wurde so eingestellt, dass ein kontinuierliches abdestillieren des entstehenden Methanols stattfand (200-220°C). Nach 7 h wurde das überschüssige Isononanol unter Vakuum destillativ entfernt. Der noch 100 °C heiße Rückstand wurde über eine Filterplatte geklärt. Nach einer Sambay-Destillation (210 °C, 0.2-0.3 mbar) wurde das Produkt als gelbe Flüssigkeit erhalten.

Aubeute: 80 %, Reinheit: 92 Fl.-% (GC)

Versuch 5: Herstellung $C_{10}$-Diester (verzweigt)

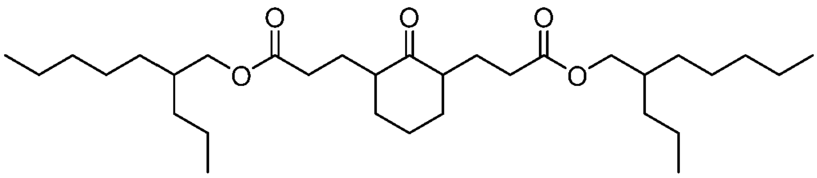

1-(1-Cyclohexen-1-yl)pyrrolidin (227 g, 1.50 mol, 1.0 Äq.) wurde in Isopropanol (2.0 L) bei Raumtemperatur gelöst. Das Heizbad wurde auf 65°C erwärmt. 2-Propylheptylacrylat (733 g, 3.45 mol, 2.3 Äq.) wurde bereits während der Aufheizzeit zudosiert. Die Dosierung wurde innerhalb von 2h beendet. Die Reaktionsmischung wurde für weitere 2 h bei 53 °C und dann bei Raumtemperatur über 2 Tage gerührt. Das Lösungsmittel wurde destillativ entfernt und der Rückstand in Toluol aufgenommen. Salzsäure (32%, 194 g, 1.7 mol) wurde innerhalb von 30 min zugetropft und im Anschluss Wasser hinzugegeben. Die wässrige Phase wurde abgetrennt und die organische Phase mit Wasser und gesättigter Kochsalzlösung gewaschen und über Natriumsulfat getrocknet. Die organische Phase wurde über 40 g Kieselgel drucklos filtriert und der Filterkuchen mit Toluol nachgespült. Das Lösungsmittel wurde destillativ entfernt. Das enthaltene überschüssige 2-Ethylhexylacrylat und Toluol wurden mittels Wasserdampfdestillation entfernt. Der Rückstand wurde über einen 2L Pall Filterhalter geklärt (Filterplatte mit Aktivkohle).

Ausbeute: 67%, Reinheit: 99% (GC)

Versuch 6: Herstellung $C_{10}$-Diester (linear)

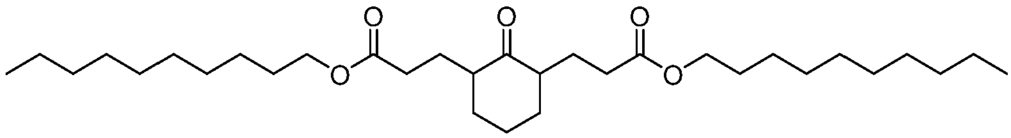

Unter einer Stickstoffatmosphäre wurde 1-(1-Cyclohexen-1-yl)pyrrolidin (231 g, 1.53 mol, 1.0 Äq.) in Isopropanol (1 L) bei Raumtemperatur gelöst. N-Decylacrylat (730 g, 3.44 mol, 2.25 Äq.) wurde über 4.6 h zudosiert. Im Anschluss wurde die Reaktionsmischung bei Raumtemperatur über Nacht gerührt. Das Isopropanol wurde destillativ entfernt und der Rückstand in Toluol (400 mL) aufgenommen. Salzsäure (32%, 278 g, 2.44 mol) wurde innerhalb von 1.5 h zugetropft und die Mischung für weitere 2 h gerührt. Die wässrige Phase wurde abgetrennt und die organische Phase mit Wasser (4 x 500 mL) und gesättigter Kochsalzlösung (1 $\times$ 300 mL) gewaschen und über Magnesiumsulfat getrocknet. Das Lösungsmittel wurde destillativ entfernt. Zur weiteren Aufreinigung wurde eine Wasserdampfdestillation (172 - 207 °C, 80 min) durchgeführt. Restliche Feuchtigkeit wurde durch Ausblasen mit Stickstoff (200 °C für 20 min) entfernt.

Ausbeute: 701 g, 1.34 mol, 88 %, Reinheit (GC): 97.7 Fl-%. Das Produkt ist ein Feststoff.

Versuch 7: Herstellung 1-(1-Cyclopenten-1-yl)pyrrolidin

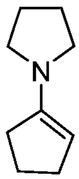

Die Reaktion wurde unter einer Stickstoffatmosphäre durchgeführt. Cyclopentanon (210 g, 2.50 mol, 1.0 Äq) wurde mit Cyclohexan (1.0 L) gemischt und bei Raumtemperatur mit Molsieb (5 A, 200 g) versetzt. Pyrrolidin (178 g, 2.50 mol, 1.0 Äq) wurde unter Rühren innerhalb von 4 h zugetropft. Die Mischung wurde bei Raumtemperatur über Nacht gerührt. Unter Stickstoffstoffschutz wurde das Molsieb über einen Faltenfilter abgetrennt. Die Leichtsieder wurden destillativ entfernt.

Ausbeute: 78 %, Reinheit: 98,2 Fl.-% (GC).

Versuch 8: Herstellung $C_8$-Diester

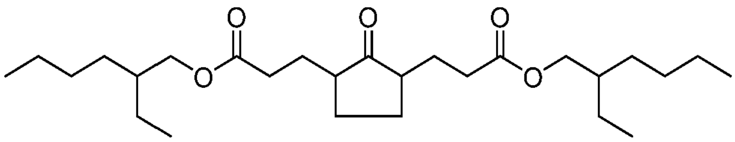

Die Reaktion wurde unter einer Stickstoffatmosphäre durchgeführt. 1-(1-Cyclopenten-1-yl)pyrrolidin (267 g, 1.91 mol, 1.0 Äq) wurde in Isopropanol (1.0 L) gelöst und auf-10 °C gekühlt. 2-Ethylhexylacrylat (670 g, 3.63 mol, 1.9 Äq) wurde bei -5 bis -11 °C über einen Zeitraum von 5 h langsam zudosiert. Das Reaktionsgemisch wurde über Nacht bei Raumtemperatur gerührt. Nach destillativer Entfernung des Lösungsmittels wurde der Rückstand in Toluol aufgenommen und über einen Zeitraum von 20 min mit Salzsäure (wässrig, 2 wt%, 228 g) versetzt. Die wässrige Phase wurde abgetrennt und die organische Phase mit Wasser gewaschen. Die organische Phase wurde über Kieselgel filtriert. Das Kieselgel wurde anschließend mit Toluol nachgespült. Die vereinigten organischen Phasen wurden am Rotationsverdampfer eingeengt. Das Produkt wurde zweimal mit Wasserdampf (156 - 175 °C, bzw. 187 °C) gestrippt.

Ausbeute: 75 %, Reinheit: 91.5 Fl.-% (GC). Nebenprodukte (GC): 6.5 Fl.-% Tetraester, 2 Fl.-% Monoester

## Versuch 9: Herstellung $C_8$-Tetraester

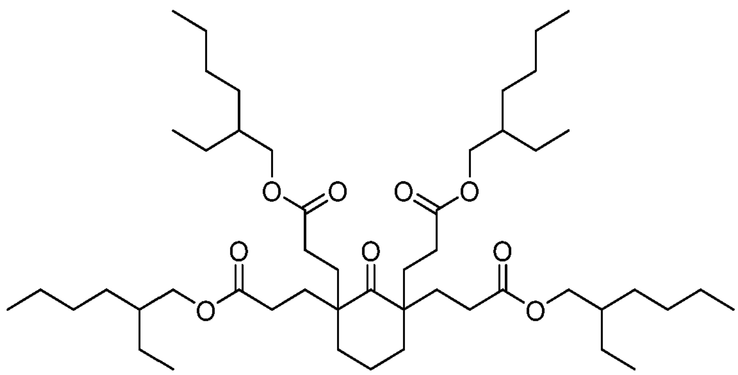

Unter einer Stickstoffatmosphäre wurde Cyclohexanon (98 g, 1.0 mol, 1.0 Äq.) in tert-Butanol (400 ml) gelöst und mit Wasser (20 ml), Natriumhydroxid (200 mg, 5.0 mmol, 0.005 Äq.) und Benzyltriethylammoniumchlorid (1.0 g, 4.39 mmol, 0.0044 Äq.) versetzt. Acrylnitril (212 g, 4.0 mol, 4.0 Äq.) wurde über 45 min bei Raumtemperatur zugetropft, wobei sich das Reaktionsgemisch auf 80 °C aufheizte. Die Reaktionsmischung rührte bei Raumtemperatur über Nacht. Nach Zugabe von Wasser (900 ml) und Salzsäure (0.5 N, 15 ml) wurde die Mischung für 2 h bei Raumtemperatur gerührt. Das ausgefallene Produkt wurde abgesaugt und mit Wasser (5x 250 ml) gewaschen. Das noch leicht wasserfeuchte Produkt wurde direkt für den nächsten Schritt verwendet.

Das noch leicht wasserfeuchte Produkt (310 g, -1.0 mol) wurde unter einer Stickstoffatmosphäre mit Salzsäure (6 N, 2.5 kg) versetzt und für 48 h bei 60 °C gerührt. Im Anschluss wurde die Reaktionsmischung für 6.5 h bei Rückfluss gerührt und dann über Nacht bei Raumtemperatur stehengelassen. Die Mischung würde mit Eiswasser (5 l) versetzt, das ausgefallene Produkt abgesaugt und mit Wasser gewaschen. Nach Trocknen im Vakuum bei 42 °C wurde das Produkt (276 g, 0.72 mol) erhalten.

Ausbeute über zwei Stufen: 72%, Reinheit: 97.6 Fl.-% (GC).

Unter einer Stickstoffatmosphäre wurde die nach Hydrolyse des tetra-Nitrils erhaltene tetra-Säure (230 g, 0.60 mol, 1.0 Äq.) mit 2-Ethylhexanol (465 g, 3.57 mol, 6.0 Äq.) und Tyzor TPT-20B (30 mg) versetzt. Das Gemisch wurde für 3 h unter Rückfluss (Sumpftemperatur 141 - 210 °C) erhitzt und dabei das entstehende Wasser entfernt. Das überschüssige 2-Ethylhexanol wurde destillativ entfernt (175-197 °C, 397-7 mbar). Das Gemisch wurde auf 80 °C gebracht und mit 2%iger Natronlauge (33 g) versetzt. Die Mischung für 10 min bei 80 °C gerührt und dann mit Wasser (23 ml) versetzt. Das Wasser wurde destillativ entfernt. Die Reaktionsmischung wurde filtriert und abschließend wurde eine Wasserdampfdestillation durchgeführt.

Ausbeute: 404 g, 81 %. Reinheit: 99.2 Fl.-% (GC).

## Versuch 10: $C_9$-Monoester

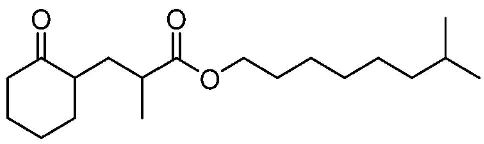

Die Reaktion wurde unter einer Stickstoffatmosphäre durchgeführt. 1-(1-Cyclohexen-1-yl)pyrrolidin (182 g, 1.20 mol, 1.0 Äq.) wurde in Isopropanol (1000 ml) vorgelegt. Isononylmethacrylat (561 g, 2.64 mol, 2.2 Äq.) wurde über 45 min bei Raumtemperatur zugetropft. Das Reaktionsgemisch wurde auf 51 °C erhitzt und für 4.5 h bei dieser Temperatur gerührt. Im Anschluss rührte das Reaktionsgemisch bei Raumtemperatur über Nacht und dann für 6.5 h bei 76 C. Das Isopropanol wurde am Rotationsverdampfer entfernt (5 mbar, 55 °C). 32%ige Salzsäure (154 g, 1.35 mol) wurde über einen Zeitraum von 2 h zu der Reaktionsmischung getropft und die Mischung im Anschluss für 1.5 h bei Raumtemperatur gerührt. Nach Zugabe von Toluol (400 ml) wurde die organische Phase abgetrennt, mit Wasser (400 ml) gewaschen und über Kieselgel (500 g) filtriert. Es wurde erneut mit Wasser (400 ml) gewaschen und das Toluol am Rotationsverdampfer (5 mbar, 55 °C) entfernt. Nach Wasserdampfdestillation (1 h, 146 - 188 °C) wurde das Produkt für 20 min unter

festem Stickstoffstrom bei 190 °C getrocknet.

Ausbeute: 237 g, Zusammensetzung (GC): 86.7 Fl.-% Monoaddukt, 8.2 Fl.-% Diaddukt, 6.2 Fl.-% Isononylmethacrylat.

## Versuch 11: $C_{10}$-Monoester

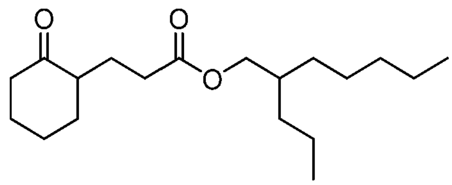

**Schritt** 1: Enamin-Bildung und Addition von Methylacrylat

Die Reaktion wurde unter einer Stickstoffatmosphäre durchgeführt. Cyclohexanon (1178 g, 12.0 mol, 1.5 Äq.) wurde vorgelegt und gerührt. Zunächst wurde Pyrrolidin (91.0 g, 1.28 mol, 0.16 Äq.) zugetropft und im Anschluss Essigsäure (9.61 g, 0.16 mol, 0.02 Äq.) zugegeben. Das Gemisch wurde auf 70 °C erhitzt und über 2.5 h Methylacrylat (689 g, 8.0 mol, 1.0 Äq.) zugetropft. Das Reaktionsgemisch wurde erhitzt und für 1.5 h unter Rückfluss gerührt. Das Gemisch wurde über Nacht stehengelassen und im Anschluss für 2 h bei 122 °C Badtemperatur und für 30 min bei 170 °C Badtemperatur gerührt. Nach Abkühlen auf Raumtemperatur wurden Cyclohexan (800 mL) und 10%ige Salzsäure (620 mL) zugegeben. Die wässrige Phase wurde abgetrennt und die organische Phase mit Wasser (3x 500 ml) und gesättigter Kochsalzlösung (500 ml) gewaschen. Nach Trocknen über Magnesiumsulfat wurde das Lösungsmittel entfernt. Das Rohprodukt wurde fraktionierend destilliert.

Ausbeute: 817 g, 4.43 mol, 55%, Reinheit (GC): 99.9 Fl-%.

**Schritt 2: Umesterung zum $C_{10}$-Monoester**

Die Reaktion wurde unter einer Stickstoffatmosphäre durchgeführt. Zu dem Methylester (369 g, 2.0 mol, 1.0 Äq.) wurde 2-Propylheptanol (633 g, 4.0 mol, 2.0 Äq.) und Tyzor TPT-20B (0.5 g) zugegeben. Das Gemisch wurde auf Rückfluss erhitzt und das dabei entstehende Methanol kontinuierlich entfernt. Überschüssige Mengen Alkohol wurden durch Vakuumdestillation entfernt (bis 214 °C, 40 mbar). Das Rohprodukt wurde im Vakuum fraktionierend destilliert (bis 185 °C, 0.12 mbar).

Ausbeute: 417 g, 1.34 mol, 67%, Reinheit (GC): 95.0 Fl-%.

Anwendungstechnische Untersuchungen

| Produkteigenschaft | Einheit | Methode |
|---|---|---|
| Dichte, 20°C | g/cm3 | DIN 51757 Verf.3, 01/11 |
| Viskosität, 20°C | mPa * s | ASTM D7042-14 |
| Brechzahl, $n_D^{20}$ | | DIN 51423 02/10 |
| Wassergehalt | Gew.% | DIN 51777-1 03/83 |
| Säurezahl | mg KOH/g | DIN EN ISO 2114 06/02 |

**(1) Bestimmung der Dichte**

Die Bestimmung der Dichte erfolgte nach DIN 51757, Januar 2011, Verfahren 3 mit Hilfe eines Stabinger Viskosimeters der Firma Anton-Paar bei 20 °C.

**(2) Bestimmung der Viskosität**

Die Bestimmung der dynamischen Viskosität erfolgte bei 20 °C nach der ASTM D7042-14 mit Hilfe eines Stabinger Viskosimeters der Firma Anton-Paar.

(3) **Bestimmung des Brechungsindex**

Die Bestimmung des Brechungsindex erfolgte nach DIN 51423, Februar 2010

(4) **Bestimmung des Wassergehaltes**

Die Bestimmung des Wassergehaltes nach Karl-Fischer erfolgte nach DIN 51777, Teil 1, März 1983.

(5) **Bestimmung der Säurezahl**

Die Bestimmung der Säurezahl erfolgte nach DIN EN ISO 2114:2002-06, Juni 2002.
Strukturformeln der untersuchten Verbindungen

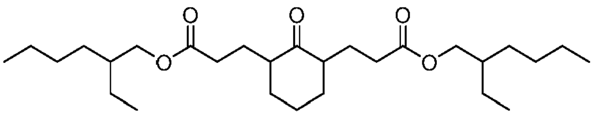

(a)

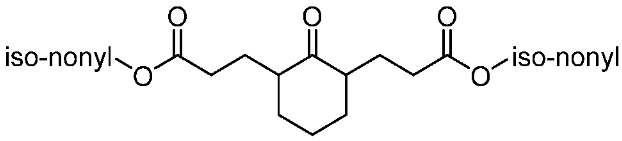

(b)

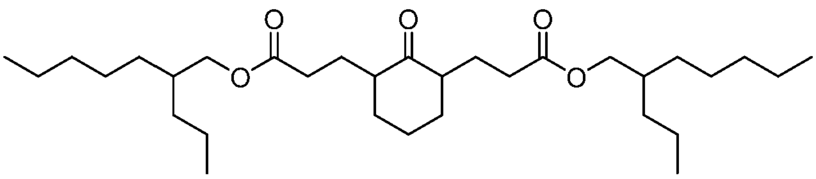

(c)

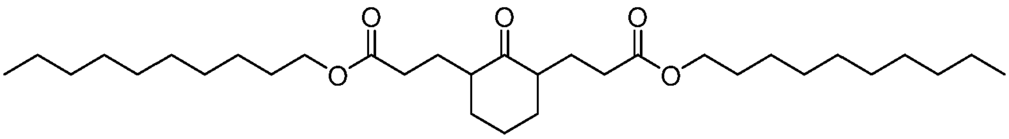

(d)

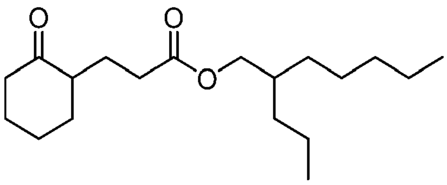

(e)

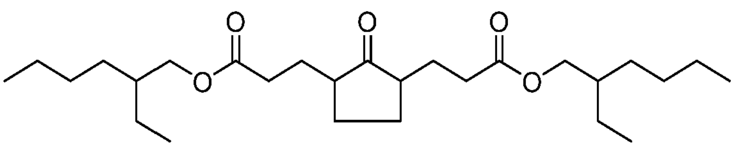

(f)

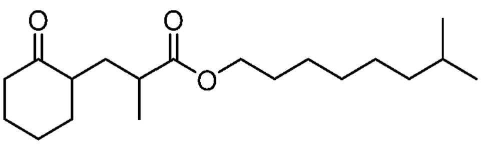

(g)

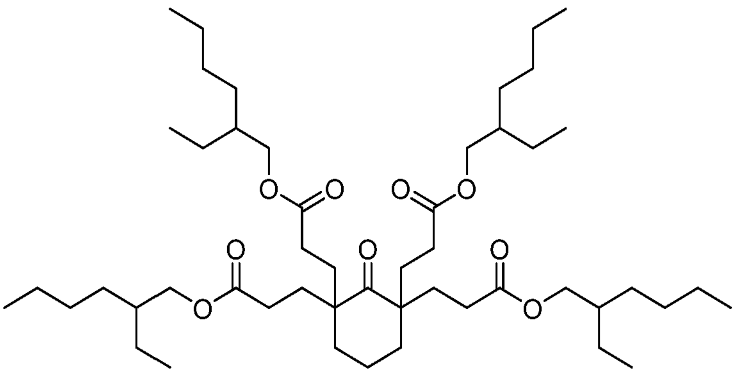

(h)

Einsatzstoffe und Abkürzungen

In den Beispielen werden folgende Einsatzstoffe verwendet:

- Suspensions-PVC-Homopolymer, Markenname Inovyn® 271 PC von INOVYN ChlorVinyls Limited, London, UK
- Suspensions-PVC-Homopolymer, Markenname Vinoflex® S7114 von BASF SE, Ludwigshafen, Germany
- Emulsions-PVC-Homopolymer, Markenname Vinnolit E 67 FF von Vinnolit GmbH & Co KG, Ismaning, Germany
- Emulsions-PVC-Homopolymer, Markenname Vestolit® E 7012 S von Vestolit GmbH; Marl, Deutschland.
- Emulsions-PVC-Homopolymer, Markenname Vestolit® P 1351 K von Vestolit GmbH; Marl, Deutschland.
- Viskositätsreduzierer, Markenname Viskobyk® 5120 von BYK Chemie GmbH, Wesel, Deutschland.
- 1,2-Cyclohexandicarbonsäure-isononylester, Markenname Hexamoll® DINCH der BASF SE, Ludwigshafen, Deutschland
- Diisononylphthalat, Markenname Palatinol® N der BASF SE, Ludwigshafen, Deutschland
- Kicker für chemische Treibmittel, Markenname Baerostab® KK 432 der Firma Baerlocher GmbH, Unterschließheim, Deutschland.
- Schäumungsmittel Azodicarbonamid, Markenname Unicell® D 200 A, Dongjin Semichem Co., Ltd., Incheon, Süd-Korea, Importeur: Tramaco GmbH, Pinneberg, Deutschland
- Ba-Zn Stabilisator, Markenname Reagens SLX/781 von Reagens S.p.A., Bologna, Italy

Anwendungstechnische Untersuchungen an Verbindungen (a) bis (h)

| | | Dichte | Viskosität | Brechzahl | Wassergehalt | Säurezahl |
|---|---|---|---|---|---|---|
| 2-Oxo-1,3-cyclohexandipropansäure 1,3-di (2-ethylhexyl ester) | (a) | 0,9766 | 95 | 1,4666 | 0,06 | 0,450 |
| 2-Oxo-1,3-cyclohexandipropansäure 1,3-diisononylester | (b) | 0,9719 | 108 | 1,4684 | 0,013 | 0,390 |
| 2-Oxo-1,3-cyclohexandipropansäure 1,3-di (2-propylheptyl ester) | (c) | 0,9583 | 128 | 1,4664 | 0,03 | 0,190 |
| 2-oxo-cyclohexanpropansäure 2-propylheptyl ester (Nicht erfindungsgemäß), | (e) | 0,9560 | 27 | 1,4637 | 0,042 | 0,39 |
| 2-Oxo-1,3-cyclopentandipropansäure 1,3-di (2-ethylhexyl ester) | (f) | 0,9756 | 75 | 1,4645 | 0,012 | 0,169 |
| α-Methyl-2-oxo-cyclohexanpropansäureisononyl ester (Nicht erfindungsgemäß), | (g) | 0,9575 | 308 | 1,4644 | 0,244 | 0,327 |

(fortgesetzt)

| | Dichte | Viskosität | Brechzahl | Wassergehalt | Säurezahl |
|---|---|---|---|---|---|
| 2-Oxo-1,1,3,3-cyclohexantetrapropansäure 1,1,3,3-tetrakis(2-ethylhexyl) ester (Nicht erfindungsgemäß), (h) | 0,9882 | 592 | 1,4729 | 0,002 | 0,6397 |

Die Viskosität ist in Fig 1 dargestellt

Untersuchungen von Plastisolen

(1) **Bestimmung** der **Lösetemperatur an PVC-Plastisolen mit** den **erfindungsgemäßen Weichmachern**

Zur Untersuchung des Gelierverhaltens von PVC-Plastisolen wurden PVC-Plastisole nach folgenden Rezepturen hergestellt:

Tabelle 1:

| | Plastisol # | | | | | | | |
|---|---|---|---|---|---|---|---|---|
| in phr | 1 | 2 | 3 | 4 | 5* | 6 | 7* | 8* |
| Vinoflex S7114, Siebfraktion<100 μm | 5 | 5 | 5 | 5 | 5 | 5 | 5 | 5 |
| Reagens SLX/781 | 0,1 | 0,1 | 0,1 | 0,1 | 0,1 | 0,1 | 0,1 | 0,1 |
| (a) | 95 | | | | | | | |
| (b) | | 95 | | | | | | |
| (c) | | | 95 | | | | | |
| (d) | | | | 95 | | | | |
| (e) | | | | | 95 | | | |
| (f) | | | | | | 95 | | |
| (g) | | | | | | | 95 | |
| (h) | | | | | | | | 95 |
| Lösetemperatur | 112 | 117 | 130 | 128 | 92 | 111 | 102 | 149 |
| *Nicht erfindungsgemäß | | | | | | | | |

Die Herstellung der Plastisole erfolgte in der Weise, dass das PVC in einem PE(Polyethylen)-Becher eingewogen wurde. Die flüssigen Komponenten wurden in einer Metallschale eingewogen. Danach wurde das PVC ebenfalls in die Metallschale überführt. Mit einem Spatel wurde vorhomogenisiert. Das entstandene Plastisol wurde in einen PE-Becher überführt. Mit Hilfe eines Rührers mit Dissolverscheibe (Fa. Jahnke & Kunkel, IKA-Werk, Typ RE-166 A, 60-6000 1/min, Durchmesser der Dissolverscheibe = 40 mm) wurde bei 2500 1/min 150 s homogenisiert. Das Plastisol wurde aus dem PE-Becher in eine Stahlschale überführt und diese in einem Exsikkator unter reduziertem Druck gesetzt. Nach dem Entweichen der Hauptmenge der im Plastisol eingeschlossenen Luft wird das Plastisol noch 15 min in dem Exsikkator bei ca. 20 mbar Vakuum belassen. Das Entweichen der im Plastisol eingeschlossenen Luft ist durch Blasenbildung an der Oberfläche des Plastisols erkennbar. Sobald keine weitere oder nur eine sehr reduzierte Blasenbildung erfolgt, ist die Hauptmenge der Luft aus dem Plastisol entwichen. Danach wird die Vakuumpumpe abgeschaltet, der Exsikkator belüftet und das Plastisol wieder in den PE-Becher überführt.

Das so hergestellte Plastisol wurde 30 min nach der Homogenisierung zur Messung der Gelierfähigkeit verwendet.

Die Viskositätsmessungen wurden mit einem beheizbaren Oszillations- und Rotations-Rheometer MCR 302 von Anton Paar in einem Rotationsversuch durchgeführt.

- Messsystem: Platte/Platte d=50 mm

(fortgesetzt)

- Scherrate: 10 $s^{-1}$
- Spaltbreite: 0,25 mm
- Ausgangstemperatur: 30 °C
- Temperaturprofil: 30 - 180 °C
- Temperatursteigerung: 5 °C/min
- Messpunkte: 600
- Messpunktdauer: 3 Sek

Die Messung erfolgte in zwei Schritten. Der erste Schritt dient zum Temperieren der Probe bei konstanten 30 °C. Bei 30 °C wird das Plastisol für 2 min mit einer Scherrate von 10 $s^{-1}$ geschert. Anschließend startet das Temperaturprogramm. Bei der Messung wird die Viskosität über die Temperatur aufgezeichnet. Die Temperatur bei der das Viskositätsmaximum erreicht ist, wird als die Lösetemperatur des Plastisols betrachtet.

Die Lösetemperatur ist in Fig 2 dargestellt

**(2) Viskositätsuntersuchung mit Lösungsmitteln**

Zur Untersuchung des rheologischen Verhaltens der erfindungsgemäßen Weichmacher in Plastisolen in Kombination mit Viskositätsreduzierern wurden PVC-Plastisole nach folgenden Rezepturen hergestellt:

Tabelle 2:

| | Plastisol # | | | | | | |
|---|---|---|---|---|---|---|---|
| in phr | 9 | 10 | 11 | 12 | 13 | 14* | 15* |
| Vestolit E 7012 S | 100 | 100 | 100 | 100 | 100 | 100 | 100 |
| (c) | 60 | 60 | 60 | 60 | 60 | | |
| Viskobyk 5120 | | 1,6 | 3,2 | 4,8 | 8 | | |
| Hexamoll DINCH | | | | | | 60 | |
| Palatinol N | | | | | | | 60 |
| *Nicht erfindungsgemäß | | | | | | | |

Die Herstellung der Plastisole erfolgte wie unter (1) beschrieben. Das so hergestellte Plastisol wurde 30 min nach der Homogenisierung zur Messung der Scherkurven verwendet. Die Messung der Scherkurven wurde mit Hilfe eines Oszillations- und Rotations-Rheometer MCR 302 von Anton Paar in einem Rotationsversuch durchgeführt.

Messsystem: Zylinder CC17
Ausgangstemperatur: 20 °C
Abschnitt 1:
    10 Minuten temperieren ohne Scherung
Abschnitt 2:
- Messpunkte: 50
- Messpunktdauer: 5 Sekunden
- Scherrate: 0,1 bis 3000 $s^{-1}$, log

Abschnitt 3:
- Messpunkte: 6
- Messpunktdauer: 5 Sekunden
- Scherrate: 3000 $s^{-1}$

Abschnitt 4:
- Messpunkte: 50
- Messpunktdauer: 5 Sek
- Scherrate: 3000 bis 0,1 $s^{-1}$, log

Die Messung erfolgte in vier Abschnitten. Der erste Abschnitt dient zum Temperieren der Probe bei konstanten 20 °C. Anschließend startet die Rotation mit dem oben genannten Profil (Abschnitte 2-4). Bei der Messung wird die Viskosität über die Schergeschwindigkeit aufgezeichnet.

Die Scherkurven sind in Fig. 3 dargestellt.

Man erkennt, daß der erfindungsgemäße Weichmacher (c) mit einem Zusatz von 2 % des Viskositätsreduzierers Viskobyk 5120 die Scherkurve des Vergleichsweichmachers Palatinol N erreicht und bei einem Zusatz von 5 % Viskobyk 5120 die Scherkurve des Vergleichsweichmachers Hexamoll DINCH erreicht.

(3) **Bestimmung** des **Schäumungsverhalten mit einem chemischen Schäumungsmittel**

Zur Bestimmung des Schäumungsverhaltens des erfindungsgemäßen Weichmachers (c) wurden Formulierungen entsprechend der nachfolgenden Tabelle 3 hergestellt:

Tabelle 3:

| | Plastisol # | |
|---|---|---|
| in phr | 16 | 17* |
| Vestolit E 7012 S | 100 | 100 |
| Baerostab KK 432 | 2 | 2 |
| Unicell D 200 A (als 50 % iges Masterbatch) | 8 | 8 |
| (c) | 56 | |
| Hexamoll DINCH | | 56 |
| *Nicht erfindungsgemäß | | |

Als Schäumungsmittel wurde ein Masterbatch aus Unicell D200A und dem entsprechenden Weichmacher erstellt, da es sich sonst nicht gleichmäßig in der Paste verteilt. Das Masterbatch besteht aus Unicell: Weichmacher im Verhältnis 50:50 und wurde mit dem Dissolver homogenisiert.

Die Herstellung der Plastisole erfolgte wie unter (1) beschrieben. Das so hergestellte Plastisol wurde 30 min nach der Homogenisierung zur Herstellung der Schäume verwendet. Dazu wurde mit Hilfe eines Rakels eine 0,5 mm dicke Schicht auf einem Releasepapier auf dem Schlitten des Mathis-Ofens aufgetragen. Der Ofen wurde zuvor auf die Schäumungstemperatur temperiert. Der Schlitten mit dem aufgerakelten Plastisol wurde dann für die angegebene Zeit im Ofen belassen, wieder ausgefahren und danach vom Releasepapier gelöst. Von jedem Schaum wurde an 2 ausgestanzten Mustern (5 x 5cm) die Dicke mit einem Mitutoyo-Taster bestimmt. Zur besseren Verteilung des Messstempel-Drucks wurde zwischen Stempel und Probe ein Mikroskop-Probenträger aus Glas gelegt. Die Dickenmessung wurde je Muster fünfmal durchgeführt und daraus der arithmetische Mittelwert gebildet. Danach wurde von beiden Mustern der arithmetische Mittelwert gebildet. Dieser Wert wurde in Relation gesetzt zur Höhe eines bei 160°C und 30 Sekunden im Mathis-Ofen gelierten Plastisols gebracht. Daraus ergibt sich die Expansionsrate in %.

Die Expansionsrate im Vergleich zum Nicht-erfindungsgemäßen Weichmacher Hexamoll DINCH ist in Fig. 4 dargestellt.

Man erkennt, dass sowohl der erfindungsgemäße Weichmacher (c) als auch der Vergleichsweichmacher Hexamoll DINCH bei 60 Sekunden Verweilzeit und 200°C die maximale Expansionsrate erreichen. Die Expansionsraten sind bei diesen Bedingungen auch nahezu gleich.

**(4) Plastisollagerstabilität**

Zur Bestimmung der Lagerstabilität der Plastisole mit den erfindungsgemäßen Weichmachern wurden Formulierungen entsprechend der nachfolgenden Tabelle 4 hergestellt.

Tabelle 4:

| | Plastisol # | |
|---|---|---|
| in phr | 18 | 19* |
| Vinnolit E 67 FF | 100 | 100 |
| Reagens 781/SLX | 2 | 2 |

(fortgesetzt)

| | Plastisol # | |
|---|---|---|
| (c) | 60 | |
| Hexamoll DINCH | | 60 |
| *Nicht erfindungsgemäß | | |

Die Herstellung der Plastisole erfolgte wie unter **(1)** beschrieben. Das so hergestellte Plastisol wurde zu den angegebenen Zeiten nach der Homogenisierung zur Messung der Plastisolviskositäten verwendet.
Die Messung der Scherkurven erfolgte mit Hilfe eines Oszillations- und Rotations-Rheometer MCR 302 von Anton Paar in einem Rotationsversuch.

Messsystem: Platte/Platte d=50 mm
Spaltbreite: 1 mm
Temperatur: 20 °C

Abschnitt 1:

- Anzahl Messpunkte: 10
- Messpunktdauer: 6 Sekunden
- Scherrate: 40 $s^{-1}$

Abschnitt 2:

- Anzahl Messpunkte: 10
- Messpunktdauer: 6 Sekunden
- Scherrate: 90 $s^{-1}$

Abschnitt 3:

- Anzahl Messpunkte: 10
- Messpunktdauer: 6 Sekunden
- Scherrate: 410 $s^{-1}$

Dazu wurden die Plastisole jeweils nach 0,5/2/6/24 Std vermessen und die Viskositätswerte bei 40/90/410 $s^{-1}$ notiert. Die Ergebnisse finden sich in Fig. 5.
Man erkennt, dass der erfindungsgemäße Weichmacher (c) in dem Plastisol nur eine sehr geringen Zunahme der Viskosität über den Zeitraum von 24 Stunden zeigt.

**(5) Messung des Gelierverhaltens an PVC-Plastisolen mit den erfindungsgemäßen Weichmachern (für Rotomolding)**

Zur Bestimmung des Gelierverhaltens im Rotomolding-Prozess der Plastisole mit den erfindungsgemäßen Weichmachern wurden Formulierungen entsprechend der nachfolgenden Tabelle 5 hergestellt.

Tabelle 5:

| | Plastisol # | | |
|---|---|---|---|
| in phr | 20 | 21* | 22* |
| Vestolit P1351 K | 100 | 100 | 100 |
| Reagens 781/SLX | 2 | 2 | 2 |
| (c) | 60 | | |

(fortgesetzt)

| | Plastisol # | | |
|---|---|---|---|
| Hexamoll DINCH | | 60 | |
| Palatinol N | | | 60 |
| *Nicht erfindungsgemäß | | | |

Das so hergestellte Plastisol wurde 30 min nach der Homogenisierung zur Messung der Gelierfähigkeit verwendet.

Die Viskositätsmessungen wurden mit einem beheizbaren Oszillations- und Rotations-Rheometer MCR 302 von Anton Paar in einem Oszillationsversuch durchgeführt.

- Messsystem: Platte/Platte d=50 mm
- Frequenz: 1 Hz
- Amplitude: 1 %
- Spaltbreite: 1 mm
- Ausgangstemperatur: 20 °C
- Temperaturprofil: 20 bis 200 °C
- Temperatursteigerung: 5 °C/min
- Anzahl Messpunkte: 201
- Messpunktdauer: 5,4 Sekunden

Die Messung erfolgte in zwei Schritten. Der erste Schritt dient zum Temperieren der Probe bei konstanten 20 °C. Bei 20 °C wird für 2 min mit einer Amplitude von 1 % und einer Frequenz von 1 Hz oszilliert. Anschließend startet das Temperaturprogramm. Bei der Messung wird die Viskosität über die Temperatur aufgezeichnet.

Die Gelierkurven sind in Fig 6 dargestellt.

Man erkennt, dass der Kurvenverlauf der Gelierkurve des erfindungsgemäßen Weichmachers (c) identisch ist mit dem Kurvenverlauf des Vergleichsweichmachers Palatinol N. Der erfindungsgemäße Weichmacher (c) ist somit gleich gut geeignet zur Verarbeitung von Plastisolen im Rotomolding-Verfahren wie Palatinol N.

Untersuchungen an Pressfolien

**(6) Herstellung der Weich-PVC-Pressfolien**

Nachfolgende Rezeptur wurde verwendet.

| **Additiv** | **phr** |
|---|---|
| PVC (Homopolymeres Suspensions-PVC, Markenname Inovyn® PVC 271 PC) | 100 |
| Weichmacher | 60 |
| Ba-Zn Stabilisator, Typ Reagens SLX/781 | 2 |

Die Komponenten wurden mit einem Handmixer bei Raumtemperatur vermischt. Die Mischung wurde anschließend auf einem ölbeheizten Labormischwalzwerk (Fa. Collin, Automatikwalzwerk Typ W 250 M x 400, Durchmesser: 252 mm, Breite: 450 mm) plastifiziert und zu einem Walzfell verarbeitet. Die Temperatur der beiden Walzen betrug wie in der Tabelle 2 angegeben; die Drehzahlen lagen bei 15 Umdrehungen/min. (vordere Walze) und 12 Umdrehungen/min. (hintere Walze); die Walzzeit betrug 5 Minuten.

Man erhielt ein Walzfell mit einer Dicke von ca. 0,53 mm. Das abgekühlte Walzfell wurde anschließend bei einer Temperatur von 10 °C höher als die Walztemperatur und einem Druck von 150 bar innerhalb 180 s auf einer Presse vom Typ "Laborplattenpresse 400 P" der Fa. Collin zu einer Weich-PVC-Folie mit einer Dicke von 0,50 mm verpresst.

Tabelle 6:

| | Walzfolie # | | | | | | | | | | | | | |
|---|---|---|---|---|---|---|---|---|---|---|---|---|---|---|
| | 1 | 2 | 3 | 4 | 5 | 6 | 7 | 8 | 9 | 10 | 11 | 12 | 13* | 14* |
| Inovyn® PVC 271 PC | 100 | 100 | 100 | 100 | 100 | 100 | 100 | 100 | 100 | 100 | 100 | 100 | 100 | 100 |
| Reagens SLX/781 | 2 | 2 | 2 | 2 | 2 | 2 | 2 | 2 | 2 | 2 | 2 | 2 | 2 | 2 |
| (a) | 50 | 60 | 70 | | | | | | | | | | | |
| (b) | | | | 60 | | | | | | | | | | |
| (c) | | | | | 50 | 60 | 70 | | 54 | 48 | 42 | | | |
| (d) | | | | | | | | 60 | | | | | | |
| (e) | | | | | | | | | 6 | 12 | 18 | | | |
| (f) | | | | | | | | | | | | 60 | | |
| (g) | | | | | | | | | | | | | 60 | |
| (h) | | | | | | | | | | | | | | 60 |
| Walztemperatur | 170 | 170 | 170 | 170 | 170 | 170 | 170 | 175 | 175 | 175 | 175 | 170 | 165 | 175 |
| *Nicht erfindungsgemäß | | | | | | | | | | | | | | |

**(7) Bestimmung der Folienflüchtigkeit von Folien mit den erfindungsgemäßen Weichmachern**

Die Folienflüchtigkeit ist ein Maß für die Flüchtigkeit eines Weichmachers im fertigen weichgemachten PVC-Artikel. Für die Prüfung der Folienflüchtigkeit wurden Folien hergestellt wie unter (6) beschrieben. An den so erhaltenen 0,5 mm dicken Folien wurde die Prüfung der Folienflüchtigkeit durchgeführt.

Prüfung der Folienflüchtigkeit über 24h bei 130°C:

Für die Bestimmung der Folienflüchtigkeit werden aus den nach (6) hergestellten Folien vier Einzelfolien (150 x 100 mm) ausgeschnitten, gelocht und gewogen. Die Folien werden an einen rotierenden Stern in einen auf 130°C eingestellten Heraeus Trockenschrank Typ 5042 E gehängt. In dem Schrank wird 18-mal pro Stunde die Luft gewechselt. Dies entspricht 800l/h Frischluft. Nach 24 h in dem Schrank werden die Folien entnommen und nach dem Abkühlen zurückgewogen. Der Gewichtsverlust in Prozent gibt die Folienflüchtigkeit der Weichmacher-Zusammensetzungen an.

Die Folienflüchtigkeit ist in Fig. 7 und 8 dargestellt

**(8) Bestimmung der Shore A-Härte von Folien mit den erfindungsgemäßen Weichmachern**

Die Shore A-Härte ist ein Maß für die Elastizität von weichgemachten PVC-Artikeln. Je niedriger die Shore-Härte, desto höher die Elastizität der PVC-Artikel. Für die Bestimmung der Shore A Härte wurden wie unter **(6)** beschrieben 49 x 49 mm große Folienstücke aus dem Walzfell ausgestanzt. Insgesamt wurden so ca. 27 Stück Folien ausgestanzt, im Pressrahmen übereinandergelegt, bei 10 °C höherer Temperatur als zur Herstellung des Walzfells verwendet wurde und zu einem 50 x 50 x 10 mm dicken Shore-Klotz gepresst.

Beschreibung der Shore-Härte Messung:

- Methode: DIN EN ISO 868, Okt. 2003
- Gerät: Fa. Hildebrand- Digital Durometer Modell DD-3
- Lagerzeit vor Messung: 7 d im Klimaraum bei 23 °C und 50 % rel. Feuchte
- Messzeit (Dauer der Nadel auf dem Probekörper bis zum Ablesen des Wertes) 15 s
- Es werden 10 Einzelwerte gemessen und daraus der arithmetische Mittelwert berechnet.

Die Shore A-Härte ist in Fig. 9 und 10 dargestellt

**(9) Bestimmung der Verträglichkeit (Permanenz) von Folien mit den erfindungsgemä-ßen Weichmachern**

Die Verträglichkeit (Permanenz) von Weichmachern in weichgemachten PVC-Artikeln charakterisiert, in welchem Ausmaß Weichmacher während des Gebrauchs der weichgemachten PVC-Artikel zum Ausschwitzen neigen und dadurch die Gebrauchseigenschaften des PVC-Artikels beeinträchtigen. Für die Prüfung der Verträglichkeit wurden Pressfolien hergestellt wie unter **(6)** beschrieben. An den so erhaltenen 0,5 mm dicken Folien wurde die Prüfung des Verträglichkeitsverhaltens durchgeführt.

Prüfmethode:

Zweck des Prüfverfahrens

Die Prüfung dient zur qualitativen und quantitativen Messung der Verträglichkeit von Weich-PVC-Rezepturen. Sie wird bei erhöhter Temperatur (70 °C) und Luftfeuchte (100 % rel. h) durchgeführt. Die erhaltenen Daten werden gegen die Lagerzeit ausgewertet.

Probekörper

Zur Standardprüfung werden je Rezeptur 10 Prüfkörper (Pressfolien) mit einer Größe von 75 x 110 x 0,5 mm verwendet. Die Folien werden an der Breitseite gelocht, beschriftet und gewogen. Die Beschriftung muss wischfest sein und kann z. B. mit dem Lötkolben erfolgen.

Prüfgeräte

Heraeus-Umlufttrockenschrank bei 70 °C, Analysenwaage, Temperaturmessgerät mit Fühler zur Messung der Innenraumtemperatur des Wärmeschrankes, Becken aus Glas, Metallgestelle aus nichtrostendem Material;

| | |
|---|---|
| Prüftemperatur: | 70 °C |
| Prüfmedium: | entstehender Wasserdampf bei 70 °C aus vollentsalztem Wasser |

Durchführung:

Die Temperatur im Innenraum des Wärmeschrankes wird auf 70 °C eingestellt. Die Prüffolien werden auf ein Drahtgestell aufgehängt und in eine Glaswanne, die etwa 5 cm hoch mit Wasser (VE-Wasser) gefüllt ist, hineingestellt. Es dürfen nur Folien gleicher Zusammensetzung in einem gekennzeichneten Becken gelagert werden, um Beeinflussung zu vermeiden und die Entnahme nach den jeweiligen Lagerzeiten zu erleichtern. Zu beachten ist, dass die Folien sich gegenseitig nicht berühren. Die Unterkanten der Folien dürfen nicht ins Wasser hängen. Der Wasserstand in den Glasbecken wird regelmäßig kontrolliert und evtl. fehlendes Wasser wird ersetzt.

Die Glaswanne wird mit einer PE-Folie wasserdampfdicht verschlossen, damit der später in der Glaswanne entstehende Wasserdampf nicht entweichen kann.

Lagerzeit

Im 1-, 3-, 7-, 14- und 28-Tage-Rhythmus werden jeweils 2 Folien (Doppelbestimmung) aus der Glaswanne entnommen und für 1 Std. frei hängend an der Luft klimatisiert. Danach wird die Folie optisch und haptisch beurteilt und anschließend mit Methanol gereinigt (mit Methanol angefeuchtetes Handtuch). Anschließend wird die Folie für 16 h bei 70 °C in einem Trockenschrank (natürliche Konvektion) frei hängend getrocknet. Nach Entnahme aus dem Trockenschrank wird die Folie für 1 Std. im Labor frei hängend konditioniert und gewogen (Trockenwert). Als Prüfergebnis angegeben wird jeweils der arithmetische Mittelwert der Gewichtsänderung aus ursprünglichem Foliengewicht und dem Trockenwert.

Die Verträglichkeit ist in Fig. 11 und 12 dargestellt

(10) Bestimmung der mechanischen Werte von Folien, enthaltend erfindungsgemäße Weichmacher

Die mechanischen Eigenschaften von weichgemachten PVC-Artikeln werden beispielsweise mit Hilfe der Parameter Bruchdehnung, Bruchspannung und des 100 % Moduls charakterisiert. Je höher der Wert der Bruchdehnung, desto besser die mechanischen Eigenschaften des weichgemachten PVC-Artikels. Niedrigere Werte für Bruchspannung

und 100 % Modul weisen auf eine effizientere Wirkung des Weichmachers hin. Für die Prüfung der mechanischen Werte wurden Pressfolien hergestellt, wie unter **(6)** beschrieben. An den so erhaltenen ca. 0,5 mm dicken Folien wurde die Prüfung der mechanischen Eigenschaften durchgeführt.

Die Parameter Bruchdehnung, Bruchspannung und 100 % Modul werden nach DIN EN ISO 527, Teil 1 u. 3, bestimmt. Im Einzelnen wird wie folgt vorgegangen.

- Maschine: Zwick Typ TMZ2.5/TH1S
- Methode: Prüfung nach DIN EN ISO 527 Teil 1 und Teil 3
- Probekörper: Folienstreifen Typ 2 nach DIN EN ISO 527 Teil 3, 150 mm lang, 15 mm breit, ausgestanzt
- Anzahl Probekörper: Pro Prüfung werden 10 Proben gerissen
- Klima: Normklima 23 °C (+- 1 °C), 50 % relative Luftfeuchtigkeit
- Lagerzeit der Probekörper vor der Messung: 7 Tage im Normklima
- Klemmen: Glatt-Konvex mit 6 bar Klemmendruck
- Einspannlänge: 100 mm
- Messlänge (= Einspannlänge): 100 mm
- Prüfgeschwindigkeit: 100 mm/min

Die Bruchdehnung, Bruchspannung und 100% Modul sind in Fig. 13 bis 16 dargestellt

**(11) Bestimmung der Kältebruchtemperatur von Folien, enthaltend erfindungsgemäße Weichmacher**

Die Kältebruchtemperatur von Weichmachern in Weich-PVC gibt die Temperatur an, bei der die Proben spröde werden und unter mechanischer Belastung brechen.

Für die Prüfung der Kältebruchtemperatur wurden Pressfolien hergestellt, wie unter (6) beschrieben. An den so erhaltenen 0,5 mm dicken Folien wurde die Prüfung der mechanischen Eigenschaften durchgeführt.

Prüfmethode:

Die Kältebruchtemperatur wird in Anlehnung an DIN 53372 bestimmt. Für die Messung der Kältebruchtemperatur werden zunächst 0,5 mm dicke Proben mit einer Länge von 60 mm und einer Breite von 15 mm zugeschnitten. Die Prüfung findet in einer Kälteprüfkammer statt, die in DIN 53372 beschrieben ist. Bei dieser Kälteprüfkammer handelt es sich um eine Eigenkonstruktion, welche sich in der Funktion an die DIN 53372 anlehnt. Entscheidender Unterschied dieser Prüfkonstruktion im Unterschied zur DIN 53372 besteht darin, dass die Hämmer nicht in freiem senkrechtem Fall auf die Prüfkörperschlaufen auftreffen. Bei dieser Konstruktion sind die Hämmer auf einer Welle befestigt und fallen nach dem Auslösen der Schlaggewichte im Kreisbogen aus gleicher Höhe (= Abstand zum Prüfkörper) auf die Prüfschlaufen herab.

Beim Aufspannen der Prüfschlaufen ist zu beachten, dass die Vorderkante der geknickten Schlaufe von der Mitte des darauf auftreffenden Hammers oder Gewichtes getroffen wird. Die Kältetruhe wird auf die Starttemperatur eingestellt und der Probenträger (Bombe) mit den Prüfkörpern eingesetzt.

Die Prüfung muss in einer trockenen Kältetruhe vorgenommen werden. Um beim Bombenwechsel eintretende Luftfeuchtigkeit zu binden, stehen Schalen mit Trockenmittel (z.B. Silicagel mit Feuchtigkeitsindikator) im Innenraum der Kältetruhe.

Zur Konditionierung der Prüfkörper werden diese pro Prüftemperatur 1 h temperiert.

Danach werden die Hämmer ausgelöst und fallen im Kreisbogen mit gleichem Gewicht auf die jeweiligen Prüfkörper.

Nach jedem Schlagvorgang ist die Eingriffsöffnung am Deckel kurz zu öffnen und die Unterseite der Hämmer mit der Hand auf anhaftendes PVC- Bruchmaterial oder Splitter zu prüfen. Diese sind zu entfernen.

Beurteilung der Prüflinge:

Es werden nur diejenigen Probeschlaufen als defekt gewertet die ganz zerbrochen sind. Um die Kältebruchtemperatur zu ermitteln, müssen mind. eine 6er-Reihe Prüflinge als positiv, sowie eine 6er-Reihe als negativ bewertet werden. Das Temperaturintervall der durchzuführenden Prüfungen beträgt jeweils 5 °C. Die Berechnung der Kältebruchtemperatur erfolgt gemäß DIN-Norm 53372.

Die Kältebruchtemperatur ist in Fig. 17 und 18 dargestellt

**(12) Bestimmung der Glasübergangstemperatur nach ISO 6721-7, Juni 1996**

Die Messung der Glasübergangstemperatur nach ISO 6721-7 erfolgt in einem Gerät Rheometrics RDA 2 der Firma TA Instruments. Ein streifenförmiger Prüfkörper (Rechteckform), hergestellt aus einer Preßfolie nach **(6),** wird vertikal an seinen Enden fest eingespannt und durch einen an der unteren Halterung angekoppelten Motor sinusförmig auf Torsion beansprucht. Dies geschieht mit einer Frequenz von 1 Hz und einem Strain von 0,2 %. Eine am oberen Streifenhalter angebrachte Kraftmessdose registriert das resultierende Drehmoment. Aus Drehmoment, Verdrehwinkel, der Phasenverschiebung zwischen beiden, und den geometrischen Dimensionen lassen sich Schubmodul (G') und Verlustfaktor (G") bestimmen.

Die Messungen werden in einem Temperaturbereich von -80oC bis +20oC unter schrittweißer Aufheizung der Probe durchgeführt. Als Resultat wird das komplexe Schubmodul in Abhängigkeit der Temperatur gemessen. Die Messungen erfolgen nach ISO 6721-7.

Glasübergänge lassen sich als Maxima in G" bestimmen. Darüber hinaus dienen diese Messungen der Charakterisierung des Dämpfungs- und Erweichungsverhaltens in Abhängigkeit der Temperatur

Test Bedingungen:

| | |
|---|---|
| Norm | ISO 6721-7 |
| Temperaturstufen | $\Delta T$ = 2 K |
| Temperatur | -80 °C bis 20 °C (unter Stickstoff) |
| Frequenz | 1 Hz |
| anfängl. Strain | 0,2 % |
| Geometrie | 40 mm x 10 mm x 0,5 mm (aus Folie gestanzt) |

Die Glasübergangstemperatur ist in Fig. 19 und 20 dargestellt

**(13) Wasserlagerung von Weich-PVC bei 50 °C**

Diese Prüfmethode wird in Anlehnung an DIN ISO 175 durchgeführt (Lagerung in Flüssigkeiten)

Zweck des Prüfverfahrens

Die Prüfung dient zur quantitativen Messung der Extraktion in VE - Wasser von Weich- PVC-Rezepturen. Sie wird bei erhöhter Temperatur (50 °C) durchgeführt. Die erhaltenen Daten werden gegen die Lagerzeit ausgewertet.

Probekörper

Zur Standardprüfung werden je Rezeptur 10 Prüfkörper (Pressfolienmit einer Größe von ca. 75 x 110 x 0,5 mm verwendet. Die Pressfolien wurden gemäß **(6)** hergestellt. Die Folien werden an der Breitseite gelocht, beschriftet und gewogen. Die Beschriftung muss wischfest sein und kann z.B. mit dem Lötkolben erfolgen.

Prüfgeräte

Wärmeschrank mit Umluft, Analysenwaage, Becken aus Glas, Metallgestelle aus nichtrostendem Material.

| | |
|---|---|
| Prüftemperatur: | 50 °C |
| Prüfmedium: | Auf eine Temperatur von 50 °C erhitztes VE - Wasser. |

Durchführung:

Die Temperatur im Innenraum des Wärmeschrankes wird auf 50 °C eingestellt. Die tarierten Prüfkörper werden auf ein Drahtgestell aufgehängt und in eine Glaswanne, die, bei voller Bestückung der 10 Prüfkörper mit den oben angegebenen Dimensionen mit mindestens 13,2 l Wasser (VE-Wasser) gefüllt ist, hineingestellt. Dabei ist zu beachten, dass das Volumen der Lagerflüssigkeit, mindestens 8 ml pro Quadratzentimeter Oberfläche der zu lagernden Prüfkörper

beträgt.

Es dürfen nur Prüfkörper mit gleicher Zusammensetzung in einem Becken gelagert werden, um die gegenseitige Beeinflussung durch unterschiedliche Formulierungsbestandteile zu vermeiden. Zu beachten ist, dass die Prüfkörper sich gegenseitig nicht berühren.

Die Unterkanten der Prüfkörper dürfen den Glasbeckenboden und Rand nicht berühren.

Die Glaswanne wird mit einer PE-Folie dicht verschlossen, damit das Wasser nicht verdampfen bzw. verdunsten kann.

Der Wasserstand in den Glasbecken wird regelmäßig kontrolliert und evtl. fehlendes Wasser wird ersetzt. Wöchentlich wird ein Wasserwechsel mit auf 50 °C vortemperiertem Wasser durchgeführt.

Lagerzeit

Im 1, 3, 7, 14, und 28-Tage-Rhythmus werden jeweils 2 Folien (Doppelbestimmung) aus der Glaswanne entnommen und in eine mit VE - Wasser gefüllte Metallschale hineingelegt. Die zuvor mit einem trockenen Handtuch abgetrockneten Prüfkörper werden für 16 h bei 80°C in einem Trockenschrank mit natürlicher Konvektion frei hängend getrocknet. Nach Entnahme aus dem Trockenschrank werden die Folien für mindestens 30 Minuten im Labor frei hängend konditioniert und anschließend gewogen.

Als Prüfergebnis angegeben wird jeweils der arithmetische Mittelwert der Gewichtsänderungen.

Die Wasserlagerung ist in Fig. 21 dargestellt.

**Patentansprüche**

1. Verbindung der allgemeinen Formel (I)

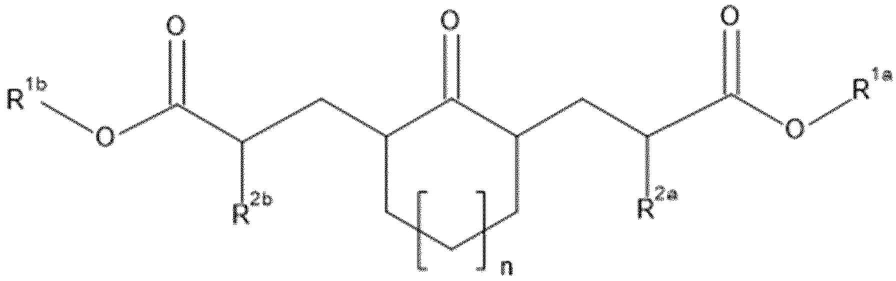

allgemeine Formel (I)

wobei

$R^{1a}$ und $R^{1b}$ unabhängig voneinander $C_8$- bis $C_{10}$-Alkyl sind,
$R^{2a}$ und $R^{2b}$ unabhängig voneinander H oder $CH_3$ sind,
und
n = 0, 1, 2 oder 7 ist.

2. Verbindung der allgemeinen Formel (I) nach Anspruch 1, wobei $R^{1a}$ und $R^{1b}$ gleich und n-Octyl, iso-Octyl, 2-Ethylhexyl, n-Nonyl, iso-Nonyl, n-Decyl, iso-Decyl, oder 2-Propylheptyl sind.

3. Verbindung der allgemeinen Formel (I) nach Anspruch 2 wobei $R^{1a}$ und $R^{1b}$ gleich und 2-Ethylhexyl, iso-Nonyl oder 2-Propylheptyl sind.

4. Verbindung der allgemeinen Formel (I) nach einem der vorangegangenen Ansprüche, wobei $R^{2a}$ und $R^{2b}$ H sind.

5. Verbindung der allgemeinen Formel (I) nach einem der vorangegangenen Ansprüche, wobei n = 0 oder 1 ist.

6. Verfahren zur Herstellung einer Verbindung der allgemeinen Formel (I), wie in einem der Ansprüche 1 bis 5 definiert, **dadurch gekennzeichnet, dass**

a) eine Verbindung der allgemeinen Formel (II)

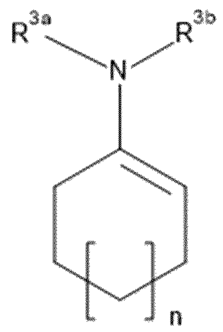

allgemeine Formel (II)

wobei

$R^{3a}$ und $R^{3b}$ unabhängig voneinander $C_1$- bis $C_{10}$-Alkyl sind oder zusammen einen Zyklus mit 4 bis 11 Kohlenstoffatomen bilden,
und
n = 0, 1, 2 oder 7 ist,
mit einer Verbindung der allgemeinen Formel (IIIa), (IIIb) oder einer Mischung aus Verbindungen der allgemeinen Formel (IIIa) und (IIIb)

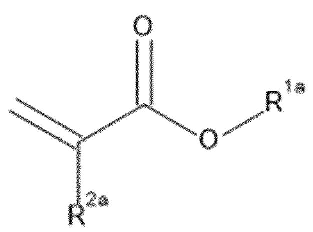

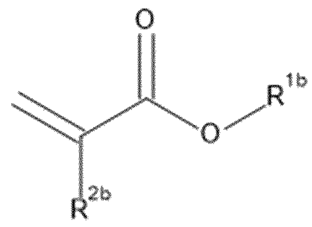

(IIIa) (IIIb)

wobei
$R^{1a}$ und $R^{1b}$ unabhängig voneinander $C_8$- bis $C_{10}$-Alkyl sind,
und
$R^{2a}$ und $R^{2a}$ unabhängig voneinander H oder $CH_3$ sind,
umgesetzt wird,

b) das Reaktionsprodukt aus Schritt a) zu einer Verbindung der allgemeinen Formel (I) hydrolysiert wird.

**7.** Verfahren nach Anspruch 6, wobei die Hydrolyse in Schritt b) durch die Zugabe wässriger Bremsted-Säure erfolgt.

**8.** Verfahren nach einem der Ansprüche 6 oder 7, wobei das Molverhältnis zwischen Verbindungen der allgemeinen Formel (II) und der Gesamtmenge der eingesetzten Verbindungen der allgemeinen Formel (III) 1 : 1 bis 1: 8 beträgt.

**9.** Verfahren nach einem der Ansprüche 6 bis 8, wobei $R^{3a}$ und $R^{3b}$ unabhängig voneinander $C_1$- bis $C_5$-Alkyl sind oder zusammen einen Zyklus mit 4 bis 6 Kohlenstoffatomen bilden, wobei der Zyklus ein oder kein Heteroatom enthält und alle Kohlenstoffatome im Zyklus als Alkanidylkohlenstoffatome vorliegen und keine von H unterschiedlichen Substituenten tragen.

**10.** Verfahren nach einem der Ansprüche 6 bis 8, wobei Schritt a) die Umsetzung einer Verbindung der allgemeinen Formel (IV)

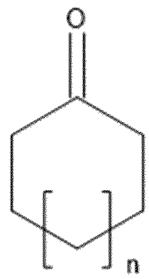

allgemeine Formel (IV)

wobei n = 0,1, 2 oder 7 ist,
mit einer Aminverbindung der allgemeinen Formel H-$NR^{3a}R^{3b}$ unter Bildung einer Verbindung der allgemeinen Formel (II) vorangeht.

**11.** Verfahren nach Anspruch 10, wobei es sich bei der Aminverbindung um Pyrrolidin, Piperidin, Hexamethylenimin, Morpholin oder um eine beliebige Mischung aus zwei oder mehr der zuvor genannten Aminverbindungen handelt.

**12.** Verfahren zur Herstellung einer Verbindung der allgemeinen Formel (I), wie in einem der Ansprüche 1 bis 5 definiert, **dadurch gekennzeichnet, dass** eine Verbindung der allgemeinen Formel (V) oder ein Säureanhydrid einer Verbindung der allgemeinen Formel (V) oder ein Säurehalogenid einer Verbindung der allgemeinen Formel (V)

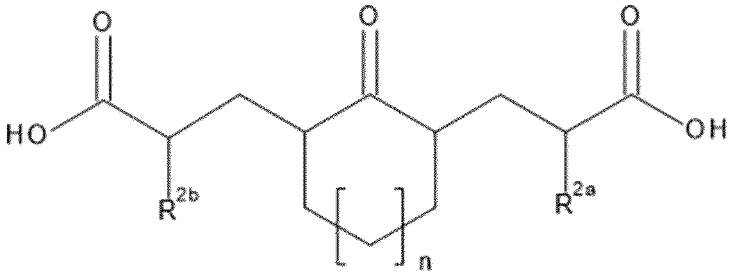

allgemeine Formel (V)

wobei

$R^{2a}$ und $R^{2b}$ unabhängig voneinander H oder $CH_3$ sind,
und
n = 0, 1, 2 oder 7 ist,
mit Alkoholen der allgemeinen Formel $R^{1a}$-OH und $R^{1b}$-OH in Gegenwart eines Veresterungskatalysators umgesetzt wird, wobei $R^{1a}$ und $R^{1b}$ unabhängig voneinander $C_8$- bis $C_{10}$-Alkyl sind.

**13.** Verfahren nach Anspruch 12 wobei es sich bei dem Veresterungskatalysator um Isopropyl-n-butyltitanat, Tetraisopropyltitanat, Tetra-butyltitanat oder eine Mischung aus zwei oder mehr der zuvor genannten Katalysatoren handelt.

**14.** Verfahren nach Anspruch 12 oder 13 wobei das Molverhältnis zwischen Verbindungen der allgemeinen Formel (V) und der Gesamtmenge der eingesetzten Alkohole der allgemeinen Formel $R^{1a}$-OH und $R^{1b}$-OH 1 : 0,5 bis 1 : 400 beträgt.

**15.** Verfahren zur Herstellung einer Verbindung der allgemeinen Formel (I), wie in einem der Ansprüche 1 bis 6 definiert, **dadurch gekennzeichnet, dass** eine Verbindung der allgemeinen Formel (VI)

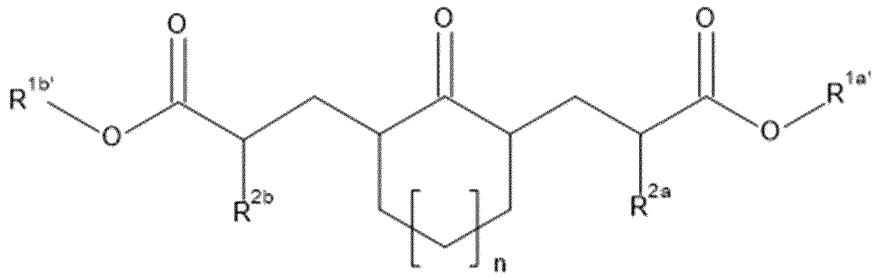

allgemeine Formel (VI)

wobei

$R^{1a'}$ und $R^{1b'}$ unabhängig voneinander Methyl, Ethyl, n-Propyl, iso-Propyl, n-Butyl, iso-Butyl, oder tert-Butyl sind,
$R^{2a}$ und $R^{2b}$ unabhängig voneinander H oder $CH_3$ sind
und
n = 0, 1, 2 oder 7 ist,
mit Alkoholen der allgemeinen Formel $R^{1a}$-OH und $R^{1b}$-OH in Gegenwart eines Umesterungskatalysators umgesetzt wird, wobei $R^{1a}$ und $R^{1b}$ unabhängig voneinander $C_8$- bis $C_{10}$-Alkyl sind.

**16.** Verfahren nach Anspruch 15 wobei es sich bei dem Umesterungskatalysator um Isopropyl-n-butyltitanat, Tetraisopropyltitanat, Tetra-butyltitanat oder eine Mischung aus zwei oder mehr der zuvor genannten Katalysatoren handelt.

**17.** Verfahren nach Anspruch 15 oder 16 wobei das Molverhältnis zwischen Verbindungen der allgemeinen Formel (V) und der Gesamtmenge der eingesetzten Alkohole der allgemeinen Formel $R^{1a}$-OH und $R^{1b}$-OH 1 : 0,5 bis 1 : 400 beträgt.

**18.** Verwendung einer oder mehrerer Verbindung(en) der allgemeinen Formel (I), wie in einem der Ansprüche 1 bis 6 definiert, als Weichmacher für ein Polymer oder eine Mischung von verschiedenen Polymeren.

**19.** Verwendung einer oder mehrerer Verbindung(en) der allgemeinen Formel (I), wie in einem der Ansprüche 1 bis 6 definiert, als Weichmacher in einem Plastisol.

**20.** Weichmacher-Zusammensetzung die eine oder mehrere Verbindungen der allgemeinen Formel (I), wie in einem der Ansprüche 1 bis 6 definiert, und einen oder mehrere Weichmacher, die von den Verbindungen der allgemeinen Formel (I) unterschiedlich sind enthält.

**21.** Weichmacher-Zusammensetzung nach Anspruch 20, wobei es sich bei Weichmacher, die von den Verbindungen der allgemeinen Formel (I) unterschiedlich sind um Cyclohexan-1,2-dicarbonsäuredialkylester, Cyclohexan-1,3-dicarbonsäuredialkylester, Cyclohexan-1,4-dicarbonsäuredialkylester, Terephthalsäuredialkylester, Phthalsäuredialkylester, Äpfelsäuredialkylester, Acetyl-Äpfelsäuredialkylester, Trimellitsäuretrialkylester, Benzoesäurealkylester, Dibenzoesäureester, tetra-Ester von Pentaerythrit wie Pentaerythritol tetra(valerat), Monocarbonsäurealkylester wie gesättigte oder ungesättigte Monocarbonsäurealkylester, Dicarbonsäuredialkylester wie gesättigte oder ungesättigte Dicarbonsäuredialkylester, aromatische Sulfonsäureester, Alkylsulfonsäureester, Glycerinester, Isosorbidester, Phosphorsäureester, Citronensäuretriester, Alkylpyrrolidonderivate, 2,5-Furandicarbonsäuredialkylester, 2,5-Tetrahydrofurandicarbonsäuredialkylester, Polyester aus aromatischen und/oder aliphatischen Polycarbonsäuren mit mindestens zweiwertigen Alkoholen, epoxidiertes Pflanzenöl oder epoxidierte Fettsäuremonoalkylester handelt.

**22.** Weichmacher-Zusammensetzung nach Anspruch 20 oder 21 wobei eine oder mehrere Verbindungen der allgemeinen Formel (I) in einer Gesamtmenge von 5 bis 95 Gewichtsprozent und ein oder mehrere Weichmacher, die von den Verbindungen der allgemeinen Formel (I) unterschiedlich sind mit einer Gesamtmenge von 95 bis 5 Gewichtsprozent enthalten sind, wobei sich die Gewichtsprozentangaben auf das Gesamtgewicht aller in der Weichmacher-Zusammensetzung enthaltenen Weichmacher beziehen und sich zu 100 Prozent addieren.

**23.** Verwendung einer Weichmacher-Zusammensetzung nach einem der Ansprüche 20 bis 22 als Weichmacher für ein

Polymer oder eine Mischung von Polymeren.

24. Verwendung einer Weichmacher-Zusammensetzung nach einem der Ansprüche 20 bis 22 als Weichmacher in einem Plastisol.

25. Formmasse enthaltend eine oder mehrere Verbindung(en) der allgemeinen Formel (I), wie in einem der Ansprüche 1 bis 6 definiert und ein oder mehrere Polymer(e).

26. Formmasse nach Anspruch 25 wobei es sich bei dem Polymer um Polyvinylchlorid handelt

27. Formmasse nach Anspruch 25 wobei die Gesamtmenge an einer oder mehrerer Verbindungen der allgemeinen Formel (I) in der Formmasse 0,5 bis 300 phr beträgt.

28. Plastisol enthaltend eine oder mehrere Verbindung(en) der allgemeinen Formel (I), wie in einem der Ansprüche 1 bis 6 definiert und einen oder mehrere Thermoplaste.

29. Plastisol nach Anspruch 28 wobei es sich bei dem Thermoplasten um Polyvinylchlorid handelt.

30. Plastisol nach einem der Ansprüche 28 oder 29 wobei eine oder mehrere Verbindungen der allgemeinen Formel (I) in einer Gesamtmenge von 30 bis 400 phr im Plastisol enthalten sind.

31. Formkörper oder Folien enthaltend eine oder mehrere Verbindungen der allgemeinen Formel (I) wie in einem der Ansprüche 1 bis 6 definiert.

## Claims

1. A compound of the general formula (I)

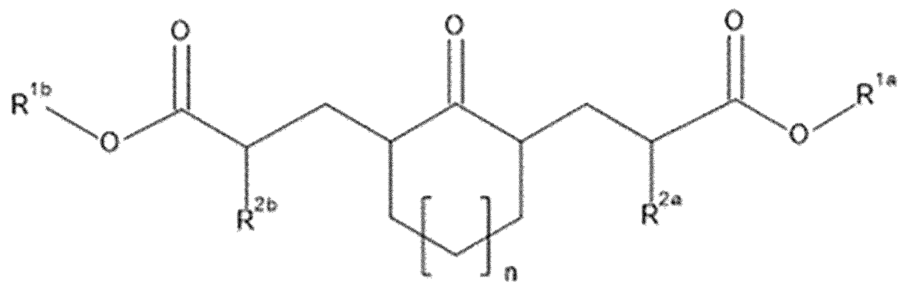

general formula (I)

where

$R^{1a}$ and $R^{1b}$ are each independently $C_8$ to $C_{10}$ alkyl,
$R^{2a}$ and $R^{2b}$ are each independently H or $CH_3$,
and
n = 0, 1, 2 or 7.

2. The compound of the general formula (I) according to claim 1, where $R^{1a}$ and $R^{1b}$ are identical and are n-octyl, isooctyl, 2-ethylhexyl, n-nonyl, isononyl, n-decyl, isodecyl or 2-propylheptyl.

3. The compound of the general formula (I) according to claim 2, where $R^{1a}$ and $R^{1b}$ are identical and are 2-ethylhexyl, isononyl or 2-propylheptyl.

4. The compound of the general formula (I) according to any of the preceding claims, where $R^{2a}$ and $R^{2b}$ are H.

5. The compound of the general formula (I) according to any of the preceding claims, where n = 0 or 1.

**6.** A method for preparing a compound of the general formula (I) as defined in any of claims 1 to 5, **characterized in that**

a) a compound of the general formula (II)

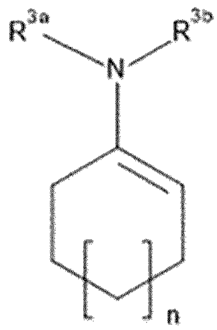

general formula (II)

where

$R^{3a}$ and $R^{3b}$ are each independently $C_1$ to $C_{10}$ alkyl or together form a ring having 4 to 11 carbon atoms and n = 0, 1, 2 or 7,
is reacted with a compound of the general formula (IIIa) or (IIIb) or with a mixture of compounds of the general formula (IIIa) and (IIIb)

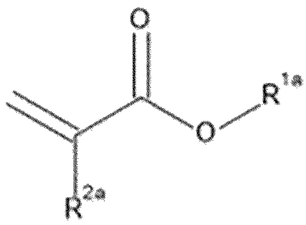

(IIIa)

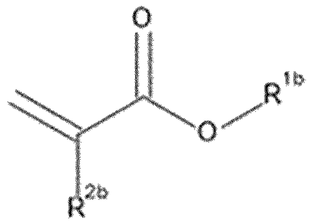

(IIIb)

where
$R^{1a}$ and $R^{1b}$ are each independently $C_8$ to $C_{10}$ alkyl and
$R^{2a}$ and $R^{2a}$ are each independently H or $CH_3$, and

b) the reaction product from step a) is hydrolyzed to a compound of the general formula (I).

**7.** The method according to claim 6, wherein the hydrolysis in step b) is effected by the addition of an aqueous Bronsted acid.

**8.** The method according to either of claims 6 and 7, wherein the molar ratio between compounds of the general formula (II) and the total amount used of compounds of the general formula (III) is 1:1 to 1:8.

**9.** The method according to any of claims 6 to 8, where $R^{3a}$ and $R^{3b}$ are each independently $C_1$ to $C_5$ alkyl or together form a ring having 4 to 6 carbon atoms, wherein the ring contains one heteroatom or none, all the carbon atoms in the ring are present in the form of alkanediyl carbon atoms, and none bear substituents different from H.

**10.** The method according to any of claims 6 to 8, wherein step a) is preceded by the reaction of a compound of the general formula (IV)

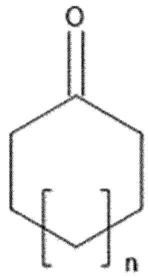

general formula (IV)

where n = 0, 1, 2 or 7,
with an amine compound of the general formula H-$NR^{3a}R^{3b}$ to form a compound of the general formula (II) .

**11.** The method according to claim 10, wherein the amine compound is pyrrolidine, piperidine, hexamethyleneimine, morpholine or any desired mixture of two or more of the aforementioned amine compounds.

**12.** A method for preparing a compound of the general formula (I) as defined in any of claims 1 to 5, **characterized in that** a compound of the general formula (V) or an acid anhydride of a compound of the general formula (V) or an acid halide of a compound of the general formula (V)

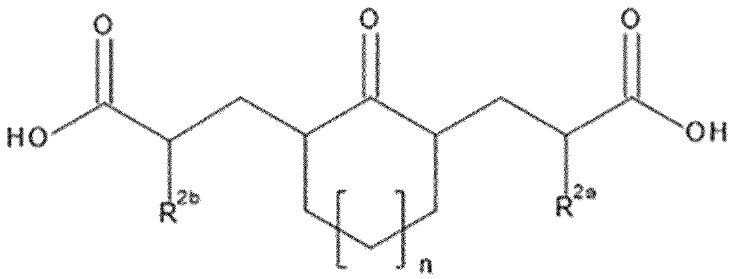

general formula (V)

where

$R^{2a}$ and $R^{2b}$ are each independently H or $CH_3$
and
n = 0, 1, 2 or 7,
is reacted with alcohols of the general formula $R^{1a}$-OH and $R^{1b}$-OH in the presence of an esterification catalyst, where $R^{1a}$ and $R^{1b}$ are each independently $C_8$ to $C_{10}$ alkyl.

**13.** The method according to claim 12, wherein the esterification catalyst is isopropyl n-butyl titanate, tetraisopropyl titanate, tetrabutyl titanate or a mixture of two or more of the aforementioned catalysts.

**14.** The method according to claim 12 or 13, wherein the molar ratio between compounds of the general formula (V) and the total amount used of alcohols of the general formula $R^{1a}$-OH and $R^{1b}$-OH is 1:0.5 to 1:400.

**15.** A method for preparing a compound of the general formula (I) as defined in any of claims 1 to 6, **characterized in that** a compound of the general formula (VI)

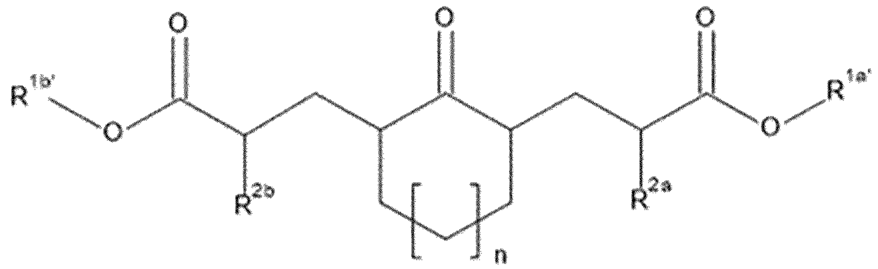

general formula (VI)

where

$R^{1a'}$ and $R^{1b'}$ are each independently methyl, ethyl, n-propyl, isopropyl, n-butyl, isobutyl or tert-butyl,
$R^{2a}$ and $R^{2b}$ are each independently H or $CH_3$,
and
n = 0, 1, 2 or 7,
is reacted with alcohols of the general formula $R^{1a}$-OH and $R^{1b}$-OH in the presence of a transesterification catalyst, where $R^{1a}$ and $R^{1b}$ are each independently $C_8$ to $C_{10}$ alkyl.

**16.** The method according to claim 15, wherein the transesterification catalyst is isopropyl n-butyl titanate, tetraisopropyl titanate, tetrabutyl titanate or a mixture of two or more of the aforementioned catalysts.

**17.** The method according to claim 15 or 16, wherein the molar ratio between compounds of the general formula (V) and the total amount used of alcohols of the general formula $R^{1a}$-OH and $R^{1b}$-OH is 1:0.5 to 1:400.

**18.** The use of one or more compounds of the general formula (I) as defined in any of claims 1 to 6 as plasticizer for a polymer or mixture of different polymers.

**19.** The use of one or more compounds of the general formula (I) as defined in any of claims 1 to 6 as plasticizer in a plastisol.

**20.** A plasticizer composition comprising one or more compounds of the general formula (I) as defined in any of claims 1 to 6 and one or more plasticizers different from the compounds of the general formula (I).

**21.** The plasticizer composition according to claim 20, wherein the plasticizers different from the compounds of the general formula (I) are dialkyl cyclohexane-1,2-dicarboxylates, dialkyl cyclohexane-1,3-dicarboxylates, dialkyl cyclohexane-1,4-dicarboxylates, dialkyl terephthalates, dialkyl phthalates, dialkyl malates, dialkyl acetylmalates, trialkyl trimellitates, alkyl benzoates, esters of dibenzoic acids, tetraesters of pentaerythritol, such as pentaerythritol tetravalerate, alkyl esters of monocarboxylic acids, such as alkyl esters of saturated or unsaturated monocarboxylic acids, dialkyl esters of dicarboxylic acids, such as dialkyl esters of saturated or unsaturated dicarboxylic acids, esters of aromatic sulfonic acids, esters of alkyl sulfonic acids, glycerol esters, isosorbide esters, esters of phosphoric acid, esters of citric acid, alkyl pyrrolidone derivatives, dialkyl furan-2,5-dicarboxylates, dialkyl tetrahydrofuran-2,5-dicarboxylates, polyesters of aromatic and/or aliphatic polycarboxylic acids with at least dihydric alcohols, epoxidized vegetable oil or epoxidized monoalkyl esters of fatty acids.

**22.** The plasticizer composition according to claim 20 or 21, wherein one or more compounds of the general formula (I) are present in a total amount of 5% to 95% by weight and one or more plasticizers different from the compounds of the general formula (I) are present in a total amount of 95% to 5% by weight, where the percentages by weight are based on the total weight of all plasticizers present in the plasticizer composition and add up to 100 percent.

**23.** The use of a plasticizer composition according to any of claims 20 to 22 as plasticizer for a polymer or mixture of polymers.

**24.** The use of a plasticizer composition according to any of claims 20 to 22 as plasticizer in a plastisol.

**25.** A molding compound comprising one or more compounds of the general formula (I) as defined in any of claims 1 to 6 and one or more polymers.

**26.** The molding compound according to claim 25, wherein the polymer is polyvinyl chloride.

**27.** The molding compound according to claim 25, wherein the total amount of one or more compounds of the general formula (I) in the molding compound is 0.5 to 300 phr.

**28.** A plastisol comprising one or more compounds of the general formula (I) as defined in any of claims 1 to 6 and one or more thermoplastics.

**29.** The plastisol according to claim 28, wherein the thermoplastic is polyvinyl chloride.

**30.** The plastisol according to either of claims 28 and 29, wherein one or more compounds of the general formula (I) is present in the plastisol in a total amount of 30 to 400 phr.

**31.** A molded article or film comprising one or more compounds of the general formula (I) as defined in any of claims 1 to 6.

## Revendications

**1.** Composé de Formule générale (I)

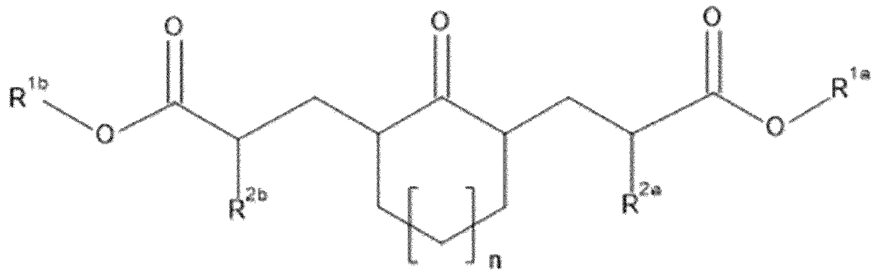

Formule générale (I)

dans laquelle

$R^{1a}$ et $R^{1b}$ représentent indépendamment l'un de l'autre un alkyle en $C_8$ à $C_{10}$,
$R^{2a}$ et $R^{2b}$ représentent indépendamment l'un de l'autre H ou $CH_3$,
et
n = 0, 1, 2 ou 7.

**2.** Composé de Formule générale (I) selon la revendication 1, dans laquelle $R^{1a}$ et $R^{1b}$ sont identiques et représentent un radical n-octyle, iso-octyle, 2-éthylhexyle, n-nonyle, iso-nonyle, n-décyle, iso-décyle ou 2-propylheptyle.

**3.** Composé de Formule générale (I) selon la revendication 2, dans laquelle $R^{1a}$ et $R^{1b}$ sont identiques et représentent un radical 2-éthylhexyle, iso-nonyle ou 2-propylheptyle.

**4.** Composé de Formule générale (I) selon l'une des revendications précédentes, dans laquelle $R^{2a}$ et $R^{2b}$ représentent H.

**5.** Composé de Formule générale (I) selon l'une des revendications précédentes, dans laquelle n = 0 ou 1.

**6.** Procédé de préparation d'un composé de Formule générale (I) tel que défini dans l'une des revendications 1 à 5, **caractérisé en ce qu'**on fait réagir

a) un composé de Formule générale (II)

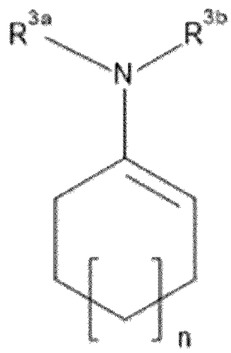

Formule générale (II)

dans laquelle

$R^{3a}$ et $R^{3b}$ représentent indépendamment l'un de l'autre un alkyle en $C_1$ à $C_{10}$ ou forment ensemble un cycle ayant 4 à 11 atomes de carbone,
et
n = 0, 1, 2 ou 7,
avec un composé de Formule générale (IIIa), (IIIb), ou avec un mélange de composés de Formules générales (IIIa) et (IIIb)

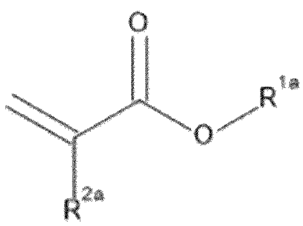

(IIIa)

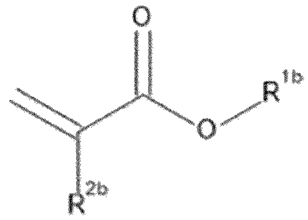

(IIIb)

dans lesquelles
$R^{1a}$ et $R^{1b}$ représentent indépendamment l'un de l'autre un alkyle en $C_8$ à $C_{10}$,
et
$R^{2a}$ et $R^{2a}$ représentent indépendamment l'un de l'autre H ou $CH_3$,

b) on hydrolyse le produit de réaction de l'étape a) pour obtenir un composé de Formule générale (I).

**7.** Procédé selon la revendication 6, dans lequel l'hydrolyse de l'étape b) est mise en œuvre par addition d'un acide de Bronsted aqueux.

**8.** Procédé selon l'une des revendications 6 ou 7, dans lequel le rapport en moles des composés de Formule générale (II) à la quantité totale des composés utilisés de Formule générale (III) est de 1:1 à 1:8.

**9.** Procédé selon l'une des revendications 6 à 8, dans lequel $R^{3a}$ et $R^{3b}$ représentent indépendamment l'un de l'autre un alkyle en $C_1$ à $C_5$ ou forment ensemble un cycle ayant 4 à 6 atomes de carbone, le cycle contenant un hétéroatome ou ne contenant aucun hétéroatome, et tous les atomes de carbone du cycle étant présents sous forme d'atomes de carbone d'alcanidyle, et ne portant aucun substituant autre que H.

**10.** Procédé selon l'une des revendications 6 à 8, dans lequel l'étape a) précède la réaction d'un composé de Formule générale (IV)

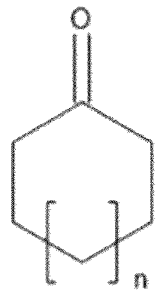

Formule générale (IV)

dans laquelle n = 0, 1, 2 ou 7,
avec un composé amine de formule générale H-$NR^{3a}R^{3b}$ avec formation d'un composé de Formule générale (II).

**11.** Procédé selon la revendication 10, dans lequel le composé amine est la pyrrolidine, la pipéridine, l'hexaméthylène-imine, la morpholine ou un mélange quelconque de deux ou plus des composés amines, mentionnés ci-dessus.

**12.** Procédé de préparation d'un composé de Formule générale (I) tel que défini dans l'une des revendications 1 à 5, **caractérisé en ce qu'**on fait réagir un composé de Formule générale (V) ou un anhydride d'acide d'un composé de Formule générale (V) ou d'un halogénure d'acyle d'un composé de Formule générale (V)

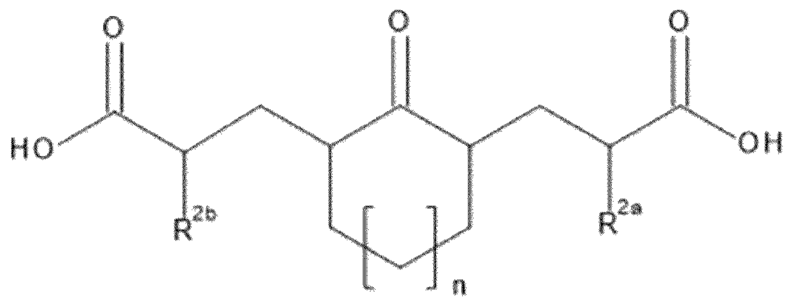

Formule générale (V)

dans laquelle

$R^{2a}$ et $R^{2b}$ représentent indépendamment l'un de l'autre H ou $CH_3$,
et
n = 0, 1, 2 ou 7,
avec des alcools de formules générales $R^{1a}$-OH et $R^{1b}$-OH en présence d'un catalyseur d'estérification, $R^{1a}$ et $R^{1b}$ représentant indépendamment l'un de l'autre un alkyle en $C_8$ à $C_{10}$.

**13.** Procédé selon la revendication 12, le catalyseur d'estérification étant le titanate d'isopropyle et de n-butyle, le titanate de tétra-isopropyle, le titanate de tétra-butyle ou un mélange de deux ou plus des catalyseurs mentionnés ci-dessus.

**14.** Procédé selon la revendication 12 ou 13, dans lequel le rapport en moles des composés de Formule générale (V) à la quantité totale des alcools utilisés de formule générale $R^{1a}$-OH et $R^{1b}$-OH est de 1:0,5 à 1:400.

**15.** Procédé de préparation d'un composé de Formule générale (I) tel que défini dans l'une des revendications 1 à 6, **caractérisé en ce qu'**on fait réagir un composé de Formule générale (VI)

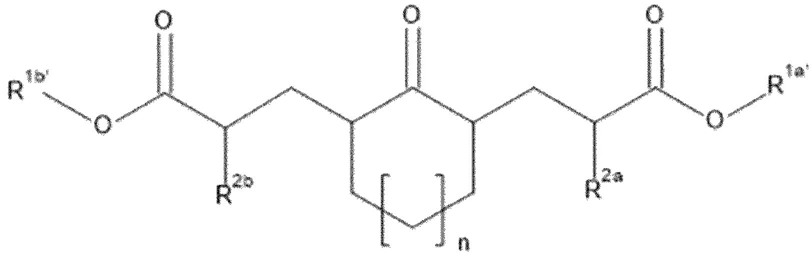

Formule (VI)

dans laquelle

$R^{1a'}$ et $R^{1b'}$ représentent indépendamment l'un de l'autre un radical méthyle, éthyle, n-propyle, isopropyle, n-butyle, iso-butyle ou tert-butyle,
$R^{2a}$ et $R^{2b}$ représentent indépendamment l'un de l'autre H ou $CH_3$,

et

n = 0, 1, 2 ou 7,
avec des alcools de formules générales $R^{1a}$-OH et $R^{1b}$-OH en présence d'un catalyseur de transestérification, $R^{1a}$ et $R^{1b}$ représentant indépendamment l'un de l'autre un alkyle en $C_8$ à $C_{10}$.

**16.** Procédé selon la revendication 15, le catalyseur de transestérification étant le titanate d'isopropyle et de n-butyle, le titanate de tétra-isopropyle, le titanate de tétra-butyle ou un mélange de deux ou plus des catalyseurs mentionnés ci-dessus.

**17.** Procédé selon la revendication 15 ou 16, dans lequel le rapport en moles des composés de Formule générale (V) à la quantité totale des alcools utilisés de formules générales $R^{1a}$-OH et $R^{1b}$-OH est de 1:0,5 à 1:400.

**18.** Utilisation d'un ou plusieurs composés de Formule générale (I) tels que définis dans l'une des revendications 1 à 6, en tant que plastifiant pour un polymère ou un mélange de différents polymères.

**19.** Utilisation d'un ou plusieurs composés de Formule générale (I) tels que définis dans l'une des revendications 1 à 6, en tant que plastifiant dans un plastisol.

**20.** Composition de plastifiants, qui contient un ou plusieurs composés de Formule générale (I) tels que définis dans l'une des revendications 1 à 6, et un ou plusieurs plastifiants, qui sont différents des composés de Formule générale (I).

**21.** Composition de plastifiants selon la revendication 20, les plastifiants qui sont différents des composés de Formule générale (I) étant les esters dialkyliques de l'acide cyclohexane-1,2-dicarboxylique, les esters dialkyliques de l'acide cyclohexane-1,3-dicarboxylique, les esters dialkyliques de l'acide cyclohexane-1,4-dicarboxylique, les esters dialkyliques de l'acide téréphtalique, les esters dialkyliques de l'acide phtalique, les esters dialkyliques de l'acide malique, les esters dialkyliques de l'acide acétyl-malique, les esters trialkyliques de l'acide trimellitique, les esters alkyliques de l'acide benzoïque, les esters de l'acide dibenzoïque, les tétra-esters du pentaérythritol tels que le tétra(valérate) de pentaérythritol, les esters alkyliques d'acides monocarboxyliques tels que les esters alkyliques d'acides monocarboxyliques saturés ou insaturés, les esters dialkyliques d'acides dicarboxyliques tels que les esters dialkyliques d'acides dicarboxyliques saturés ou insaturés, les esters d'acides sulfoniques aromatiques, les esters d'acides alkylsulfoniques, les esters du glycérol, les esters d'isosorbide, les esters de l'acide phosphorique, les esters de l'acide citrique, les dérivés des alkylpyrrolidones, les esters dialkyliques de l'acide 2,5-furanedicarboxylique, les esters dialkyliques de l'acide 2,5-tétrahydrofuranedicarboxylique, les polyesters d'acides polycarboxyliques aromatiques et/ou aliphatiques avec des alcools au moins divalents, une huile végétale époxydée ou les esters monoalkyliques d'acides gras époxydés.

**22.** Composition de plastifiants selon la revendication 20 ou 21, dans laquelle un ou plusieurs composés de Formule générale (I) sont présents en une quantité totale de 5 à 95 pour cent en poids et un ou plusieurs plastifiants qui sont différents des composés de Formule générale (I) sont présents en une quantité totale de 95 à 5 pour cent en poids,

les indications de pourcentage en poids étant rapportées au poids total de la totalité des plastifiants contenus dans la composition de plastifiants, et leur somme faisant 100 pour cent.

**23.** Utilisation d'une composition de plastifiants selon l'une des revendications 20 à 22 en tant que plastifiant pour un polymère ou un mélange de polymères.

**24.** Utilisation d'une composition de plastifiants selon l'une des revendications 20 à 22 en tant que plastifiant dans un plastisol.

**25.** Mélange à mouler contenant un ou plusieurs composés de Formule générale (I) tels que définis dans l'une des revendications 1 à 6 et un ou plusieurs polymères.

**26.** Mélange à mouler selon la revendication 25, dans lequel le polymère est le poly(chlorure de vinyle).

**27.** Mélange à mouler selon la revendication 25, dans lequel la quantité totale d'un ou plusieurs composés de Formule générale (I) dans le mélange à mouler est de 0,5 à 300 phr.

**28.** Plastisol contenant un ou plusieurs composés de Formule générale (I) tels que définis dans l'une des revendications 1 à 6 et un ou plusieurs composés thermoplastiques.

**29.** Plastisol selon la revendication 28, le thermoplastique étant le poly(chlorure de vinyle).

**30.** Plastisol selon l'une des revendications 28 ou 29, dans lequel un ou plusieurs composés de Formule générale (I) sont présents en une quantité totale de 30 à 400 phr dans le plastisol.

**31.** Objet moulé ou feuilles contenant un ou plusieurs composés de Formule générale (I) tels que définis dans l'une des revendications 1 à 6.

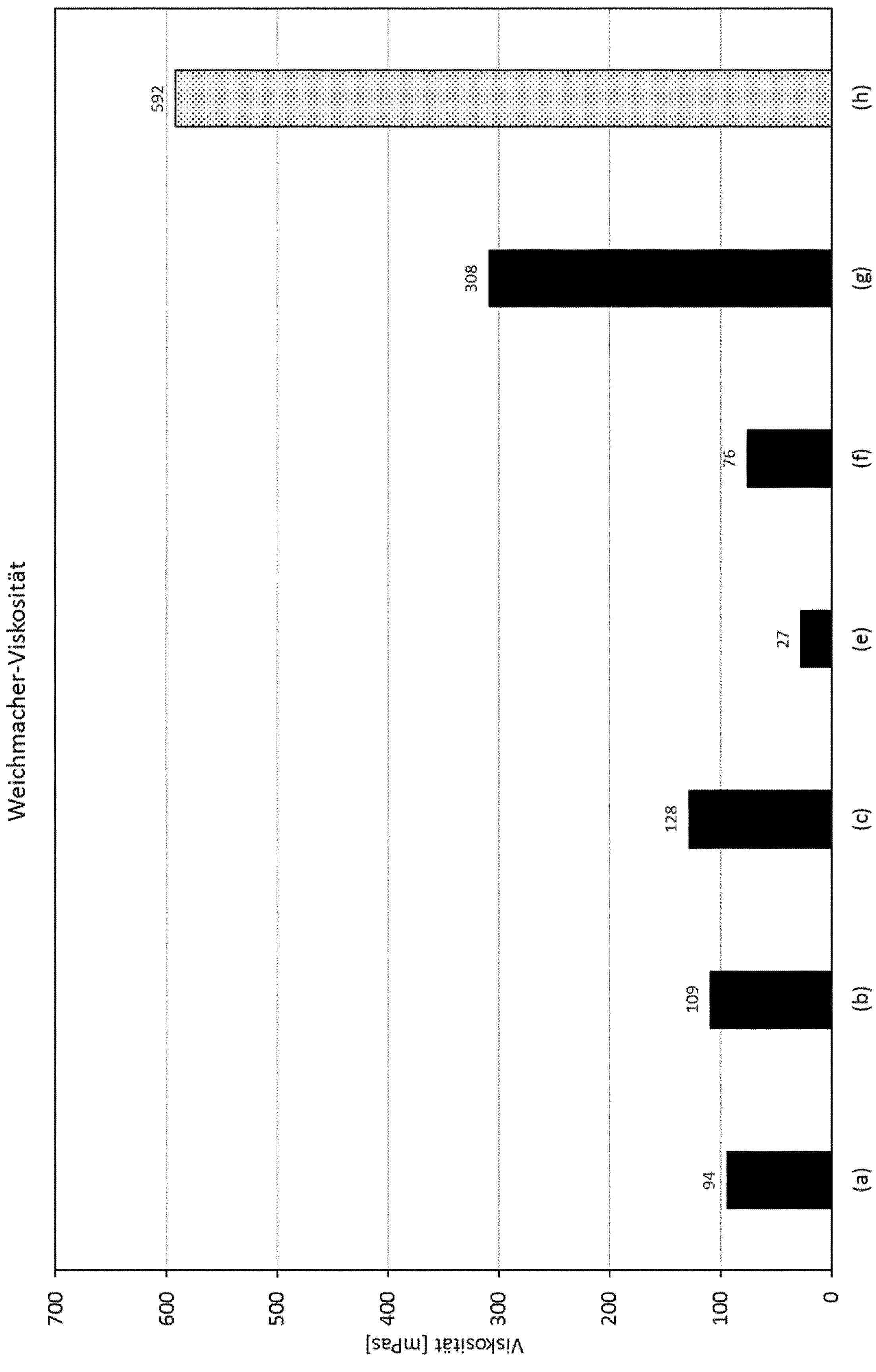
Weichmacher-Viskosität
Viskosität [mPas]
0
100
200
300
400
500
600
700
94
109
128
27
76
308
592
(a)
(b)
(c)
(e)
(f)
(g)
(h)

Fig. 1

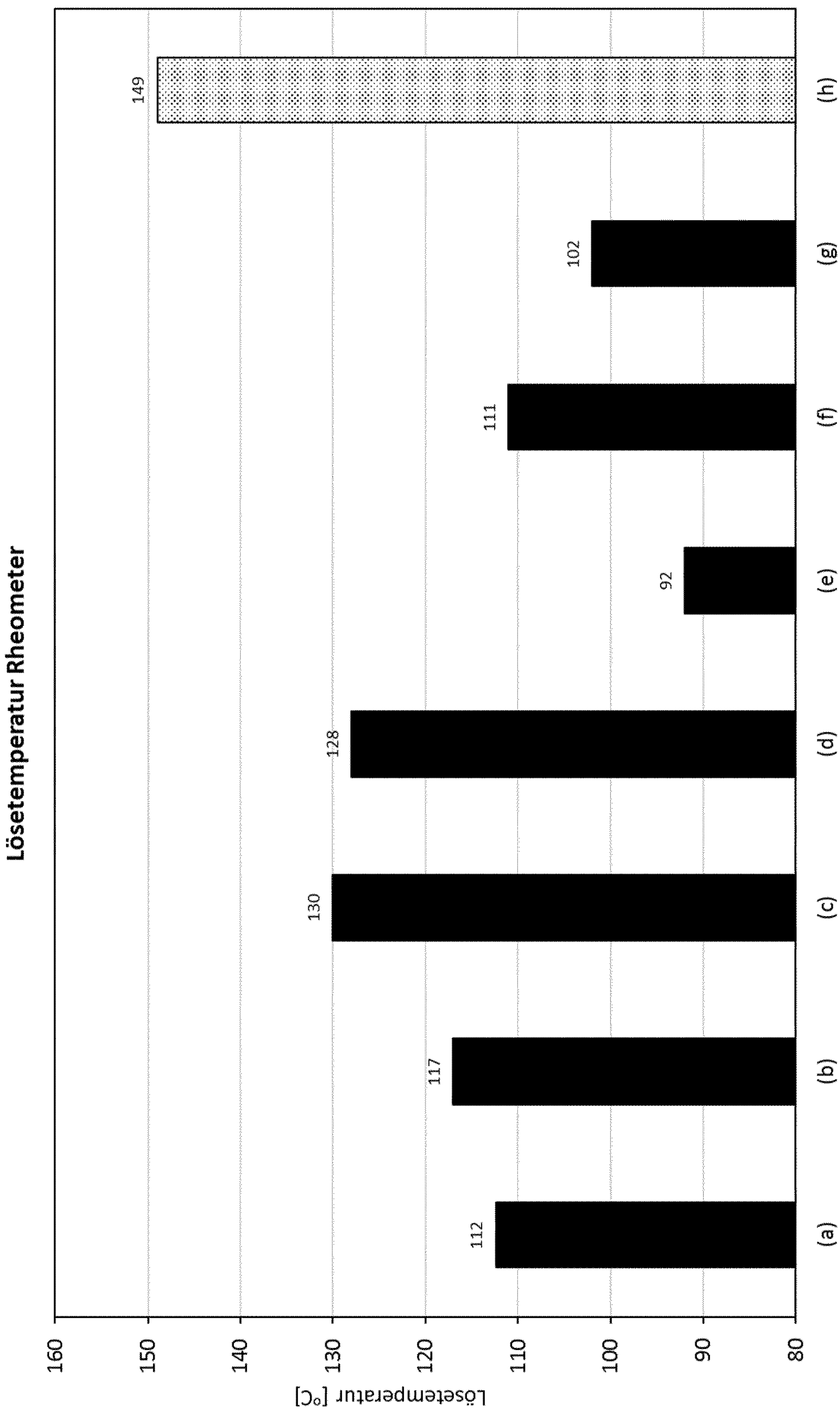
Lösetemperatur Rheometer
Lösetemperatur [°C]
160
150
140
130
120
110
100
90
80
112
117
130
128
92
111
102
149
(a)
(b)
(c)
(d)
(e)
(f)
(g)
(h)

Fig. 2

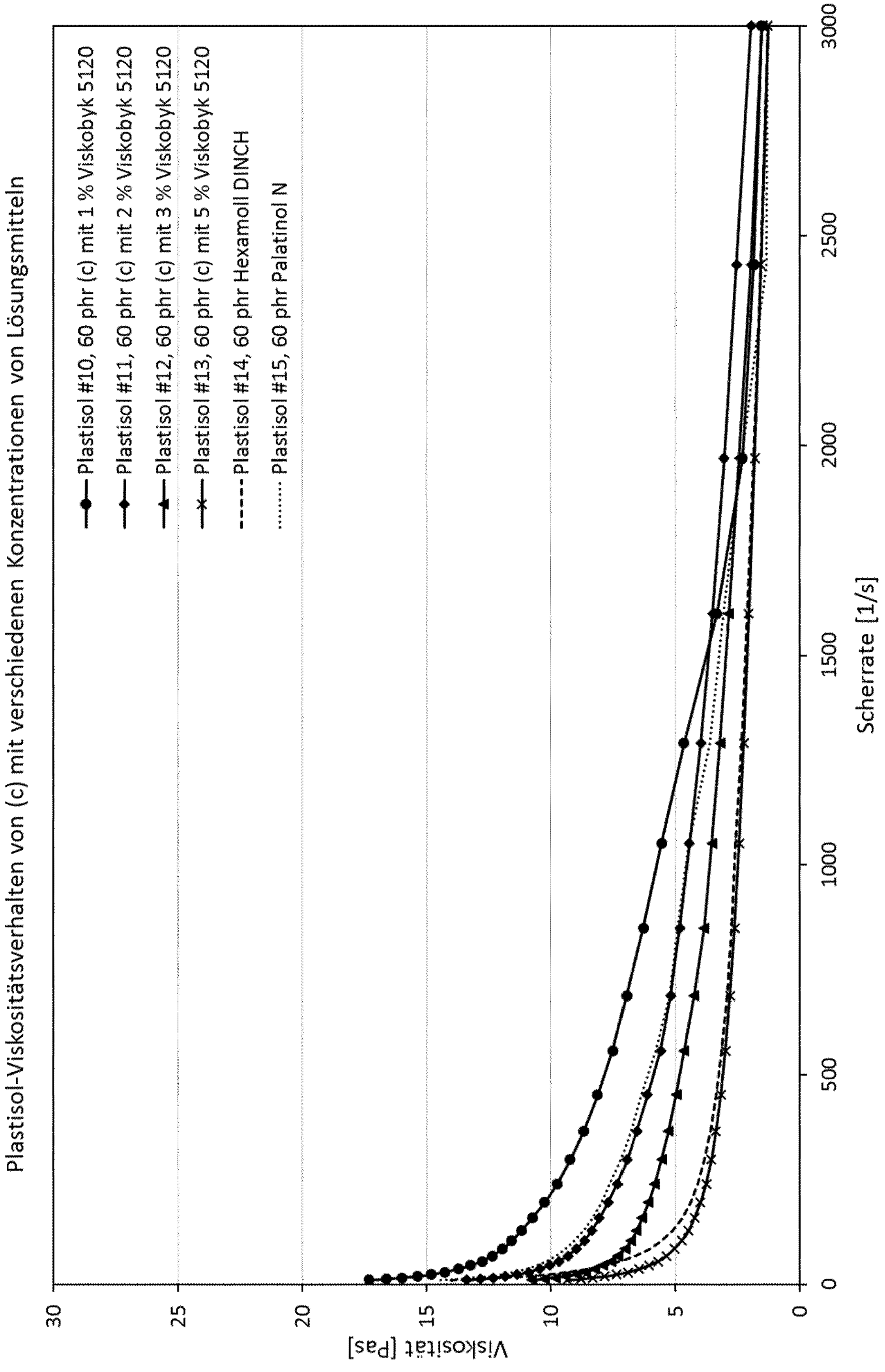
Plastisol-Viskositätsverhalten von (c) mit verschiedenen Konzentrationen von Lösungsmitteln
Plastisol #10, 60 phr (c) mit 1 % Viskobyk 5120
Plastisol #11, 60 phr (c) mit 2 % Viskobyk 5120
Plastisol #12, 60 phr (c) mit 3 % Viskobyk 5120
Plastisol #13, 60 phr (c) mit 5 % Viskobyk 5120
Plastisol #14, 60 phr Hexamoll DINCH
Plastisol #15, 60 phr Palatinol N
Viskosität [Pas]
Scherrate [1/s]
0
5
10
15
20
25
30
0
500
1000
1500
2000
2500
3000

Fig. 3

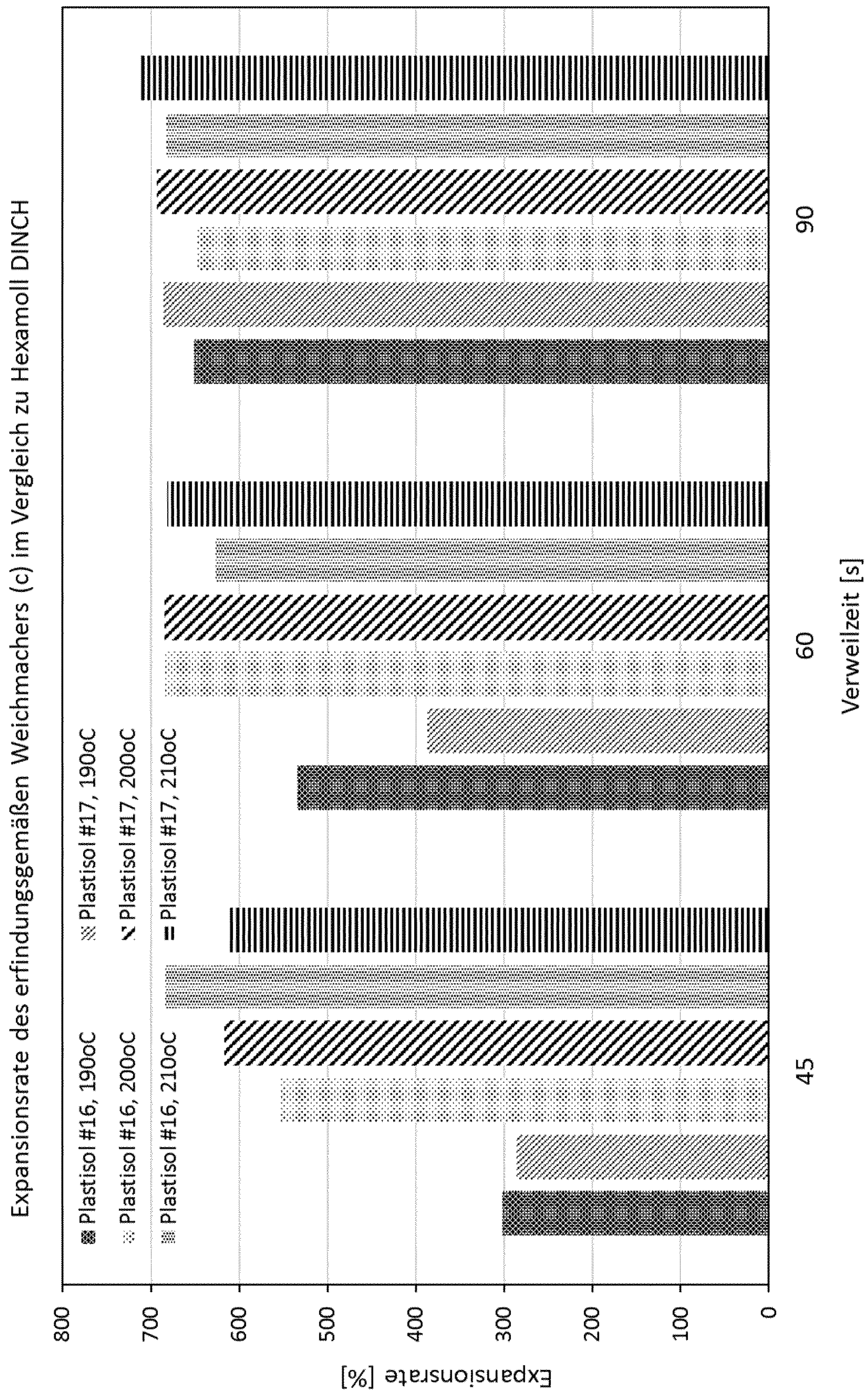

Expansionsrate des erfindungsgemäßen Weichmachers (c) im Vergleich zu Hexamoll DINCH
Plastisol #16, 190oC
Plastisol #16, 200oC
Plastisol #16, 210oC
Plastisol #17, 190oC
Plastisol #17, 200oC
Plastisol #17, 210oC
Expansionsrate [%]
800
700
600
500
400
300
200
100
0
45
60
90
Verweilzeit [s]


Fig. 4

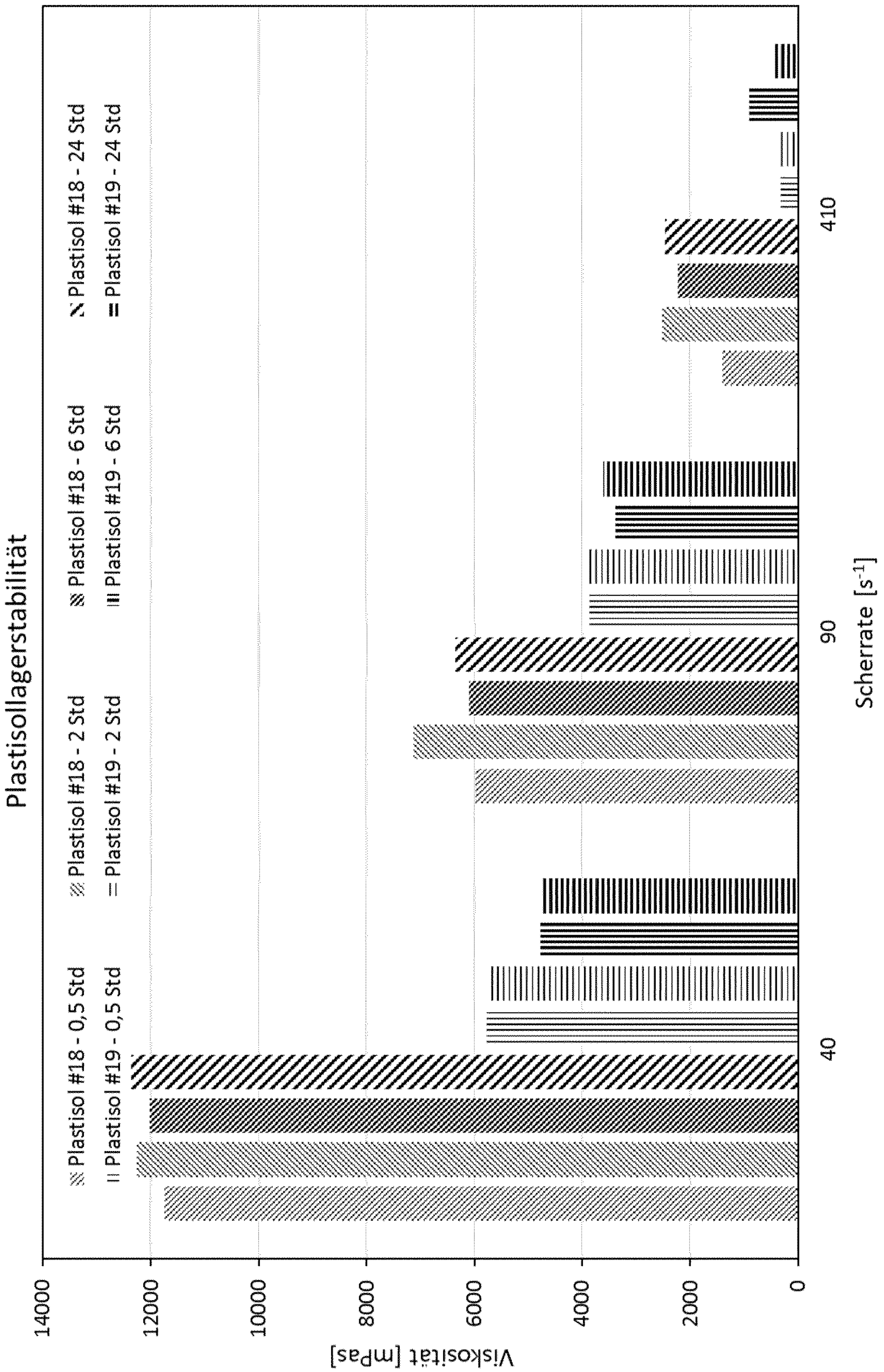
Plastisollagerstabilität
Plastisol #18 - 0,5 Std
Plastisol #19 - 0,5 Std
Plastisol #18 - 2 Std
Plastisol #19 - 2 Std
Plastisol #18 - 6 Std
Plastisol #19 - 6 Std
Plastisol #18 - 24 Std
Plastisol #19 - 24 Std
Viskosität [mPas]
14000
12000
10000
8000
6000
4000
2000
0
40
90
410
Scherrate [s-1]

Fig. 5

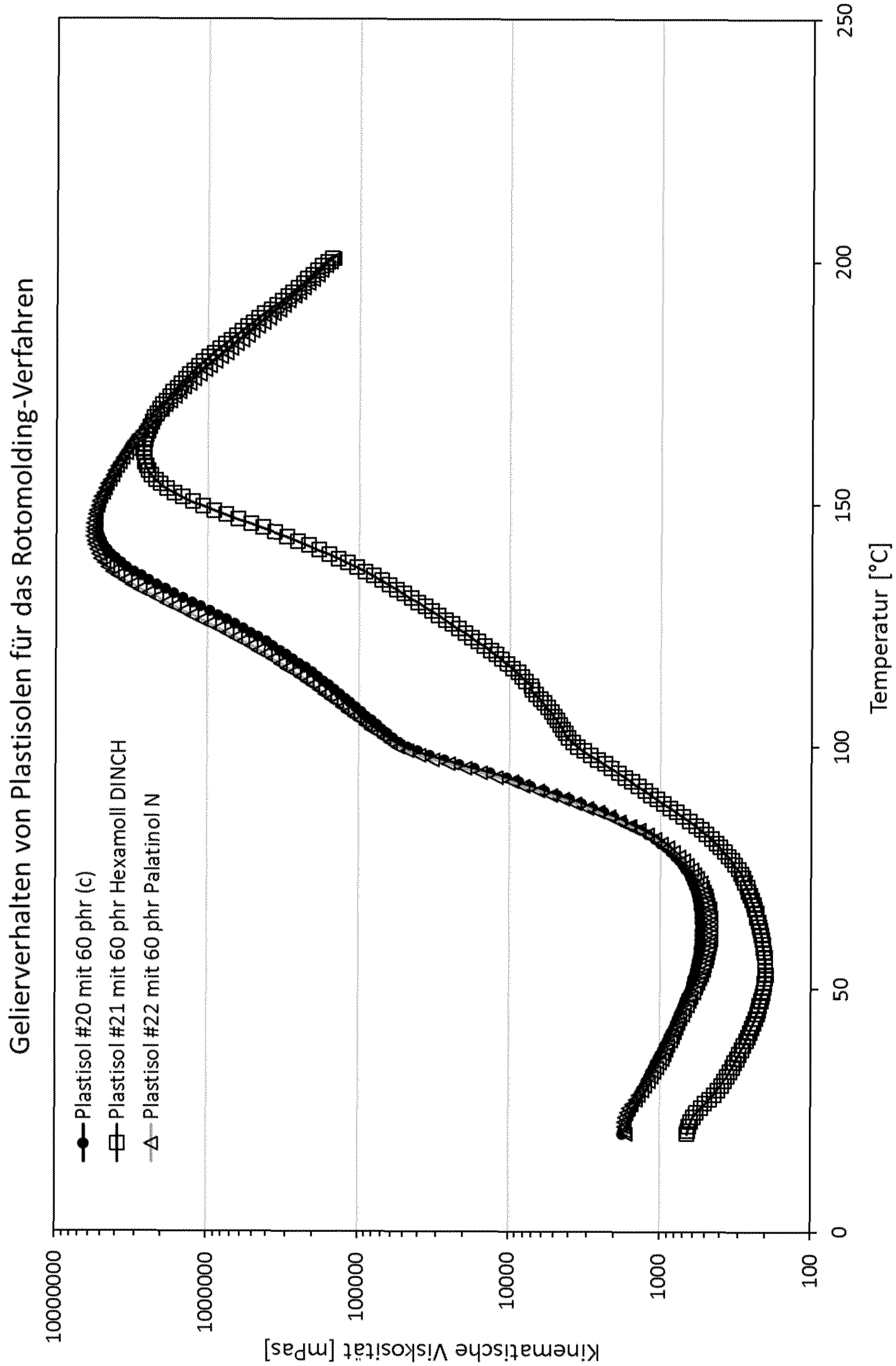
Gelierverhalten von Plastisolen für das Rotomolding-Verfahren
Plastisol #20 mit 60 phr (c)
Plastisol #21 mit 60 phr Hexamoll DINCH
Plastisol #22 mit 60 phr Palatinol N
Kinematische Viskosität [mPas]
10000000
1000000
100000
10000
1000
100
Temperatur [°C]
0
50
100
150
200
250

Fig. 6

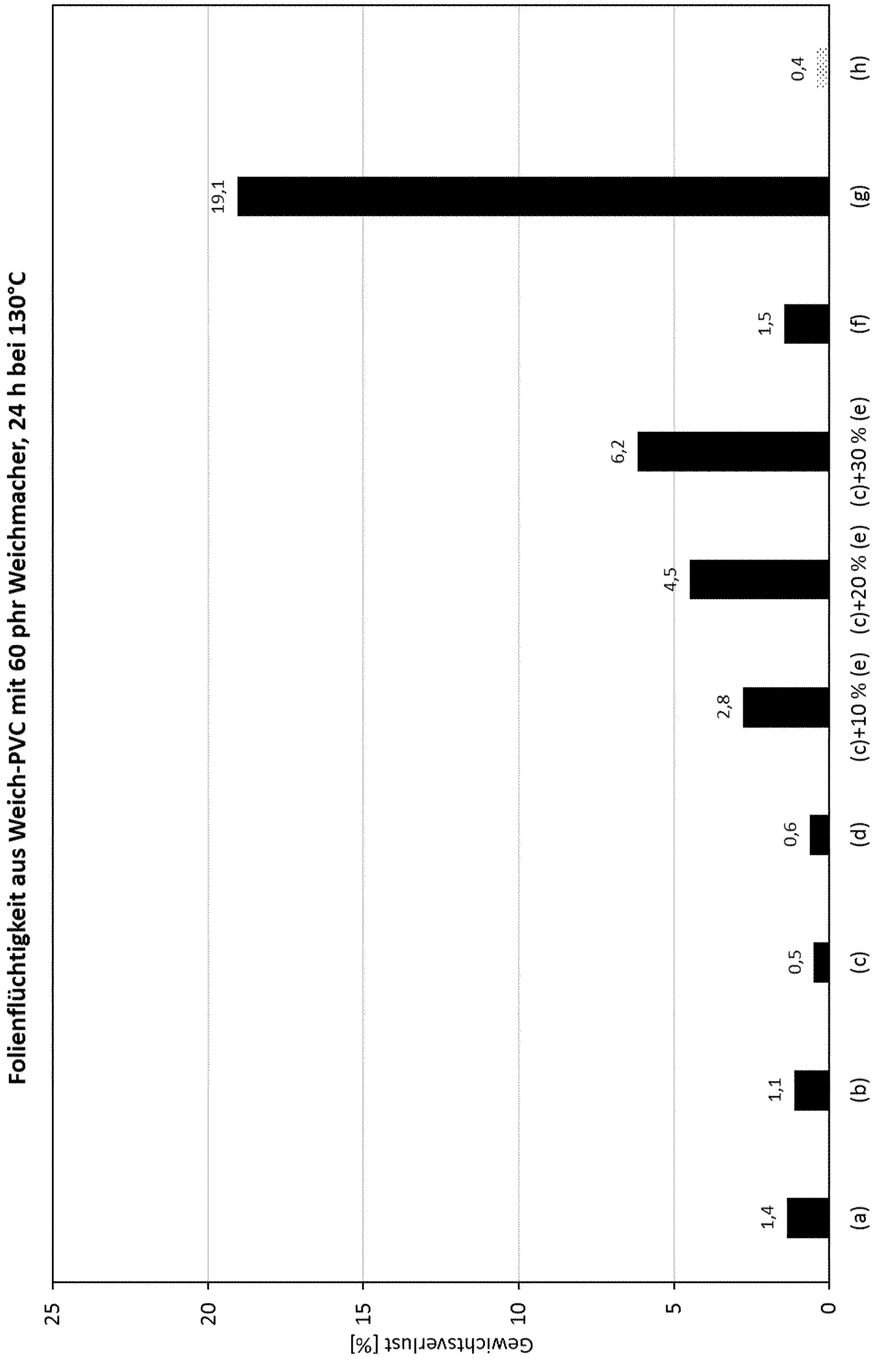
Folienflüchtigkeit aus Weich-PVC mit 60 phr Weichmacher, 24 h bei 130°C
Gewichtsverlust [%]
0
5
10
15
20
25
1,4
1,1
0,5
0,6
2,8
4,5
6,2
1,5
19,1
0,4
(a)
(b)
(c)
(d)
(c)+10 % (e)
(c)+20 % (e)
(c)+30 % (e)
(f)
(g)
(h)

Fig. 7

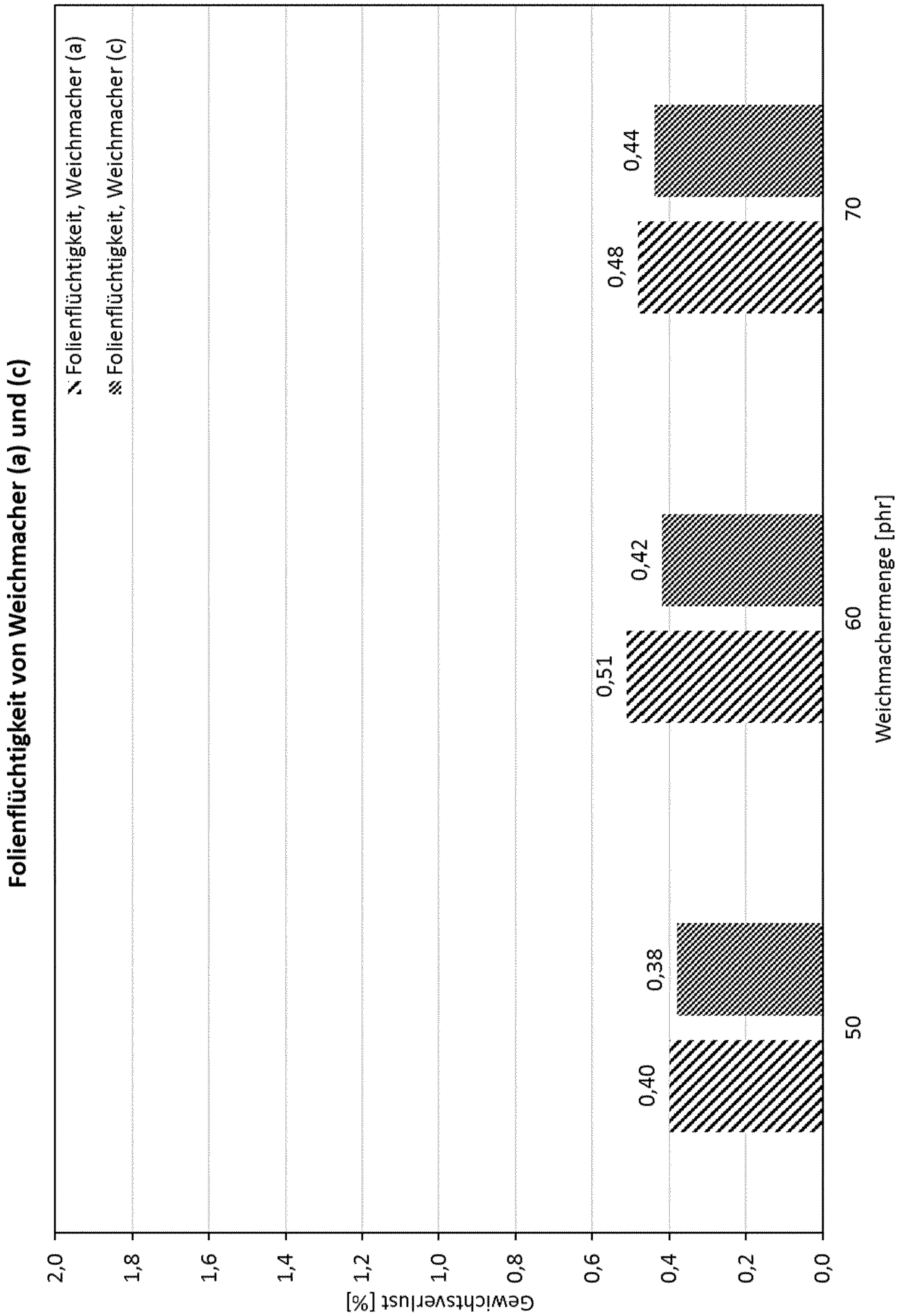
Folienflüchtigkeit von Weichmacher (a) und (c)
Folienflüchtigkeit, Weichmacher (a)
Folienflüchtigkeit, Weichmacher (c)
Gewichtsverlust [%]
2,0
1,8
1,6
1,4
1,2
1,0
0,8
0,6
0,4
0,2
0,0
0,40
0,38
0,51
0,42
0,48
0,44
50
60
70
Weichmachermenge [phr]

Fig. 8

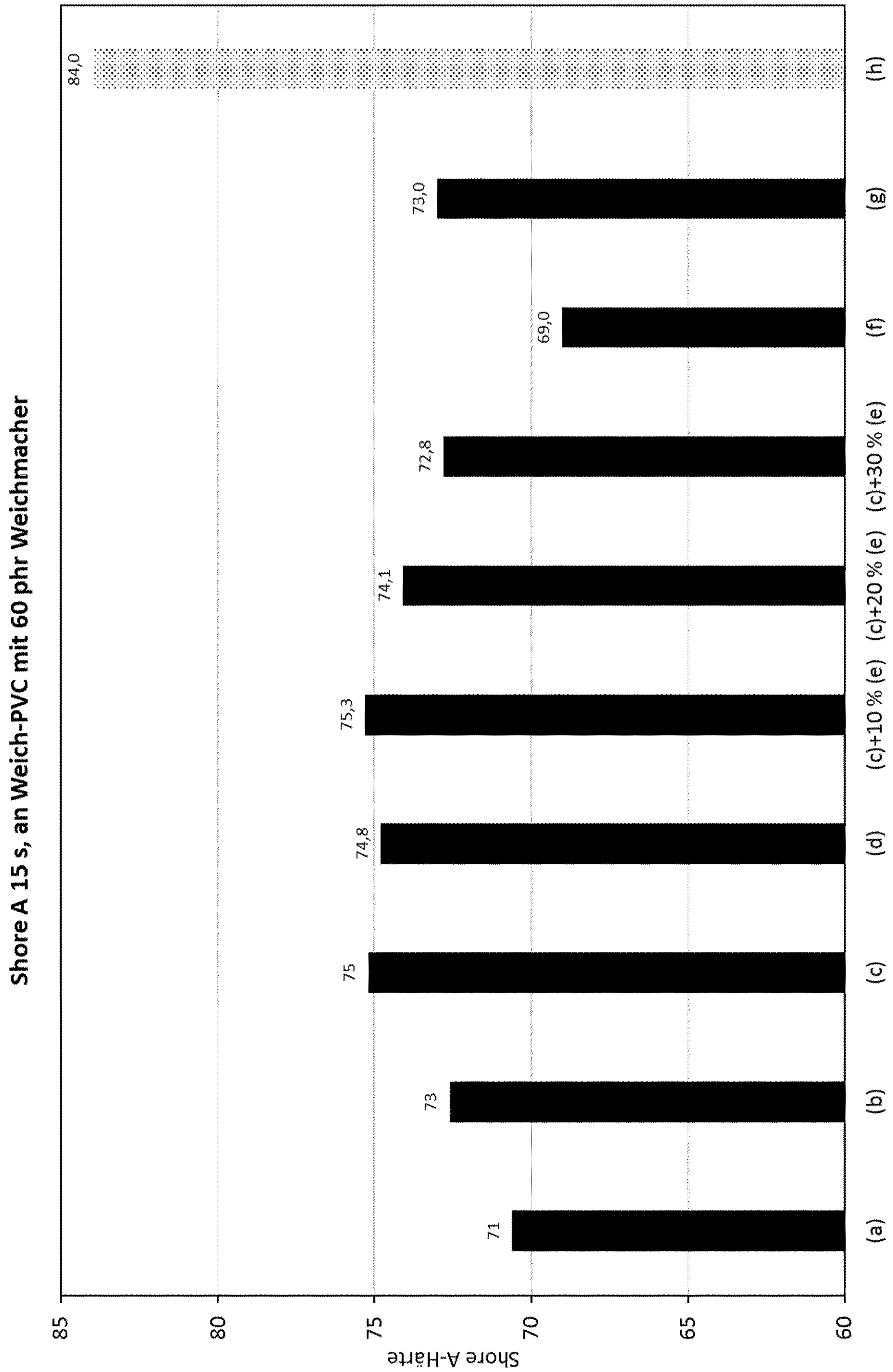
Shore A 15 s, an Weich-PVC mit 60 phr Weichmacher
Shore A-Härte
85
80
75
70
65
60
71
73
75
74,8
75,3
74,1
72,8
69,0
73,0
84,0
(a)
(b)
(c)
(d)
(c)+10 % (e)
(c)+20 % (e)
(c)+30 % (e)
(f)
(g)
(h)

Fig. 9

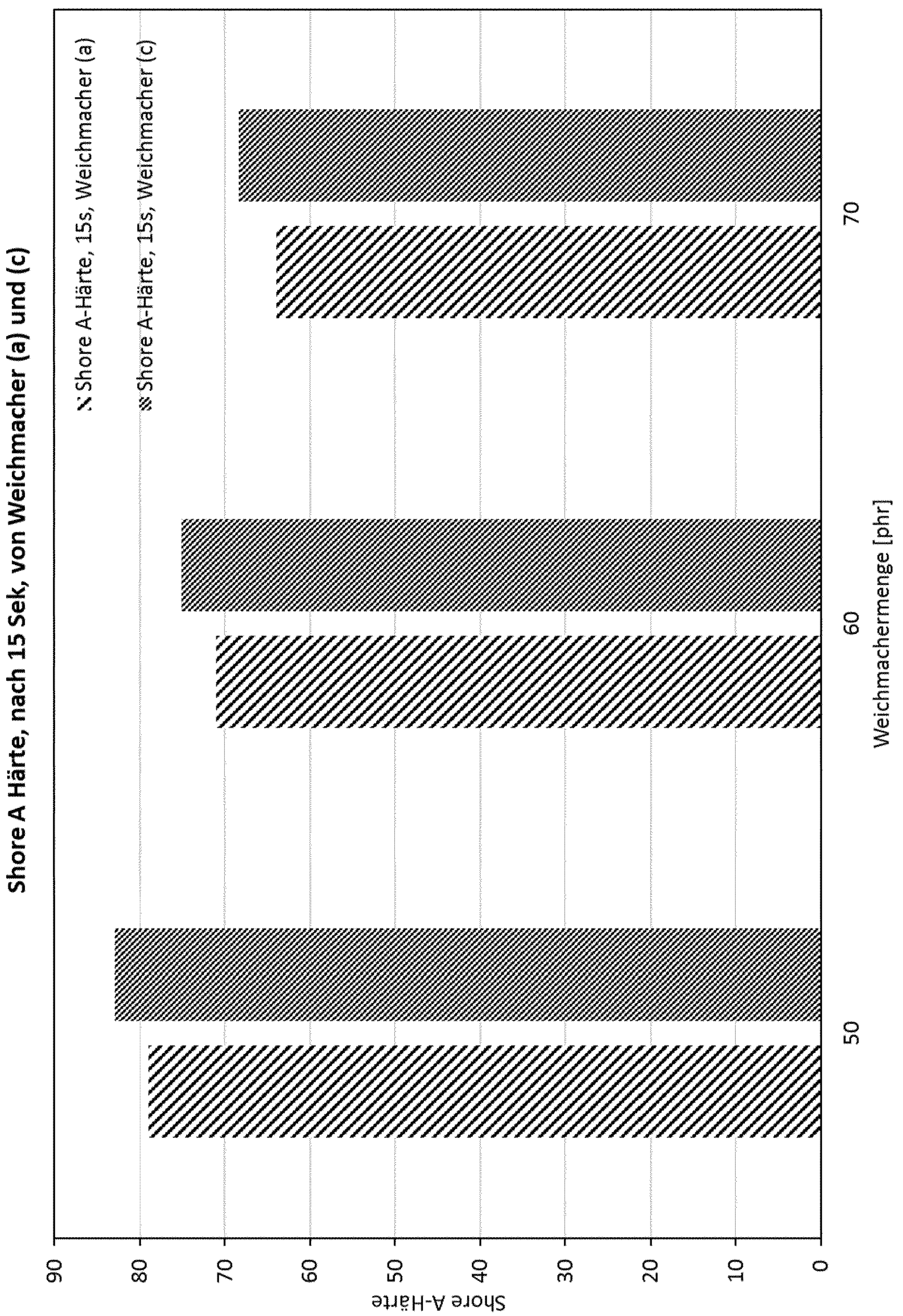
Shore A Härte, nach 15 Sek, von Weichmacher (a) und (c)
Shore A-Härte, 15s, Weichmacher (a)
Shore A-Härte, 15s, Weichmacher (c)
Shore A-Härte
90
80
70
60
50
40
30
20
10
0
50
60
70
Weichmachermenge [phr]

Fig. 10

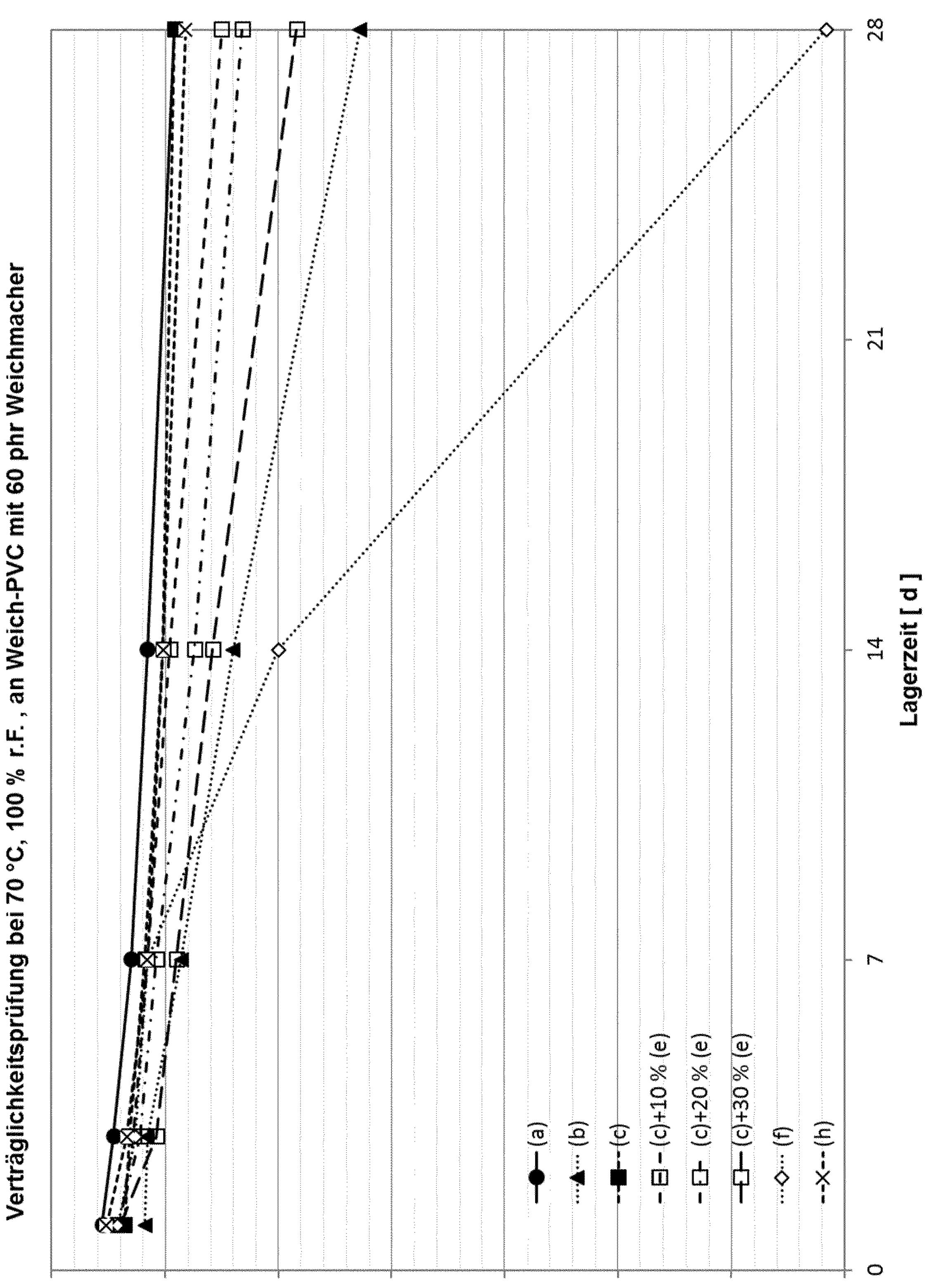
Verträglichkeitsprüfung bei 70 °C, 100 % r.F. , an Weich-PVC mit 60 phr Weichmacher
Gewichtsverlust [%]
0,0
-0,5
-1,0
-1,5
-2,0
-2,5
-3,0
-3,5
Lagerzeit [ d ]
0
7
14
21
28
(a)
(b)
(c)
(c)+10 % (e)
(c)+20 % (e)
(c)+30 % (e)
(f)
(h)

Fig. 11

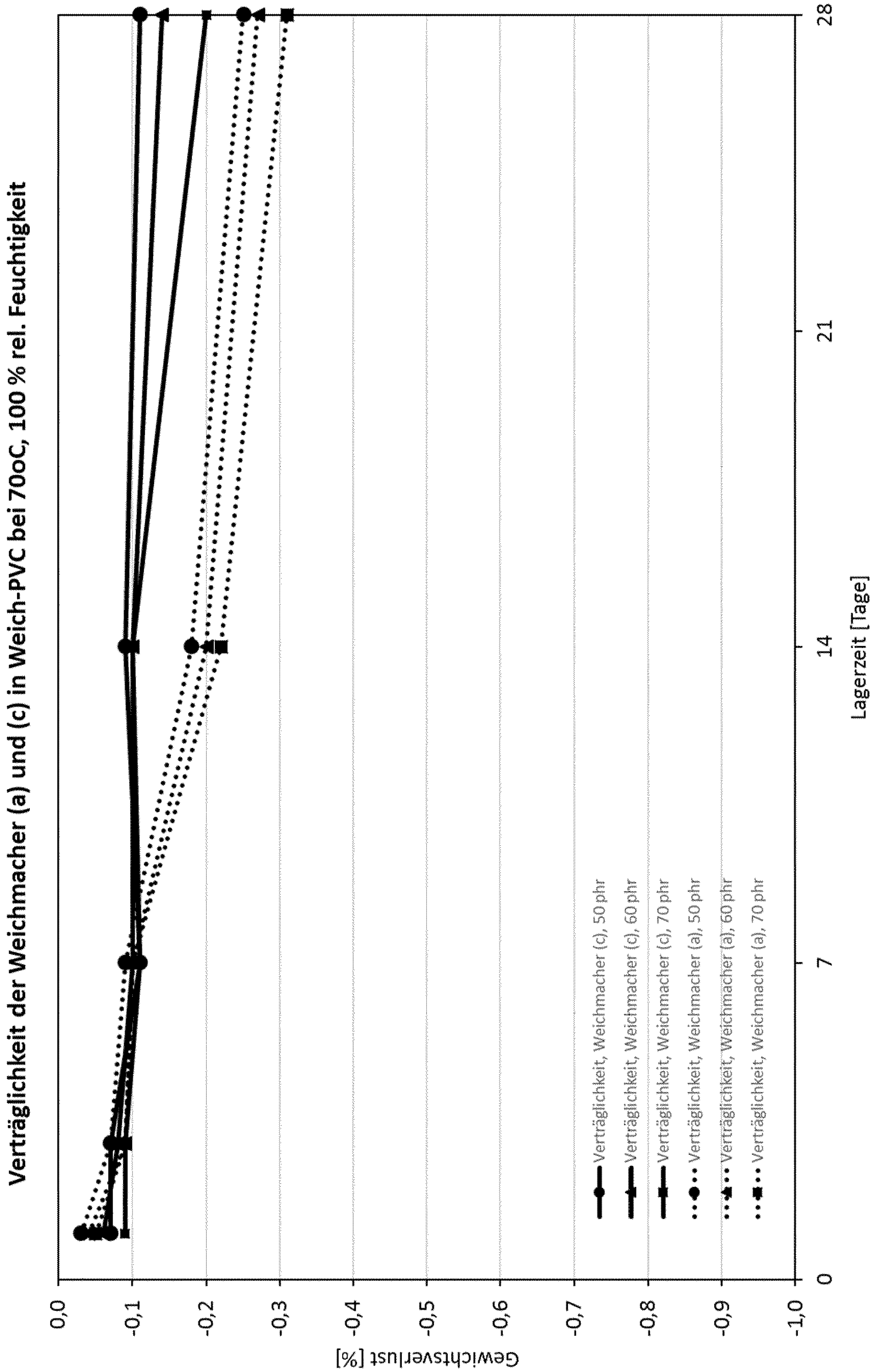

Verträglichkeit der Weichmacher (a) und (c) in Weich-PVC bei 70oC, 100 % rel. Feuchtigkeit
Gewichtsverlust [%]
0,0
-0,1
-0,2
-0,3
-0,4
-0,5
-0,6
-0,7
-0,8
-0,9
-1,0
0
7
14
21
28
Lagerzeit [Tage]
Verträglichkeit, Weichmacher (c), 50 phr
Verträglichkeit, Weichmacher (c), 60 phr
Verträglichkeit, Weichmacher (c), 70 phr
Verträglichkeit, Weichmacher (a), 50 phr
Verträglichkeit, Weichmacher (a), 60 phr
Verträglichkeit, Weichmacher (a), 70 phr


Fig. 12

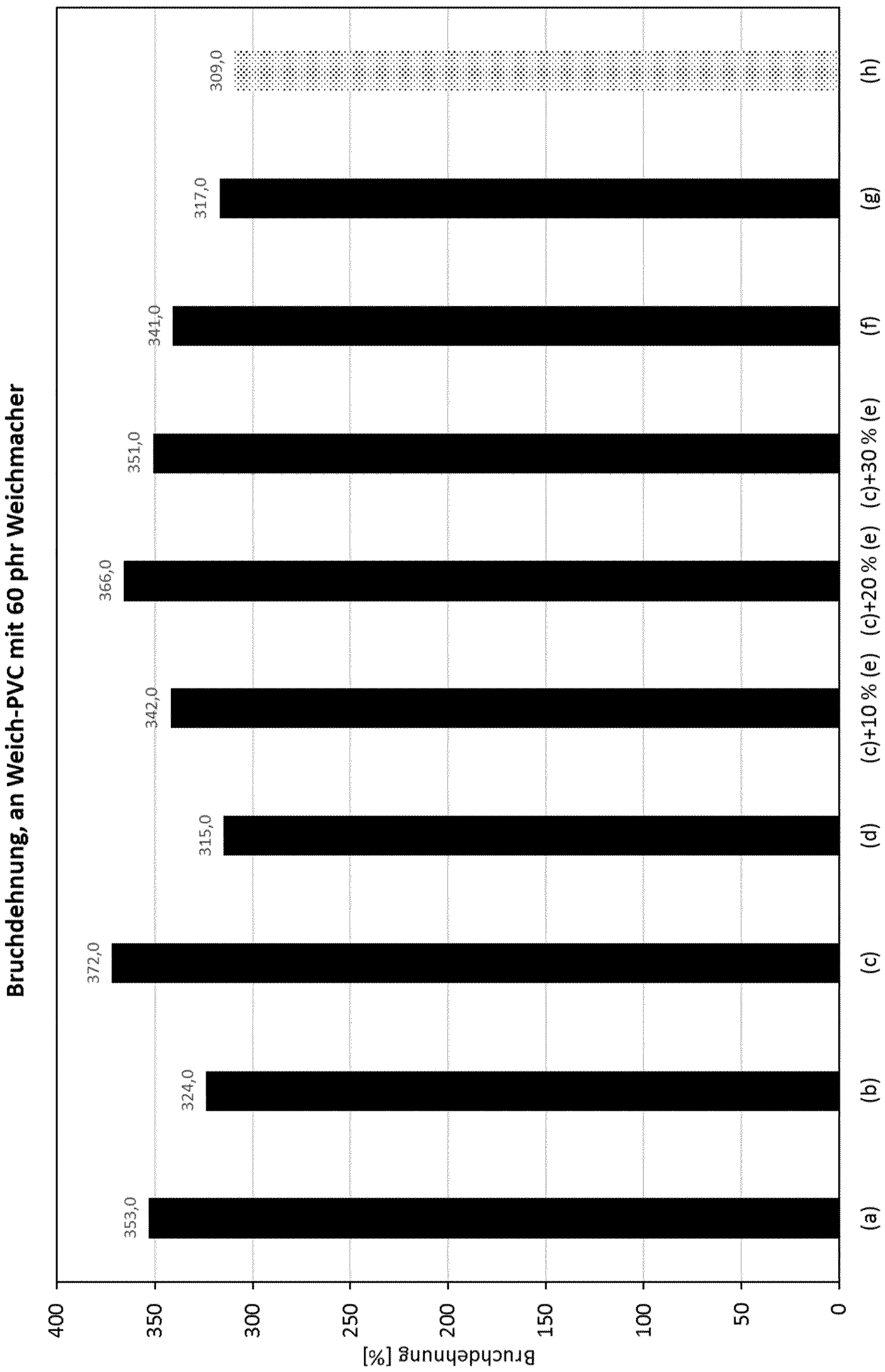
Bruchdehnung, an Weich-PVC mit 60 phr Weichmacher
Bruchdehnung [%]
400
350
300
250
200
150
100
50
0
353,0
324,0
372,0
315,0
342,0
366,0
351,0
341,0
317,0
309,0
(a)
(b)
(c)
(d)
(c)+10 % (e)
(c)+20 % (e)
(c)+30 % (e)
(f)
(g)
(h)

Fig. 13

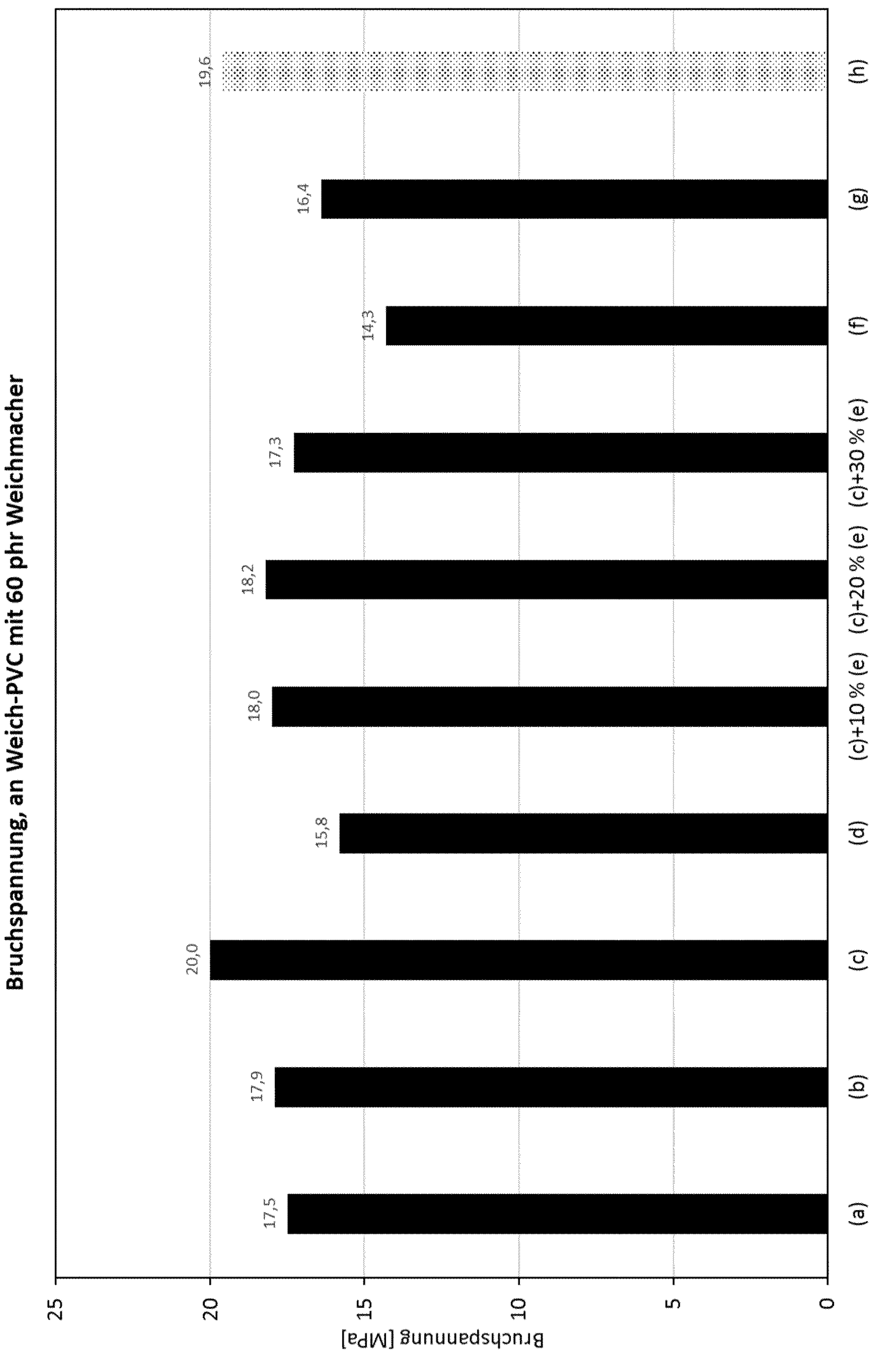
Bruchspannung, an Weich-PVC mit 60 phr Weichmacher
Bruchspannung [MPa]
0
5
10
15
20
25
17,5
17,9
20,0
15,8
18,0
18,2
17,3
14,3
16,4
19,6
(a)
(b)
(c)
(d)
(c)+10 % (e)
(c)+20 % (e)
(c)+30 % (e)
(f)
(g)
(h)

Fig. 14

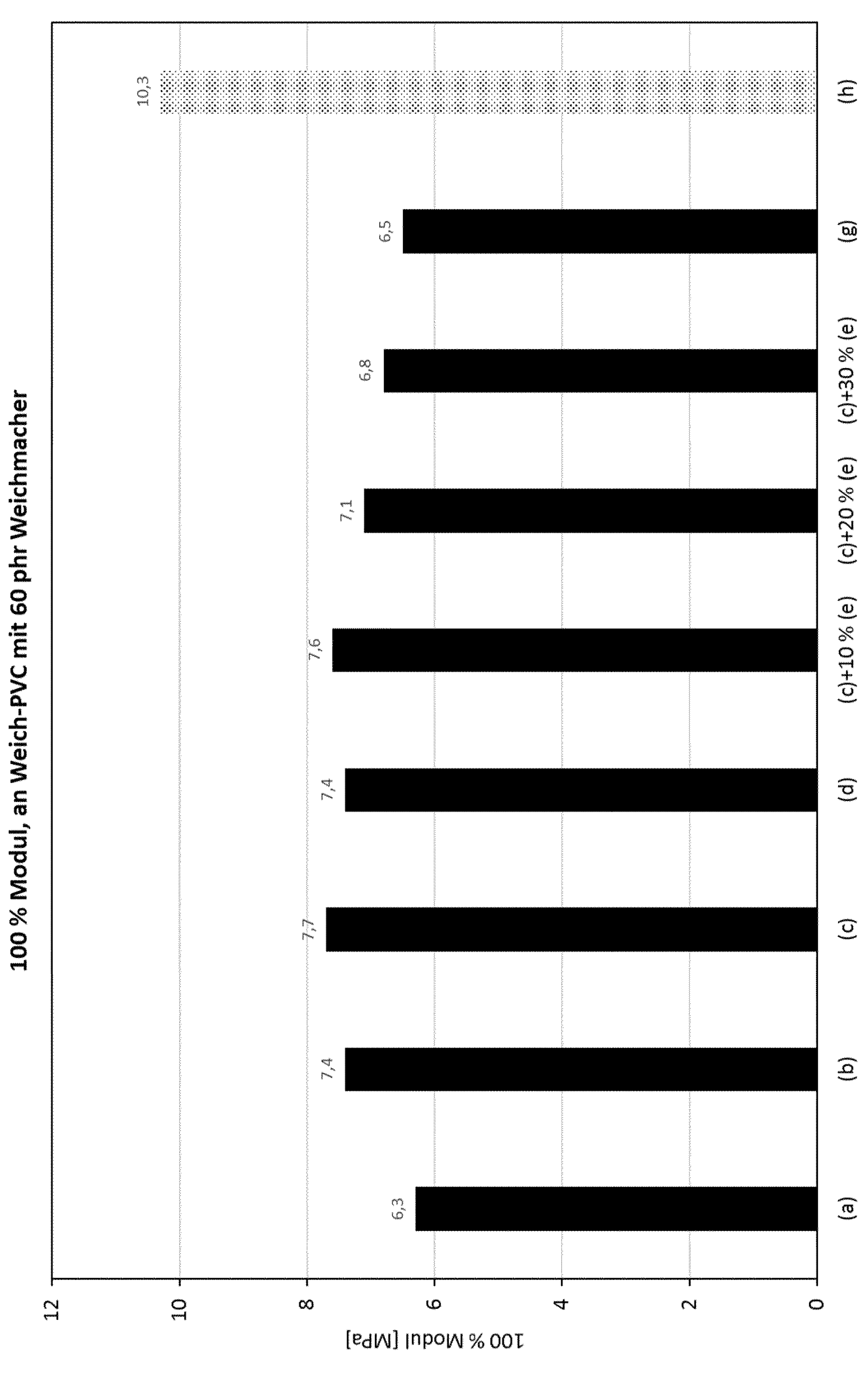
100 % Modul, an Weich-PVC mit 60 phr Weichmacher
100 % Modul [MPa]
0
2
4
6
8
10
12
6,3
7,4
7,7
7,4
7,6
7,1
6,8
6,5
10,3
(a)
(b)
(c)
(d)
(c)+10 % (e)
(c)+20 % (e)
(c)+30 % (e)
(g)
(h)

Fig. 15

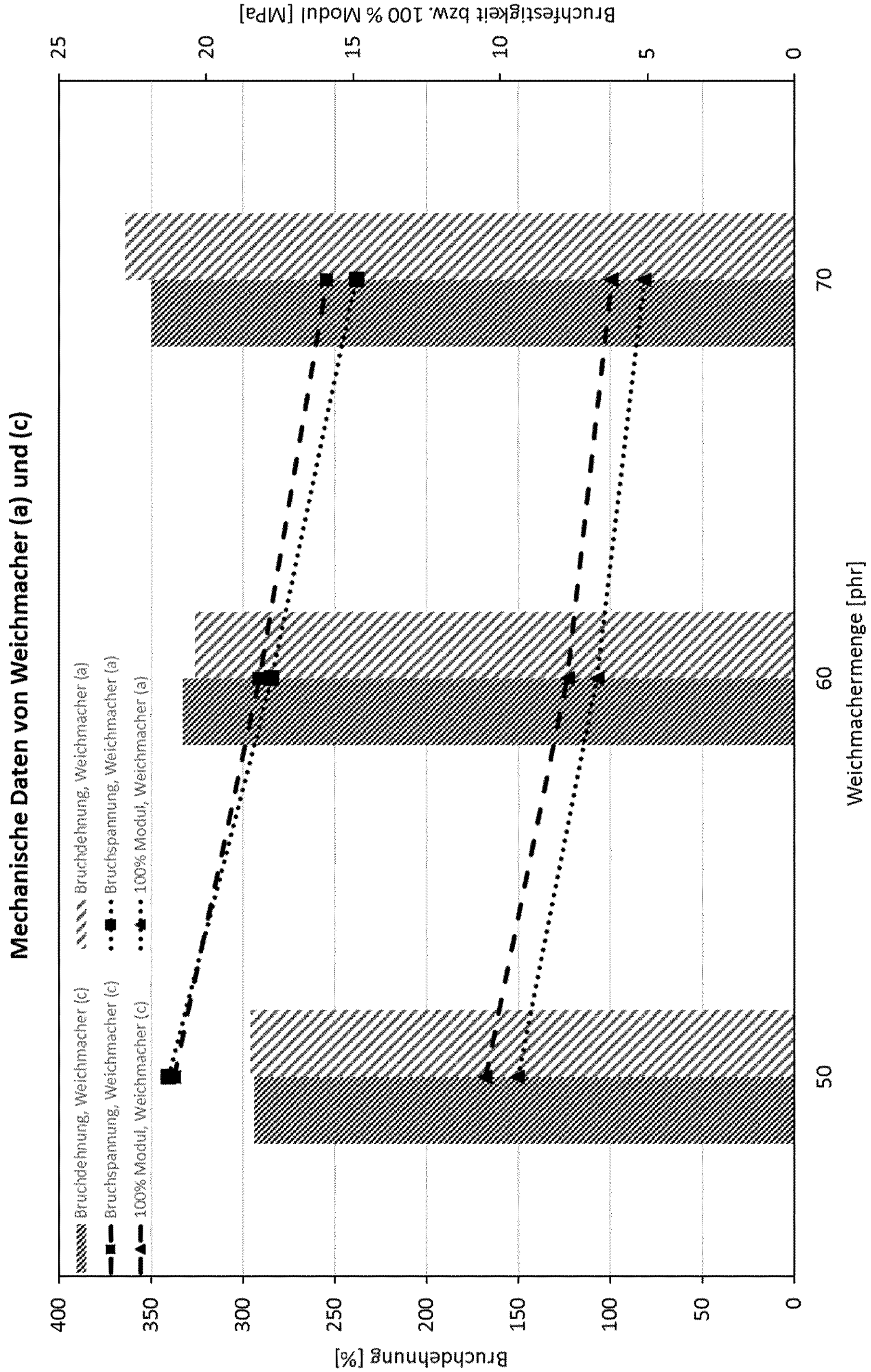

Mechanische Daten von Weichmacher (a) und (c)
Bruchdehnung, Weichmacher (c)
Bruchspannung, Weichmacher (c)
100% Modul, Weichmacher (c)
Bruchdehnung, Weichmacher (a)
Bruchspannung, Weichmacher (a)
100% Modul, Weichmacher (a)
Bruchdehnung [%]
0
50
100
150
200
250
300
350
400
Bruchfestigkeit bzw. 100 % Modul [MPa]
0
5
10
15
20
25
50
60
70
Weichmachermenge [phr]


Fig. 16

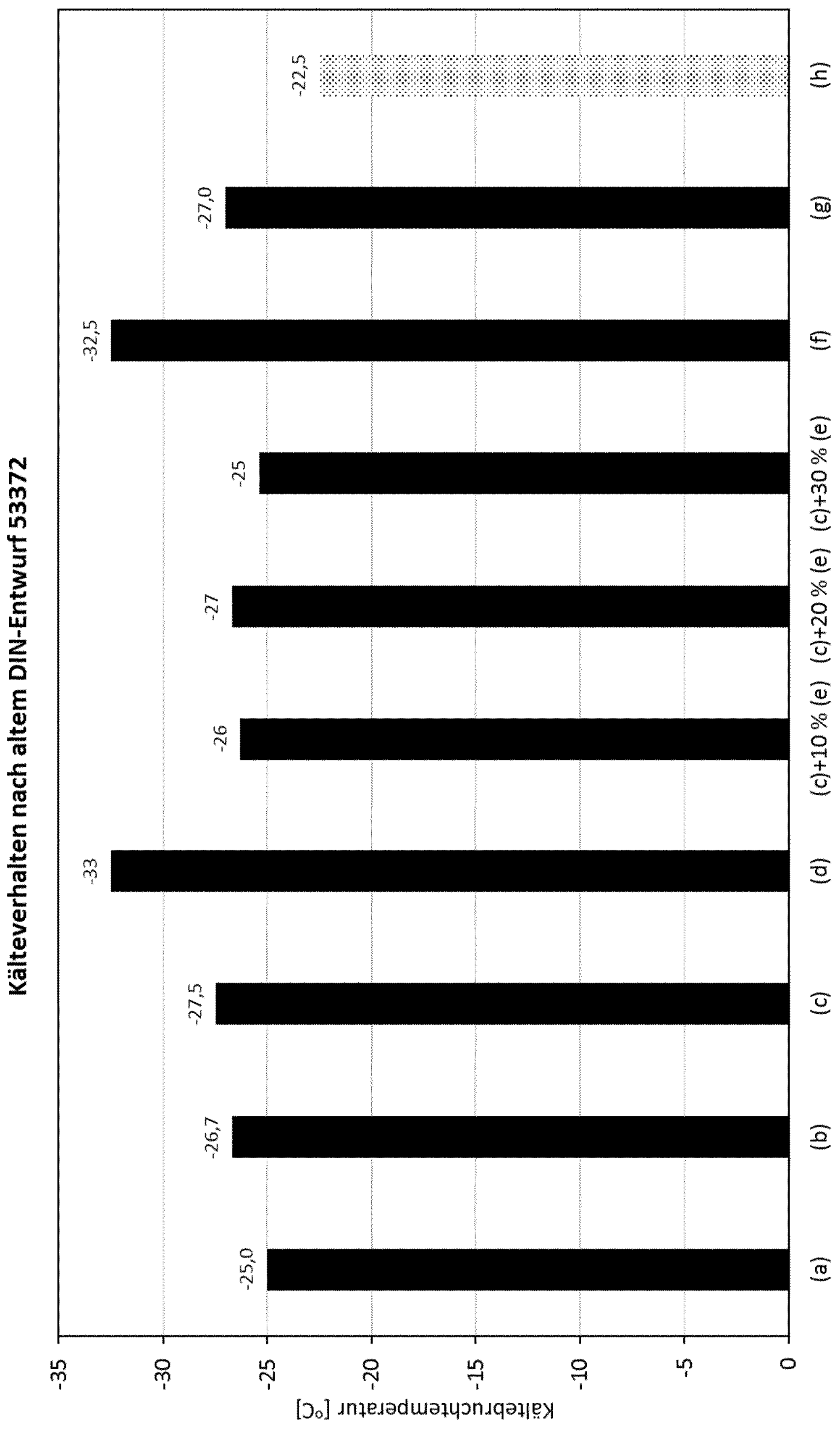
Kälteverhalten nach altem DIN-Entwurf 53372
Kältebruchtemperatur [°C]
-35
-30
-25
-20
-15
-10
-5
0
-25,0
-26,7
-27,5
-33
-26
-27
-25
-32,5
-27,0
-22,5
(a)
(b)
(c)
(d)
(c)+10 % (e)
(c)+20 % (e)
(c)+30 % (e)
(f)
(g)
(h)

Fig. 17

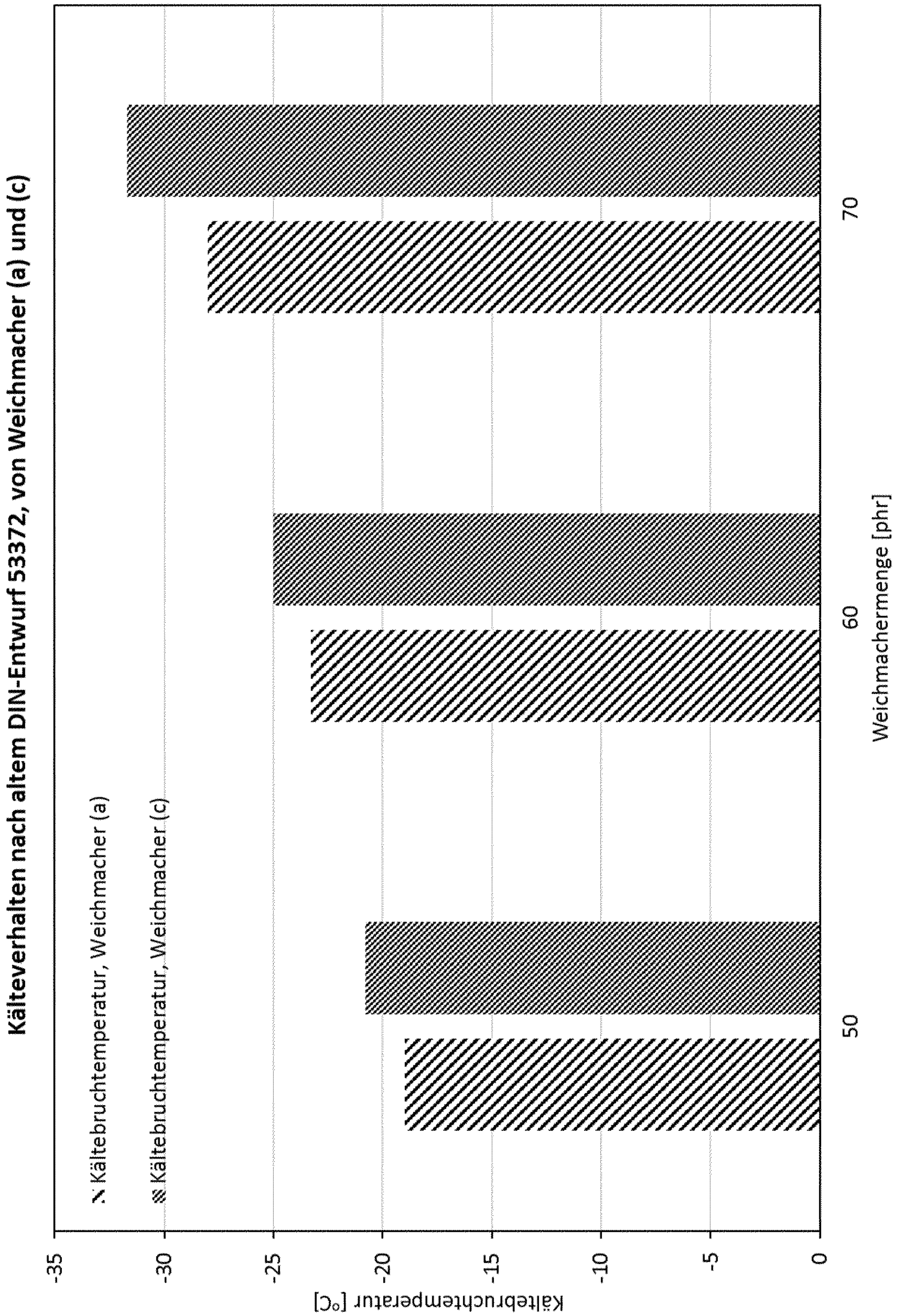
Kälteverhalten nach altem DIN-Entwurf 53372, von Weichmacher (a) und (c)
Kältebruchtemperatur, Weichmacher (a)
Kältebruchtemperatur, Weichmacher (c)
Kältebruchtemperatur [°C]
-35
-30
-25
-20
-15
-10
-5
0
50
60
70
Weichmachermenge [phr]

Fig. 18

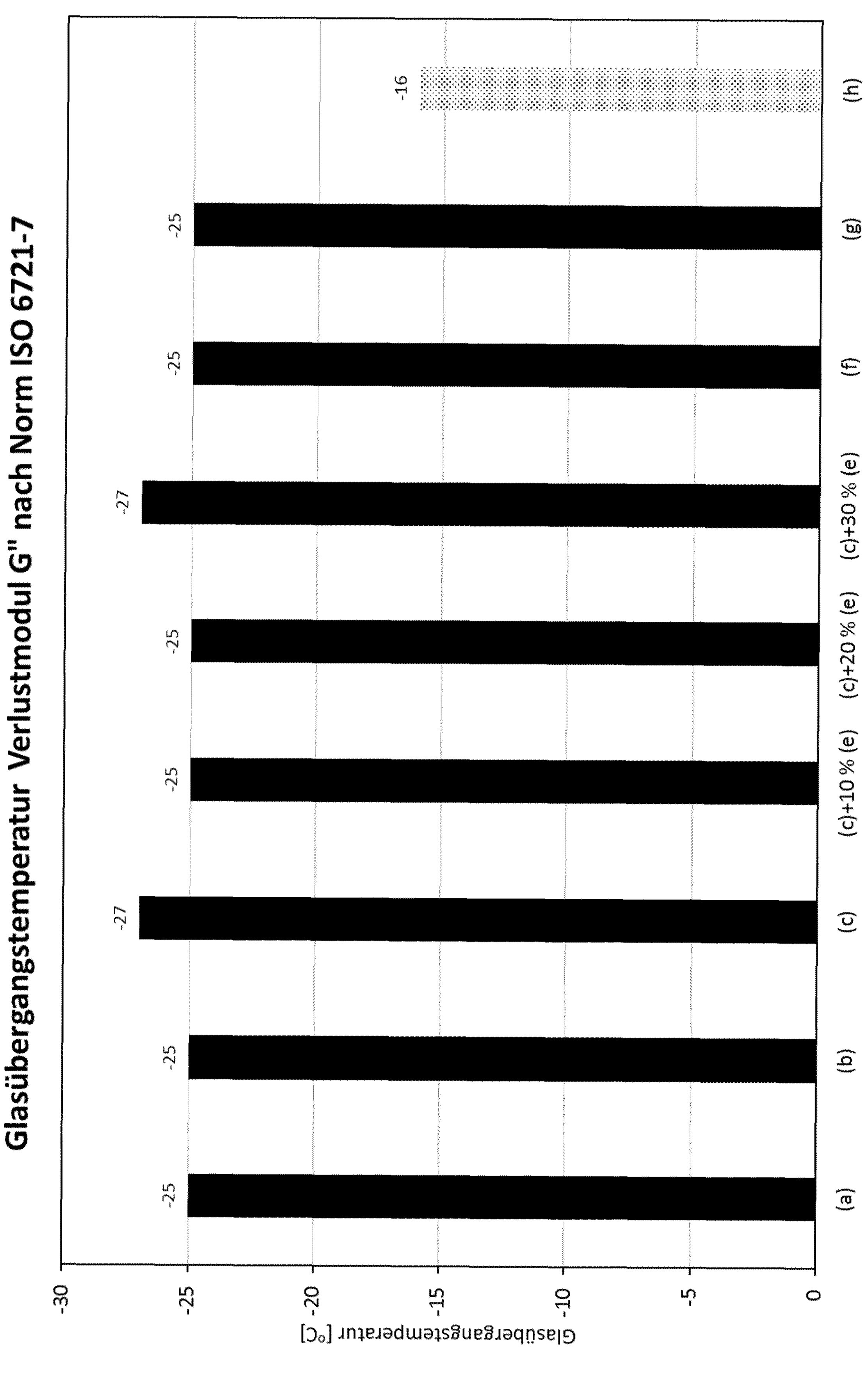
Glasübergangstemperatur Verlustmodul G'' nach Norm ISO 6721-7
Glasübergangstemperatur [°C]
-30
-25
-20
-15
-10
-5
0
-25
-25
-27
-25
-25
-27
-25
-25
-16
(a)
(b)
(c)
(c)+10 % (e)
(c)+20 % (e)
(c)+30 % (e)
(f)
(g)
(h)

Fig. 19

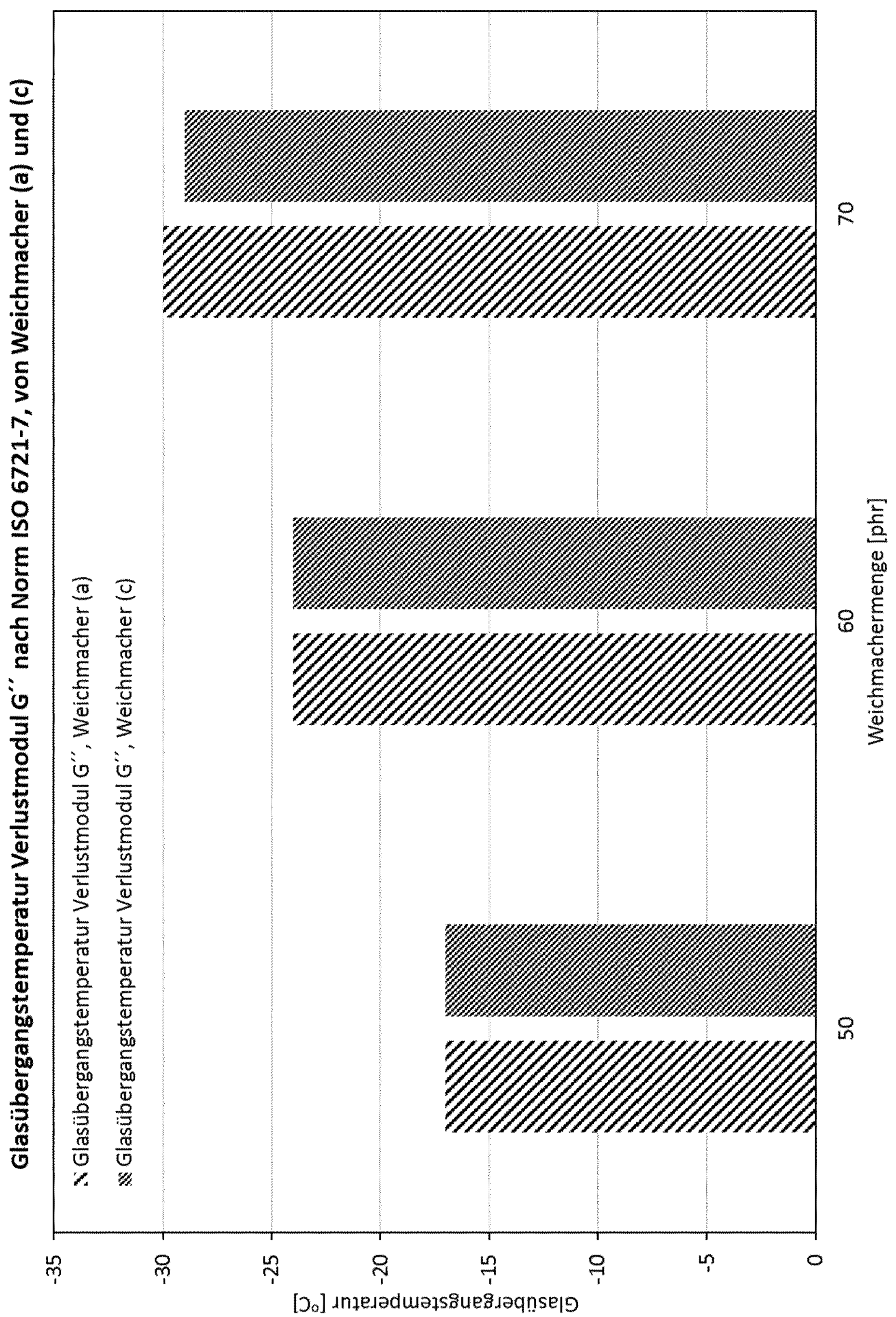
Glasübergangstemperatur Verlustmodul G´´ nach Norm ISO 6721-7, von Weichmacher (a) und (c)
Glasübergangstemperatur Verlustmodul G´´, Weichmacher (a)
Glasübergangstemperatur Verlustmodul G´´, Weichmacher (c)
Glasübergangstemperatur [°C]
-35
-30
-25
-20
-15
-10
-5
0
50
60
70
Weichmachermenge [phr]

Fig. 20

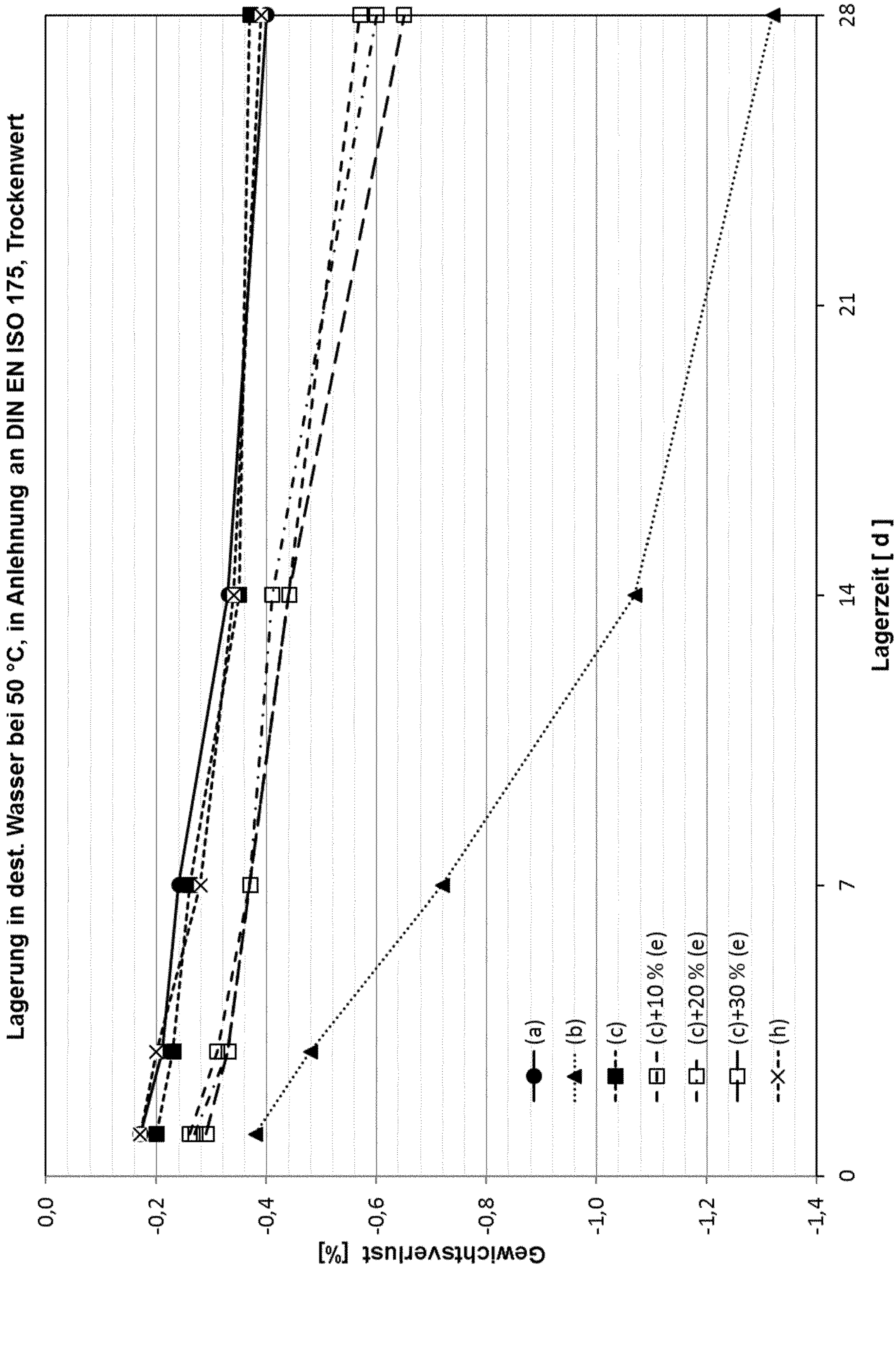
Lagerung in dest. Wasser bei 50 °C, in Anlehnung an DIN EN ISO 175, Trockenwert
Gewichtsverlust [%]
0,0
-0,2
-0,4
-0,6
-0,8
-1,0
-1,2
-1,4
Lagerzeit [ d ]
0
7
14
21
28
(a)
(b)
(c)
(c)+10 % (e)
(c)+20 % (e)
(c)+30 % (e)
(h)

Fig. 21

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9932427 A
- JP 49085136 A
- US 3076775 A
- WO 2018024591 A1
- WO 02038531 A
- US 6310235 B
- US 5324853 A
- DE 2612355 A
- DE 1945359 A
- US 5288918 A
- DE 1593368 A
- DE 2139630 A
- DE 2244373 A
- DE 2404855 A
- WO 01014297 A
- US 4148830 A
- EP 695734 A
- EP 880494 B
- EP 1047655 B
- DE 3228881 A
- DE 2628987 A
- DE 2445303 A
- WO 01087809 A
- WO 9514647 A
- US 2921089 A
- WO 05028407 A
- WO 0283695 A
- EP 366089 A
- US 4426524 A
- US 5434313 A
- WO 9823566 A
- WO 0063151 A
- DE 4339713 A


### In der Beschreibung aufgeführte Nicht-Patentliteratur


- Plasticizers. **A. D. GODWIN.** Applied Polymer Science 21st Century. Elsevier, 2000, 157-175
- Plasticizers. **D. F. CADIGAN ; C. J. HOWICK.** Ullmann's Encyclopedia of Industrial Chemistry. Wiley-VCH Verlag GmbH & Co. KGaA, 599-618
- **HUANG, HUAMIN et al.** *Gaodeng Xuexiao Huaxue Xuebao,* 1987, vol. 8 (6), 533-7
- Poly(Vinyl Chloride). **A. W. COAKER.** Applied Polymer Science 21st Century. Elsevier, 2000, 107-155
- Poly(Vinyl Chloride). **I. FISCHER ; W. F. SCHMITT ; H. C. PORTH ; M .W. ALLSOPP ; G. VIANELLO.** Ullmann's Encyclopedia of Industrial Chemistry. Wiley-VCH Verlag GmbH & Co. KGaA, 1-29
- **G. STORK et al.** *J. Am. Chem. Soc.,* 1963, vol. 85, 207-222
- **N. MATSUMOTO et al.** *Journal of Polymer Science: Polymer Chemistry Edition,* 1980, vol. 18, 1665-1678
- Acrylic Acid and Derivatives. *Ullmann's Encyclopedia of Industrial Chemistry*
- **FALBE.** New Syntheses with Carbon Monoxide. Springer, 1980, 162-168
- Ullmann's Encyclopedia of Industrial Chemistry. VCH Verlagsgesellschaft GmbH, 1987, vol. A 10, 137-140
- **BEISPIEL CHAUVIN et al.** *Chem. Ind.,* Mai 1974, 375-378
- **BEISPIEL CORNILS.** Hermann: Applied Homogeneous Catalysis with Organometallic Compounds. Wiley-VCH, 2002, vol. 1, 254-259
- *Hydrocarbon Processing,* Februar 1986, 31-33
- **BEISPIEL CHITNIS et al.** *Hydrocarbon Engineering,* Juni 2005, vol. 10 (6
- **BEISPIEL WEISERMEL.** Arpe: Industrielle Organische Chemie. Wiley-VCH, 1998, 96
- **BEISPIEL.** UII-mann's Encyclopedia of Industrial Chemistry. VCH Verlagsgesellschaft GmbH, 1995, vol. A1, 291-292
- Ullmann's Encyclopedia of Industrial Chemistry. VCH Verlagsgesellschaft GmbH, 1985, vol. A1, 293
- *CHEMICAL ABSTRACTS,* 10042-59-8
- *CHEMICAL ABSTRACTS,* 27458-94-2